# EUROPEAN PATENT APPLICATION

(11) **EP 2 258 385 A2**
(43) Date of publication of application: **08.12.2010**
(21) Application number: 10177887.6
(22) Date of filing: 31.07.2003
(51) Int. Cl.: A61K 39/095

(54) **Neisseria vaccine composition**

(30) Priority: 02.08.2002 GB 0218037; 02.08.2002 GB 0218036; 02.08.2002 GB 0218035; 02.08.2002 GB 0218051; 30.08.2002 GB 0220197; 30.08.2002 GB 0220199; 01.11.2002 GB 0225524; 01.11.2002 GB 0225531; 24.12.2002 GB 0230164; 24.12.2002 GB 0230168; 24.12.2002 GB 0230170; 05.03.2003 GB 0305028
(62) Divisional of application: 03784153.3
(71) Applicant: GlaxoSmithKline Biologicals S.A., 1330 Rixensart (BE)
(72) Inventor: Berthet, Francois-Xavier Jacques, 1330 Rixensart (BE); Biemans, Ralph, 1330 Rixensart (BE); Denoel, Philippe, 1330 Rixensart (BE); Feron, Christiane, 1330 Rixensart (BE); Goraj, Carine, 1330 Rixensart (BE); Poolman, Jan, 1330 Rixensart (BE); Weynants, Vincent, 1330 Rixensart (BE)
(74) Representative: Whitaker, Duncan

(57) **Abstract**

The present invention relates to immunogenic compositions and vaccines for the treatment and prevention of Neisserial disease. Immunogenic compositions of the invention contain combinations of antigens selected from at least two different classes of antigens including adhesins, autotransporter proteins, toxins, iron acquisitions proteins and membrane-associated protein (preferably integral outer membrane protein)s. Such combinations of antigens are able to target the immune response against different aspects of the neisserial life cycle, resulting in a more effective immune response.

## Description

### Technical Field

The present invention relates to the field of Neisserial immunogenic compositions and vaccines, their manufacture and the use of such compositions in medicine. More particularly, it relates to vaccine compositions comprising a combination of antigens which have qualities allowing the vaccines of the invention to elicit a surprising good immune response as measured in a protection assay or a serum bactericidal assay.

### Background

Neisserial strains of bacteria are the causative agents for a number of human pathologies, against which there is a need for effective vaccines to be developed. In particular *Neisseria gonorrhoeae* and *Neisseria meningitidis* cause pathologies which could be treated by vaccination.

*Neisseri-a gonorrhoeae* is the etiologic agent of gonorrhea, one of the most frequently reported sexually transmitted diseases in the world with an estimated annual incidence of 62 million cases (Gerbase et al 1998 Lancet 351; (Suppl 3) 2-4). The clinical manifestations of gonorrhea include inflammation of the mucus membranes of the urogenital tract, throat or rectum and neonatal eye infections. Ascending gonococcal infections in women can lead to infertility, ectopic pregnancy, chronic pelvic inflammatory disease and tubo-ovarian abscess formation. Septicemia, arthritis, endocarditis and menigitis are associated with complicated gonorrhea.

The high number of gonococcal strains with resistance to antibiotics contributes to increased morbidity and complications associated with gonorrhea. An attractive alternative to treatment of gonorrhea with antibiotics would be its prevention using vaccination. No vaccine currently exists for *N. gonorrhoeae* infections.

*Neisseria meningitidis* is an important pathogen, particularly in children and young adults. Septicemia and meningitis are the most life-threatening forms of invasive meningococcal disease (IMD). This disease has become a worldwide health problem because of its high morbidity and mortality.

Thirteen *N. meningitidis* serogroups have been identified based on antigenic differences in the capsular polysaccharides, the most common being A, B and C which are responsible for 90% of disease worldwide. Serogroup B is the most common cause of meningococcal disease in Europe, USA and several countries in Latin America.

Vaccines based on the capsular polysaccharide of serogroups A, C, W and Y have been developed and have been shown to control outbreaks of meningococcal disease (Peltola et al 1985 Pediatrics 76; 91-96). However serogroup B is poorly immunogenic and induces only a transient antibody response of a predominantly IgM isotype (Ala'Aldeen D and Cartwright K 1996, J. Infect. 33; 153-157). There is therefore no broadly effective vaccine currently available against the serogroup B meningococcus which is responsible for the majority of disease in most temperate countries. This is particularly problematic since the incidence of serotype B disease is increasing in Europe, Australia and America, mostly in children under 5. The development of a vaccine against serogroup B meningococcus presents particular difficulties because the polysaccharide capsule is poorly immunogenic owing to its immunologic similarity to human neural cell adhesion molecule. Strategies for vaccine production have therefore concentrated on the surface exposed structures of the meningococcal outer membrane but have been hampered by the marked variation in these antigens among strains.

Further developments have led to the introduction of vaccines made up of outer membrane vesicles which will contain a number of proteins that make up the normal content of the bacterial membrane. One of these is the VA-MENGOC-BC ® Cuban vaccine against *N. meningitidis* serogroups B and C (Rodriguez et al 1999 Mem Inst. Oswaldo Cruz, Rio de Janeiro 94; 433-440). This vaccine was designed to combat an invasive meningococcal disease outbreak in Cuba which had not been eliminated by a vaccination programme using a capsular polysaccharide AC vaccine. The prevailing serogroups were B and C and the VA-MENGOC-BC ® vaccine was successful at controlling the outbreak with an estimated vaccine efficiency of 83% against serogroup B strains of *N. meningitidis* (Sierra et al 1990 In Neisseria, Walter Cruyter, Berlin, m. Atchman et al (eds) p 129-134, Sierra et al 1991, NIPH Ann 14; 195-210). This vaccine was effective against a specific outbreak, however the immune response elicited would not protect against other strains of *N. meningitidis.*

Subsequent efficacy studies conducted in Latin America during epidemics caused by homologous and heterologous serogroup B meningococcal strains have shown some efficacy in older children and adults but its effectiveness was significantly lower in younger children who are at greatest risk of infection (Milagres et al 1994, Infect. Immun. 62; 4419-4424). It is questionable how effective such a vaccine would be in countries with multistrain endemic disease such as the UK. Studies of immunogenicity against heterologous strains have demonstrated only limited cross-reactive serum bactericidal activity, especially in infants (Tappero et al 1999, JAMA 281; 1520-1527).

A second outer membrane vesicle vaccine was developed in Norway using a serotype B isolate typical of those prevalent in Scandinavia (Fredriksen et al 1991, NIPH Ann, 14; 67-80). This vaccine was tested in clinical trials and found to have a protective efficacy after 29 months of 57% (Bjune et al 1991, Lancet, 338; 1093-1096).

However, the use of outer membrane vesicles in vaccines is associated with some problems. For instance, the OMV contain toxic lipopolysaccharides and they may contain immunodominant antigens which are either strain specific or are expressed variably. Several processes have been described which could be used to overcome some of the problems of outer membrane vesicle preparation vaccines. WO01/09350 describes processes that address some of these problems for instance by reducing toxicity and modifying the antigens present on the outer membrane vesicles.

WO01/52885 describes the possibility of combining outer membrane vesicles with other antigens and a list of over 2,000 potential Neisserial proteins is included from which it is speculated that a vaccines with efficacy against a broader range of serotypes could be developed.

There are diverse problems with the anti-meningococcal vaccines currently available. The protein based outer membrane vaccines tend to be specific and effective against only a few strains. The polysaccharide vaccines are also suboptimal since they tend to elicit poor and short immune responses, particularly against serogroup B (Lepow et al 1986; Peltola 1998, Pediatrics 76; 91-96).

Neisseria infections represent a considerable health care problem for which no vaccines are available in the case of *N. gonorrhoeae* or vaccines with limitations on their efficacy and ability to protect against heterologous strains are available in the case of *N. meningitidis.* Clearly there is a need to develop superior vaccines against Neisserial infections that will improve on the efficacy of currently available vaccines and allow for protection against a wider range of strains.

### Description of Figures

Figure 1. - Detection of TbpA and Hsf in OMV's prepared from a recombinant *N. meningitidis* strain up-regulated for the expression of *tbpA* and *hsf.* Separation of OMV preparations (10µg) by SDS-PAGE analysis (4-20% gradient gels) stained with Coomassie brilliant blue.
Figure 2.- Detection of recombinant Hsf passenger domain produced in E. coli, 10ug of purified Hsf passenger protein (Lane 2 & 3) was separated by SDS-PAGE on a 12% gel in comparison to a molecular weight marker (Lane 1).
Figure 3. - Analysis of Hap passenger purity as detected by (A) Coomassie staining, (B) silver staining, (C) anti-His5 western blotting and (D) anti-*E. coli.* 10µg of purified antigens was separated by SDS-PAGE on a 4-20% acrylamide gradient gel.
Figure 4. - Regions of sequence similarity shared by FrpA and FrpC proteins isolated from *N. meningitidis* strain FAM20. (A) Domain organization of *N. meningitidis* strain FAM20 RTX proteins FrpA and FrpC. (B) FrpA/C Amplification products obtained from *N. meningitidis* strain H44/76.
Figure 5. - Expression of recombinant Frp23 (FrpA/C conserved region with 23 repeats) antigen in *E. coli.* SDS-PAGE analysis of non-induced (NI) and induced (I) total cell extracts of *E. coli* BL21DE3 tranformed with control vectors (pET24d) or recombinant constructs (Frp3, Frp13 and Frp 23 respectively). Gels were stained with Coomassie blue (A) or transferred to nitrocellulose and immuno-detected with anti-His6 mouse serum.
Figure 6. - Preferred DNA sequence of the FHAB 2/3^{rd} fragment expressed in *E. coli.*
Figure 7. - Purification of recombinant FHAB 2/3^{rd} from induced *E. coli* B121DE3 extracts. (A) Main steps in the purification process. (B) SDS-PAGE analysis of protein fractions sampled at different steps of the purification process.
Figure 8. - Adhesion blocking activities of anti-sera directed against the FHAB2/3^{ra}, Hap, Hap passenger domain, Hsf and Hsf passenger domain antigens of *N. meningitidis.*

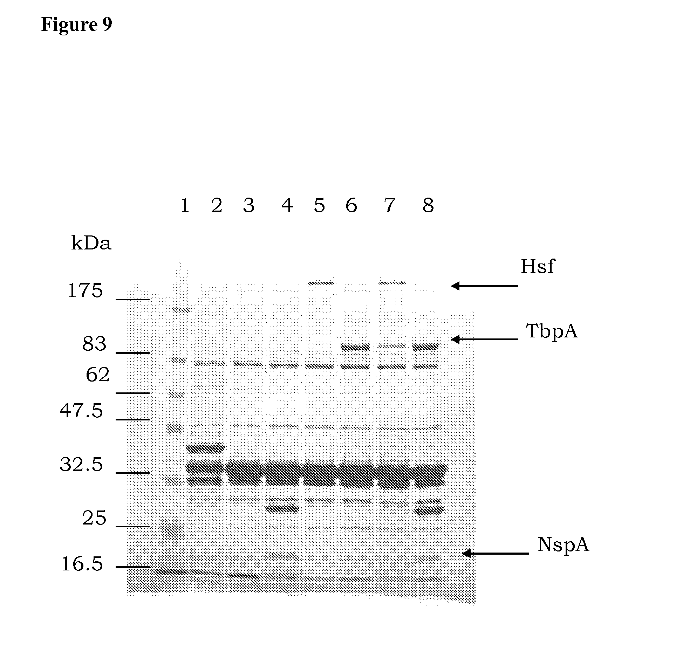

Figure 9. - A Coomassie stained gel showing expression levels of Hsf, TbpA and NspA in outer membrane vesicle preparations derived from different *N. meningitidis* stains. Lane 1 - molecular weight markers; lane 2 - outer membrane vesicles prepared from strain H44/76 in which capsular polysaccharides were downregulated; lane 3 - outer membrane vesicles prepared from strain H44/76 in which capsular polysaccharides and PorA were downregulated; lane 4 - outer membrane vesicles prepared from strain H44/76 in which capsular polysaccharides and PorA were downregulated and NspA was upregulated; lane 5 - outer membrane vesicles prepared from strain H44/76 in which capsular polysaccharides and PorA were downregulated and Hsf was upregulated; lane 6 - outer membrane vesicles prepared from strain H44/76 in which capsular polysaccharides and PorA were downregulated and TbpA was upregulated; lane 7 - outer membrane vesicles prepared from strain H44/76 in which capsular polysaccharides and PorA were downregulated and TbpA and Hsf were upregulated; lane 8 - outer membrane vesicles prepared from strain H44/76 in which capsular polysaccharides and PorA were downregulated and TbpA and NspA were upregulated.

### Detailed description

The present invention discloses particular combinations of Neisserial antigens which when combined, lead to a surprising enhancement of the efficacy of the vaccine against Neisserial infection.

Neisserial infections progress through several different stages. For example, the meningococcal life cycle involve nasopharyngeal colonisation, mucosal attachment, crossing into the bloodstream, multiplication in the blood, induction of toxic shock, crossing the blood/brain barrier and multiplication in the cerebrospinal fluid and/or the meninges. Different molecules on the surface of the bacterium will be involved in different steps of the infection cycle. By targeting the immune response against an effective amount of a combination of particular antigens, involved in different processes of Neisserial infection, a Neisserial vaccine with surprisingly high efficacy can be achieved.

In particular, combinations of certain antigens from different classes, some of which are involved in adhesion to host cells, some of which are involved in iron acquisition, some of which are autotransporters and some of which are toxins, can elicit an immune response which protects against multiple stages of infection. Such combinations of antigens can surprisingly lead to improved (preferably synergistically improved) vaccine efficacy against Neisserial infection where more that one function of the bacterium is targeted by the immune response in an optimal fashion.

The efficacy of vaccines can be assessed through a variety of assays. Protection assays in animal models are well known in the art. Furthermore, serum bactericidal assay (SBA) is the most commonly agreed immunological marker to estimate the efficacy of a meningococcal vaccine (Perkins et al. J Infect Dis. 1998, 177:683-691).

Some combinations of antigens (for example, combinations of certain autotransporter proteins and certain iron acquisition proteins) can lead to improved protection in animal model assays and/or synergistically higher SBA titres. Without wishing to be bound by theory, such synergistic combinations of antigens are enabled by a number of characteristics of the immune response to the antigen combination. The antigens themselves are usually surface exposed on the Neisserial cells and tend to be conserved but also tend not to be present in sufficient quantity on the surface cell for an optimal bactericidal response to take place using antibodies elicited against the antigen alone. Combining the antigens of the invention can result in a formulation eliciting an advantageous combination of bactericidal antibodies which interact with the Neisserial cell beyond a critical threshold. At this critical level, sufficient antibodies of sufficient quality bind to the surface of the bacterium to allow efficient killing by complement and much higher bactericidal effects are seen as a consequence.

As serum bactericidal assays (SBA) closely reflect the efficacy of vaccine candidates, the attainment of good SBA titres by a combination of antigens is a good indication of the protective efficacy of a vaccine containing that combination of antigens. The invention relates to the use of a combination of two antigens either isolated or enriched in a mixture with other antigens. When combined, such antigen combinations interact advantageously, and preferably synergistically to elicit an immune response that is higher in terms of bactericidal activity (for example as measured by serum bactericidal assay or SBA), and preferably higher than the additive response elicited by the antigens individually, more preferably by a factor of at least 1.2, 1.5, two, three, four, five, six, seven, eight, nine, most preferably by a factor of at least ten.

An additional advantage of the invention is that the combination of the antigens of the invention from different families of proteins in an immunogenic composition may enable protection against a wider range of strains.

The invention relates to immunogenic compositions comprising a plurality (two or more) of proteins selected from at least two different categories of protein, having different functions within Neisseria. Examples of such categories of proteins are adhesins, autotransporter proteins, toxins, integral outer membrane proteins and Fe acquisition proteins. The vaccine combinations of the invention show surprising improvement in vaccine efficacy against homologous Neisserial strains (strains from which the antigens are derived) and preferably also against heterologous Neisserial strains.

The invention provides immunogenic compositions comprising at least or exactly two, three, four, five six, seven, eight, nine or ten of different antigens selected from at least or exactly two, three , four or all five categories of antigens selected from the following:
- at least one Neisserial adhesin;
- at least one Neisserial autotransporter;
- at least one Neisserial toxin;
- at least one Neisserial Fe acquisition protein;
- at least one Neisserial membrane-associated protein ( preferably outer membrane protein, particularly integral outer membrane protein).

Preferably, the invention provides immunogenic compositions that comprise at least or exactly two, three, four, five, six, seven, eight, nine or ten different Neisseria antigens. Most preferably these antigens are selected from at least or exactly two, three, four or five groups of proteins selected from the following:
- at least one Neisserial adhesin selected from the group consisting of FhaB, NspA PilC, Hsf, Hap, MafA, MafB, Omp26, NMB 0315, NMB 0995, NMB 1119 and NadA;
- at least one Neisserial autotransporter selected from the group consisting of Hsf, Hap, IgA protease, AspA,and NadA;
- at least one Neisserial toxin selected from the group consisting of FrpA, FrpC, FrpA/C, VapD, NM-ADPRT and either or both of LPS immunotype L2 and LPS immunotype L3;
- at least one Neisserial Fe acquisition protein selected from the group consisting of TbpA, TbpB, LbpA, LbpB, HpuA, HpuB, Lipo28 (GNA2132), Sibp, NMB0964, NMB0293, FbpA, Bcp, BfrA, BfrB and P2086 (XthA); and
- at least one Neisserial membrane-associated protein, preferably outer membrane protein, particularly integral outer membrane protein, selected from the group consisting of PilQ, OMP85, FhaC, NspA, TbpA, LbpA, TspA, TspB, TdfH, PorB, MltA, HpuB, HimD, HisD, GNA1870, OstA, HlpA (GNA1946), NMB 1124, NMB 1162, NMB 1220, NMB 1313, NMB 1953, HtrA, and PldA (Omp 1A).

The antigens of the present invention are all isolated, meaning that they are altered by the hand of man. Preferably they are purified to some degree, most preferably more than 40, 50, 60, 70, 80, 90, 95 or 99% pure (before combination with the other components of the immunogenic compositions of the invention).

Preferably the immunogenic composition of the invention comprises at least one Neisserial adhesin and at least one Neisserial autotranporter and optionally a Neisserial toxin, a Neisserial Fe acquisition protein or a Neisserial membrane-associated protein (preferably integral outer membrane protein). Preferably the antigens are selected from the above named antigens.

Preferably the immunogenic composition of the invention comprises at least one Neisserial adhesin and at least one Neisserial toxin and optionally a Neisserial autotranporter, a Neisserial Fe acquisition protein or a Neisserial membrane-associated protein (preferably integral outer membrane protein). Preferably the antigens are selected from the above named antigens.

Preferably the immunogenic composition of the invention comprises at least one Neisserial adhesin and at least one Neisserial Fe acquisition protein and optionally a Neisserial toxin, a Neisserial autotransporter or a Neisserial membrane-associated protein (preferably integral outer membrane protein). Preferably the antigens are selected from the above named antigens.

Preferably the immunogenic composition of the invention comprises at least one Neisserial adhesin and at least one Neisserial membrane-associated protein (preferably integral outer membrane protein) and optionally a Neisserial toxin, a Neisserial Fe acquisition protein or a Neisserial autotransporter. Preferably the antigens are selected from the above named antigens.

Preferably the immunogenic composition of the invention comprises at least one Neisserial autotranporter and at least one Neisserial toxin and optionally a Neisserial adhesin, a Neisserial Fe acquisition protein or a Neisserial membrane-associated protein (preferably integral outer membrane protein). Preferably the antigens are selected from the above named antigens.

Preferably the immunogenic composition of the invention comprises at least one Neisserial autotranporter and at least one Neisserial Fe acquisition protein and optionally a Neisserial adhesin, a Neisserial toxin or a Neisserial membrane-associated protein (preferably integral outer membrane protein). Preferably the antigens are selected from the above named antigens.

Preferably the immunogenic composition of the invention comprises at least one Neisserial autotranporter and at least one Neisserial membrane-associated protein (preferably integral outer membrane protein) and optionally a Neisserial adhesin, a Neisserial Fe acquisition protein or a Neisserial toxin. Preferably the antigens are selected from the above named antigens.

Preferably the immunogenic composition of the invention comprises at least one Neisserial toxin and at least one Neisserial Fe acquisition protein and optionally a Neisserial adhesin, a Neisserial autotransporter or a Neisserial membrane-associated protein (preferably integral outer membrane protein). Preferably the antigens are selected from the above named antigens.

Preferably the immunogenic composition of the invention comprises at least one Neisserial toxin and at least one Neisserial membrane-associated protein (preferably integral outer membrane protein) and optionally a Neisserial adhesin, a Neisserial autotransporter or a Neisserial toxin. Preferably the antigens are selected from the above named antigens.

Preferably the immunogenic composition of the invention comprises at least one Neisserial Fe acquisition protein and at least one Neisserial membrane-associated protein (preferably integral outer membrane protein) and optionally a Neisserial adhesin, a Neisserial autotransporter or a Neisserial toxin. Preferably the antigens are selected from the above named antigens.

Preferably all five groups of antigen are represented in the immunogenic composition of the invention.

Where a protein is specifically mentioned herein, it is preferably a reference to a native, full-length protein, and to its natural variants (i.e. to a native protein obtainable from a Neisserial, preferably meningococcal strain) but it may also encompass antigenic fragments thereof (particularly in the context of subunit vaccines). These are fragments (often specifically described herein) containing or comprising at least 10 amino acids, preferably 20 amino acids, more preferably 30 amino acids, more preferably 40 amino acids or most preferably 50 amino acids, taken contiguously from the amino acid sequence of the protein. In addition, antigenic fragments denotes fragments that are immunologically reactive with antibodies generated against the Neisserial full-length proteins or with antibodies generated by infection of a mammalian host with Neisseria. Antigenic fragments also includes fragments that when administered at an effective dose, elicit a protective immune response against Neisserial infection, more preferably it is protective against *N. meningitidis* and/or *N. gonorrhoeae* infection, most preferably it is protective against *N. meningitidis* serogroup B infection.

Also included in the invention are recombinant fusion proteins of Neisserial proteins of the invention, or fragments thereof. These may combine different Neisserial proteins or fragments thereof in the same polypeptide. Alternatively, the invention also includes individual fusion proteins of Neisserial proteins or fragments thereof, as a fusion protein with heterologous sequences such as a provider of T-cell epitopes or purification tags, for example: β-galactosidase, glutathione-S-transferase, green fluorescent proteins (GFP), epitope tags such as FLAG, myc tag, poly histidine, or viral surface proteins such as influenza virus haemagglutinin, tetanus toxoid, diphtheria toxoid, CRM 197.

### Antigens of the invention

NMB references refer to reference numbers to sequences which can be accessed from www.neisseria.org.

### 1. Adhesins

Adhesins include FhaB (WO98/02547), NadA (J. Exp.Med (2002) 195:1445; NMB 1994), Hsf also known as NhhA (NMB 0992) (WO99/31132), Hap (NMB 1985)(WO99/55873), NspA (WO96/29412), MafA (NMB 0652) and MafB (NMB 0643) (Annu Rev Cell Dev Biol. 16; 423-457 (2000); Nature Biotech 20; 914-921 (2002)), Omp26 (NMB 0181), NMB 0315, NMB 0995, NMB 1119 and PilC (Mol. Microbiol.1997, 23; 879-892). These are proteins that are involved in the binding of Neisseria to the surface of host cells. Hsf is an example of an adhesin, as well as being an autotranporter protein. Immunogenic compositions of the invention may therefore include combinations of Hsf and other autotransporter proteins where Hsf contributes in its capacity as an adhesin. These adhesins may be derived from *Neisseria, meningitidis* or *Neisseria gonorrhoeae* or other Neisserial strains. The invention also includes other adhesins from Neisseria.

### FhaB

This antigen has been described in WO98/02547 SEQ ID NO 38 (nucleotides 3083-9025) - see also NMB0497. The present inventors have found FhaB to be particularly effectively at inducing anti-adhesive antibodies alone and in particular with other antigens of the invention. Although full length FhaB could be used, the inventors have found that particular C-terminal truncates are surprisingly at least as effective and preferably even more effective in terms of cross-strain effect. Such truncates have also been advantageously shown to be far easier to clone. FhaB truncates of the invention typically correspond to the N-terminal two-thirds of the FhaB molecule, preferably the new C-terminus being situated at position 1200-1600, more preferably at position 1300-1500, and most preferably at position 1430-1440. Specific embodiments have the C-terminus at 1433 or 1436. Accordingly such FhaB truncates of the invention and vaccines comprising such truncates are independent aspects of the present invention as well as being components of the combination immunogenic compositions of the invention. The N-terminus may also be truncated by up to 10, 20, 30, 40 or 50 amino acids.

### 2. Autotransporter proteins

Autotransporter proteins typically are made up of a signal sequence, a passenger domain and an anchoring domain for attachment to the outer membrane. Examples of autotransporter proteins include Hsf (WO99/31132) (NMB 0992), HMW, Hia (van Ulsen et al Immunol. Med. Microbiol. 2001 32; 53-64), Hap (NMB 1985) (WO99/55873; van Ulsen et al Immunol. Med. Microbiol. 2001 32; 53-64), UspA, UspA2, NadA (NMB 1994) (Comanducci et al J. Exp. Med. 2002 195; 1445-1454), AspA (Infection and Immunity 2002, 70(8); 4447-4461; NMB 1029), Aida-1 like protein, SSh-2 and Tsh. NadA (J. Exp.Med (2002) 195:1445) is another example of an autotransporter proteins, as well as being an adhesin. Immunogenic compositions of the invention may therefore include combinations of NadA and adhesins where NadA contributes in its capacity as an autotransporter protein.These proteins may be derived from *Neisseria meningitidis* or *Neisseria gonorrhoeae* or other Neiserial strians. The invention also includes other autotransporter proteins from Neisseria.

### Hsf

Hsf has a structure that is common to autotransporter proteins. For example, Hsf from *N. meningitidis* strain H44/76 consists of a signal sequence made up of amino acids 1-51, a head region at the amino terminus of the mature protein (amino acids 52-479) that is surface exposed and contains variable regions (amino acids 52-106, 121-124, 191-210 and 230-234), a neck region (amino acids 480-509), a hydrophobic alpha-helix region (amino acids 518-529) and an anchoring domain in which four transmembrane strands span the outer membrane (amino acids 539-591).

Although full length Hsf may be used in immunogenic compositions of the invention, various Hsf truncates and deletions may also be advantageously used depending on the type of vaccine.

Where Hsf is used in a subunit vaccine, it is preferred that a portion of the soluble passenger domain is used; for instance the complete domain of amino acids 52 to 479, most preferably a conserved portion thereof, for instance the particularly advantageous sequence of amino acids 134 to 479. Preferred forms of Hsf may be truncated so as to delete variable regions of the protein disclosed in WO01/55182. Preferred variants would include the deletion of one, two, three, four, or five variable regions as defined in WO01/55182. The above sequences and those described below, can be extended or truncated by up to 1, 3, 5, 7, 10 or 15 amino acids at either or both N or C termini.

Preferred fragments of Hsf therefore include the entire head region of Hsf, preferably containing amino acids 52-473. Additional preferred fragments of Hsf include surface exposed regions of the head including one or more of the following amino acid sequences; 52-62, 76-93, 116-134, 147-157, 157-175, 199-211, 230-252, 252-270, 284-306, 328-338, 362-391, 408-418, 430-440 and 469-479.

Where Hsf is present in an outer membrane vesicle preparation, it may be expressed as the full-length protein or preferably as an advantageous variant made up of a fusion of amino acids 1-51 and 134-591 (yielding a mature outer membrane protein of amino acid sequence 134 to the C-terminus). Preferred forms of Hsf may be truncated so as to delete variable regions of the protein disclosed in WO01/55182. Preferred variants would include the deletion of one, two, three, four, or five variable regions as defined in WO01/55182. Preferably the first and second variable regions are deleted. Preferred variants would delete residues from between amino acid sequence 52 through to 237 or 54 through to 237, more preferably deleting residues between amino acid 52 through to 133 or 55 through to 133. The mature protein would lack the signal peptide.

### Hap

Computer analysis of the Hap-like protein from *Neisseria meningitidis* reveals at least three structural domains. Considering the Hap-like sequence from strain H44/76 as a reference, Domain 1, comprising amino-acid 1 to 42, encodes a sec-dependant signal peptide characteristic of the auto-transporter family, Domain 2, comprising amino-acids 43 to 950, encode the passenger domain likely to be surface exposed and accessible to the immune system, Domain 3, comprising residues 951 to the C-terminus (1457), is predicted to encode a beta-strands likely to assemble into a barrel-like structure and to be anchored into the outer-membrane. Since domains 2 is likely to be surface-exposed, well conserved (more than 80% in all strain tested) and could be produced as subunit antigens in *E. coli,* it represents an interesting vaccine candidates. Since domains 2 and 3 are likely to be surface-exposed, are well conserved (Pizza et al. (2000), Science 287: 1816-1820), they represent interesting vaccine candidates. Domain 2 is known as the passenger domain.

Immunogenic compositions of the invention may comprise the full-length Hap protein, preferably incorporated into an OMV preparation. Immunogenic compositions of the invention may also comprise the passenger domain of Hap which in strain H44/76 is composed of amino acid residues 43-950. This fragment of Hap would be particularly advantageously used in a subunit composition of the invention.The above sequence for the passenger domain of Hap can be extended or truncated by up to 1, 3, 5, 7, 10 ,15, 20, 25, or 30 amino acids at either or both N or C termini.

### 3. Iron acquisition proteins

Iron aquisition proteins include TbpA (NMB 0461) (WO92/03467, US5912336, WO93/06861 and EP586266), TbpB (NMB 0460) (WO93/06861 and EP586266), LbpA (NMB 1540) (Med Microbiol (1999) 32:1117), LbpB (NMB 1541)(WO/99/09176), HpuA (U73112.2) (Mol Microbiol. 1997, 23; 737-749), HpuB (NC_003116.1) (Mol Microbiol. 1997, 23; 737-749), P2086 also known as XthA (NMB 0399) (13^{th} International Pathogenic Neisseria Conference 2002), FbpA (NMB 0634), FbpB, BfrA (NMB 1207), BfrB (NMB 1206), Lipo28 also known as GNA2132 (NMB 2132), Sibp (NMB 1882), HmbR, HemH, Bcp (NMB 0750), Iron (III) ABC transporter-permease protein (Tettelin et al Science 287; 1809-1815 2000), Iron (III) ABC transporter - periplasmic (Tettelin et al Science 287; 1809-1815 2000), TonB-dependent receptor (NMB 0964 and NMB 0293)(Tettelin et al Science 287; 1809-1815 2000) and transferrin binding protein related protein (Tettelin et al Science 287; 1809-1815 2000). These proteins may be derived from *Neisseria meningitidis, Neisseria gonorrhoeae* or other Neisserial strains. The invention also includes other iron aquisition proteins from Neisseria.

### TbpA

TbpA interacts with TbpB to form a protein complex on the outer membrane of Neisseria, which binds transferrin. Structurally, TbpA contains an intracellular N-terminal domain with a TonB box and plug domain, multiple transmembrane beta strands linked by short intracellular and longer extracellular loops.

Two families of TbpB have been distinguished, having a high molecular weight and a low molecular weight respectively. High and low molecular weight forms of TbpB associate with different families of TbpA which are distinguishable on the basis of homology. Despite being of similar molecular weight, they are known as the high molecular weight and low molecular weight families because of their association with the high or low molecular weight form of TbpB (Rokbi et al FEMS Microbiol. Lett. 100; 51, 1993). The terms TbpA(high) and TbpA(low) are used to refer to these two forms of TbpA, and similarly for TbpB. Immunogenic compositions of the invention may comprise TbpA and TbpB from serogroups A, B, C, Y and W-135 of *N. meningitidis* as well as iron acquisition proteins from other bacteria including *N. gonorrhoeae.* Transferrin binding proteins TbpA and TbpB have also been referred to as Tbp1 and Tbp2 respectively (Cornelissen et al Infection and Immunity 65; 822, 1997).

TbpA contains several distinct regions. For example, in the case of TbpA from *N. meningitidis* strain H44/76, the amino terminal 186 amino acids form an internal globular domain, 22 beta strands span the membrane, forming a beta barrel structure. These are linked by short intracellular loops and larger extracellular loops. Extracellular loops 2, 3 and 5 have the highest degree of sequence variability and loop 5 is surface exposed. Loops 5 and 4 are involved in ligand binding.

Preferred fragments of TbpA include the extracellular loops of TbpA. Using the sequence of TbpA from *N. meningitidis* strain H44/76, these loops correspond to amino acids 200-202 for loop1, amino acids 226-303 for loop 2, amino acids 348-395 for loop 3, amino acids 438-471 for loop 4, amino acids 512-576 for loop 5, amino acids 609-625 for loop 6, amino acids 661-671 for loop 7, amino acids 707-723 for loop 8, amino acids 769-790 for loop 9, amino acids 814-844 for loop 10 and amino acids 872-903 for loop 11. The corresponding sequences, after sequence alignment, in other Tbp proteins would also constitute preferred fragments. Most preferred fragments would include amino acid sequences constituting loop 2, loop 3, loop 4 or loop 5 of Tbp.

Where the immunogenic compositions of the invention comprise TbpA, it is preferable to include both TbpA(high) and TbpA (low).

Although TbpA is preferably presented in an OMV vaccine, it may also be part of a subunit vaccine. For instance, isolated iron acquisition proteins which could be introduced into an immmunogenic composition of the invention are well known in the art (WO00/25811). They may be expressed in a bacterial host, extracted using detergent (for instance 2% Elugent) and purified by affinity chromatography or using standard column chromatography techniques well known to the art (Oakhill et al Biochem J. 2002 364; 613-6).

Where TbpA is presented in an OMV vaccine, its expression can be upregulated by genetic techiques discussed herein, or may preferably be upregulated by growth of the parent strain under iron limitation conditions as described below. This process will also result in the upregulation of variable iron-regulated proteins, particularly FrpB which may become immunodominant and it is therefore advantageous to downregulate the expression of (and preferably delete the genes encoding) such proteins (particularly FrpB) as described below, to ensure that the immunogenic composition of the invention elicits an immune response against antigens present in a wide range of Neisserial strains. It is preferred to have both TbpA(high) and TbpA(low) present in the immunogenic composition and this is preferably achieved by combining OMVs derived from two strains, expressing the alternative forms of TbpA.

### 4. Toxins

Toxins include FrpA (NMB 0585; NMB 1405), FrpA/C (see below for definition), FrpC (NMB 1415; NMB 1405) (WO92/01460), NM-ADPRT (NMB 1343) (13th International Pathogenic Neisseria Conference 2002 Masignani et al p135), VapD (NMB 1753), lipopolysaccharide (LPS; also called lipooligosaccharide or LOS) immunotype L2 and LPS immunotype L3. FrpA and FrpC contain a region which is conserved between these two proteins and a preferred fragment of the proteins would be a polypeptide containing this conserved fragment, preferably comprising amino acids 227-1004 of the sequence of FrpA/C. These antigens may be derived from *Neisseria meningitidis* or *Neisseria gonorrhoeae* or other Neisserial strains. The invention also includes other toxins from Neisseria.

In an alternative embodiment, toxins may include antigens involved in the regulation of toxicity, for example OstA which functions in the synthesis of lipopolysaccharides.

### FrpA and FrpC

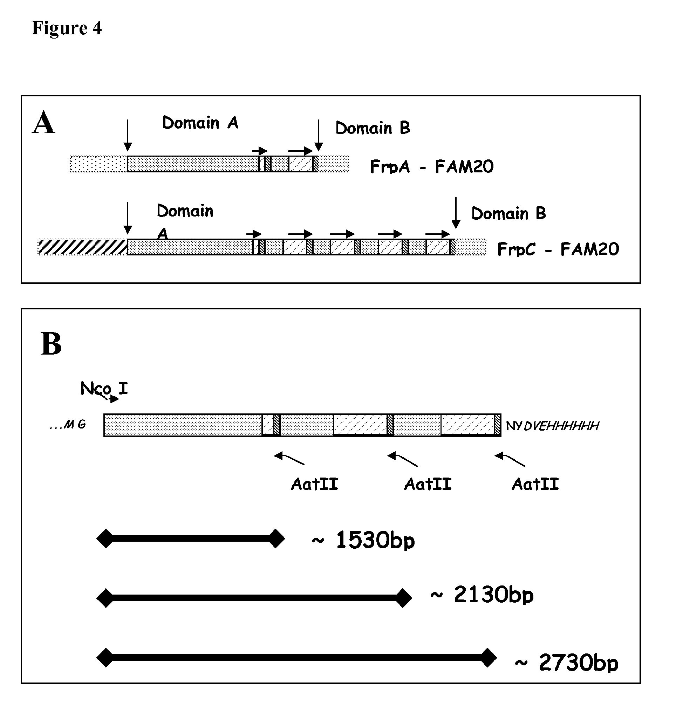

*Neisseria meningitidis* encodes two RTX proteins, referred to as FrpA & FrpC secreted upon iron limitation (Thompson et al., (1993) J. Bacteriol. 175:811-818; Thompson et al., (1993) Infect. Immun.. 61:2906-2911). The RTX (Repeat ToXin) protein family have in common a series of 9 amino acid repeat near their C-termini with the consensus: Leu Xaa Gly Gly Xaa Gly (Asn/Asp) Asp Xaa.
(LXGGXGN_{/D}DX). The repeats in *E. coli* HlyA are thought to be the site of Ca2+ binding. As represented in Figure 4, meningococcal FrpA and FrpC proteins, as characterized in strain FAM20, share extensive amino-acid similarity in their central and C-terminal regions but very limited similarity (if any) at the N-terminus. Moreover, the region conserved between FrpA and FrpC exhibit some polymorphism due to repetition (13 times in FrpA and 43 times in FrpC) of a 9 amino acid motif.

Immunogenic compositions of the invention may comprise the full length FrpA and/or FrpC or preferably, a fragment comprising the sequence conserved between FrpA and FrpC. The conserved sequence is made up of repeat units of 9 amino acids. Immunogenic compositions of the invention would preferably comprise more that three repeats, more than 10 repeats, more than 13 repeats, more than 20 repeats or more than 23 repeats.

Such truncates have advantageous properties over the full length molecules and vaccines comprising such antigens form an independent aspect of invention as sell as being incorporated in the immunogenic compositions of the invention.

Sequences conserved between FrpA and FrpC are designated FrpA/C and whereever FrpA or FrpC forms a constituent of immunogenic compositions of the invention, FrpA/C could be advantageously used. Amino acids 277-1004 of the FrpA sequence is the preferred conserved region. The above sequence can be extended or truncated by up to 1, 3, 5, 7, 10, 15, 20, 25, or 30 amino acids at either or both N or C termini.

### LPS

LPS (lipopolysaccharide, also known as LOS - lipooligosaccharide) is the endotoxin on the outer membrane of Neisseria. The polysaccharide moiety of the LPS is known to induce bactericidal antibodies.

Heterogeneity within the oligosaccharide moiety of the LPS generates structural and antigenic diversity among different neisserial strains (Griffiss et al. Inf. Immun. 1987; 55: 1792-1800). This has been used to subdivide meningococcal strains into 12 immunotypes (Scholtan et al. J Med Microbiol 1994, 41:236-243). Immunotypes L3, L7, & L9 are immunologically identical and are structurally similar (or even the same) and have therefore been designated L3,7,9 (or, for the purposes of this specification, generically as "L3"). Meningococcal LPS L3,7,9 (L3), L2 and L5 can be modified by sialylation, or by the addition of cytidine 5'-monophosphate-N-acetylneuraminic acid. Although L2, L4 and L6 LPS are distinguishable immunologically, they are structurally similar and where L2 is mentioned herein, either L4 or L6 may be optionally substituted within the scope of the invention. See M. P. Jennings et al, Microbiology 1999, 145, 3013-3021 and Mol Microbiol 2002, 43:931-43 for further illustration of LPS structure and heterogeneity.

Where LPS, preferably meningococcal LPS, is included in a vaccine of the invention, preferably and advantageously either or both of immunotypes L2 and L3 are present. LPS is preferably presented in an outer membrane vesicle (preferably where the vesicle is extracted with a low percentage detergent, more preferably 0-0.5%, 0.02-0.4%, 0.04-0.3%, 0.06-0.2%, 0.08-0.15% or 0.1%, most preferably deoxycholate [DOC]) but may also be part of a subunit vaccine. LPS may be isolated using well known precedure including the hot water-phenol procedure (Wesphal and Jann Meth. Carbo. Chem. 5; 83-91 1965). See also Galanos et al. 1969, Eur J Biochem 9:245-249, and Wu et al. 1987, Anal Bio Chem 160:281-289. LPS may be used plain or conjugated to a source of T-cell epitopes such as tetanus toxoid, Diphtheria toxoid, CRM-197 or OMV outer membrane proteins. Techniques for conjugating isolated LOS are also known (see for instance EP 941738 incorporated by reference herein).

Where LOS (in particular the LOS of the invention) is present in a bleb formulation the LOS is preferably conjugated in situ by methods allowing the conjugation of LOS to one or more outer membrane proteins also present on the bleb preparation (e.g. PorA or PorB in meningococcus).

This process can advantageously enhance the stability and/or immunogenicity (providing T-cell help) and/or antigenicity of the LOS antigen within the bleb formulation - thus giving T-cell help for the T-independent oligosaccharide immunogen in its most protective conformation - as LOS in its natural environment on the surface of meningococcal outer membrane. In addition, conjugation of the LOS within the bleb can result in a detoxification of the LOS (the Lipid A portion being stably buried in the outer membrane thus being less available to cause toxcity). Thus the detoxification methods mentioned herein of isolating blebs from htrB⁻ or msbB⁻ mutants, or by adding non toxic peptide functional equivalent of polymyxin B [a molecule with high affinity to Lipid A] to the composition (see WO 93/14115, WO 95/03327, Velucchi et al (1997) J Endotoxin Res 4: 1-12, and EP 976402 for further details of non-toxic peptide functional equivalents of polymyxin B - particularly the use of the peptide SAEP 2 (of sequence KTKCKFLKKC where the 2 cysteines form a disulphide bridge)) may not be required (but which may be added in combination for additional security). Thus the inventors have found that a composition comprising blebs wherein LOS present in the blebs has been conjugated in an intra-bleb fashion to outer membrane proteins also present in the bleb can form the basis of a vaccine for the treatment or prevention of diseases caused by the organism from which the blebs have been derived, wherein such vaccine is substantially non-toxic and is capable of inducing a T-dependent bactericidal response against LOS in its native environment.

This invention therefore further provides such an intra-bleb LOS conjugated meningococcal bleb preparation.

Such bleb preparations may be isolated from the bacterial in question (see WO 01/09350), and then subjected to known conjugation chemistries to link groups (e.g. NH₂ or COOH) on the oligosaccharide portion of LOS to groups (e.g. NH₂ or COOH) on bleb outer membrane proteins. Cross-linking techniques using glutaraldehyde, formaldehyde, or glutaraldehyde/formaldehyde mixes may be used, but it is preferred that more selective chemistries are used such as EDAC or EDAC/NHS (J.V. Staros, R.W. Wright and D. M. Swingle. Enhancement by N-hydroxysuccinimide of water-soluble carbodiimide-mediated coupling reactions. Analytical chemistry 156: 220-222 (1986); and Bioconjugates Techniques. Greg T. Hermanson (1996) pp173-176). Other conjugation chemistries or treatments capable of creating covalent links between LOS and protein molecules that could be used are described in EP 941738.

Preferably the bleb preparations are conjugated in the absence of capsular polysaccharide. The blebs may be isolated from a strain which does not produce capsular polysaccharide (naturally or via mutation as described below), or may be purified from most and preferably all contaminating capsular polysaccharide. In this way, the intra-bleb LOS conjugation reaction is much more efficient.

Preferably more than 10, 20, 30, 40, 50, 60, 70, 80, 90, or 95% of the LOS present in the blebs is cross-linked/conjugated.

Intrableb conjugation should preferably incorporate 1, 2 or all 3 of the following process steps: conjugation pH should be greater than pH 7.0, preferably greater than or equal to pH 7.5 (most preferably under pH 9); conditions of 1-5% preferably 2-4% most preferably around 3% sucrose should be maintained during the reaction; NaCl should be minimised in the conjugation reaction, preferably under 0.1M, 0.05M, 0.01M, 0.005M, 0.001M, and most preferably not present at all. All these process features make sure that the blebs remain stable and in solution throughout the conjugation process.

The EDAC/NHS conjugation process is a preferred process for intra-bleb conjugation. EDAC/NHS is preferred to formalydehyde which can cross-link to too high an extent thus adversely affecting filterability. EDAC reacts with carboxylic acids (such as KDO in LOS) to create an active-ester intermediate. In the presence of an amine nucleophile (such as lysines in outer membrane proteins such as PorB), an amide bond is formed with release of an isourea by-product. However, the efficiency of an EDAC-mediated reaction may be increased through the formation of a Sulfo-NHS ester intermediate. The Sulfo-NHS ester survives in aqueous solution longer than the active ester formed from the reaction of EDAC alone with a carboxylate. Thus, higher yields of amide bond formation may be realized using this two-stage process. EDAC/NHS conjugation is discussed in J.V. Staros, R.W. Wright and D. M. Swingle. Enhancement by N-hydroxysuccinimide of water-soluble carbodiimide-mediated coupling reactions. Analytical chemistry 156: 220-222 (1986); and Bioconjugates Techniques. Greg T. Hermanson (1996) pp173-176. Preferably 0.01-5 mg EDAC /mg bleb is used in the reaction, more preferably 0.05-1 mg EDAC/mg bleb. The amount of EDAC used depends on the amont of LOS present in the sample which in turn depends on the deoxycholate (DOC) % used to extract the blebs. At low % DOC (e.g. 0.1%), high amounts of EDAC are used (1mg/mg and beyond), however at higher % DOC (e.g. 0.5%), lower amounts of EDAC are used (0.025-0.1mg/mg) to avoid too much inter-bleb crosslinking.

A preferred process of the invention is therefore a process for producing intrableb conjugated LOS (preferably meningococcal) comprising the steps of conjugating blebs in the presence of EDAC/NHS at a pH between pH 7.0 and pH 9.0 (preferably around pH 7.5), in 1-5% (preferably around 3%) sucrose, and optionally in conditions substantially devoid of NaCl (as described above), and isolating the conjugated blebs from the reaction mix.

The reaction may be followed on Western separation gels of the reaction mixture using anti-LOS (e.g. anti-L2 or anti-L3) mAbs to show the increase of LOS molecular weight for a greater proportion of the LOS in the blebs as reaction time goes on.
Yields of 99% blebs can be recovered using such techniques.
EDAC was found to be an excellent intra-bleb cross-linking agent in that it cross-linked LOS to OMP sufficiently for improved LOS T-dependent immunogenicity, but did not cross link it to such a high degree that problems such as poor filterability, aggregation and inter-bleb cross-linking occurred. The morphology of the blebs generated is similar to that of unconjugated blebs (by electron microscope). In addition, the above protocol avoided an overly high cross-linking to take place (which can decrease the immunogenicity of protective OMPs naturally present on the surface of the bleb e.g. TbpA or Hsf).

It is preferred that the meningococcal strain from which the blebs are derived is a mutant strain that cannot produce capsular polysaccharide (e.g. one of the mutant strains described below, in particular siaD⁻). It is also preferred that immunogenic compositions effective against meningococcal disease comprise both an L2 and and L3 bleb, wherein the L2 and L3 LOS are both conjugated to bleb outer membrane proteins. Furthermore, it is preferred that the LOS structure within the intra-bleb conjugated bleb is consistent with it having been derived from an lgtB⁻ meningococcal strain (as described below). Most preferably immunogenic compositions comprise intrableb-conjugated blebs: derived from a mutant meningococcal strain that cannot produce capsular polysaccharide and is lgtB⁻; comprising L2 and L3 blebs derived from mutant meningococcal strains that cannot produce capsular polysaccharide; comprising L2 and L3 blebs derived from mutant meningococcal strains that are lgtB⁻; or most preferably comprising L2 and L3 blebs derived from mutant meningococcal strains that cannot produce capsular polysaccharide and are lgtB⁻.

Typical L3 meningococcal strain that can be used for the present invention is H44/76 menB strain. A typical L2 strain is the B16B6 menB strain or the 39E meningococcus type C strain.

As stated above, the blebs of the invention have been detoxified to a degree by the act of conjugation, and need not be detoxified any further, however further detoxification methods may be used for additional security, for instance using blebs derived from a meningococcal strain that is htrB⁻ or msbB⁻ or adding a non-toxic peptide functional equivalent of polymyxin B [a molecule with high affinity to Lipid A] (preferably SEAP 2) to the bleb composition (as described above).

In the above way meningococcal blebs and immunogenic compositions comprising blebs are provided which have as an important antigen LOS which is substantially non-toxic, devoid of autoimmunity problems, has a T-dependent character, is present in its natural environment, and is capable of inducing a bactericidal antibody response against more than 90% of meningococcal strains (in the case of L2+L3 compositions).

Preferably intrableb LOS conjugation should incorporate 1, 2 or all 3 of the following process steps: conjugation pH should be greater than pH 7.0, preferably greater than or equal to pH 7.5 (most preferably under pH 9); conditions of 1-5% preferably 2-4% most preferably around 3% sucrose should be maintained during the reaction; NaCl should be minimised in the conjugation reaction, preferably under 0.1M, 0.05M, 0.01M, 0.005M, 0.001M, and most preferably not present at all. All these process features make sure that the blebs remain stable and in solution throughout the conjugation process.

Although LOS can be conjugated within blebs to outer membrane proteins by various techniques and chemistries, the EDAC/NHS conjugation process is a preferred process for intra-bleb conjugation. EDAC/NHS is preferred to formalydehyde which can cross-link to too high an extent thus adversely affecting filterability. EDAC reacts with carboxylic acids to create an active-ester intermediate. In the presence of an amine nucleophile, an amide bond is formed with release of an isourea by-product. However, the efficiency of an EDAC-mediated reaction may be increased through the formation of a Sulfo-NHS ester intermediate. The Sulfo-NHS ester survives in aqueous solution longer than the active ester formed from the reaction of EDAC alone with a carboxylate. Thus, higher yields of amide bond formation may be realized using this two-stage process. EDAC/NHS conjugation is discussed in J.V. Staros, R.W. Wright and D. M. Swingle. Enhancement by N-hydroxysuccinimide of water-soluble carbodiimide-mediated coupling reactions. Analytical chemistry 156: 220-222 (1986); and Bioconjugates Techniques. Greg T. Hermanson (1996) pp173-176.

A preferred process of the invention is therefore a process for producing intrableb conjugated LOS (preferably meningococcal) comprising the steps of conjugating blebs in the presence of EDAC/NHS at a pH between pH 7.0 and pH 9.0 (preferably around pH 7.5), in 1-5% (preferably around 3%) sucrose, and optionally in conditions substantially devoid of NaCl (as described above), and isolating the conjugated blebs from the reaction mix.

The reaction may be followed on separation gels of the reaction mixture using anti-LOS (e.g. anti-L2 or anti-L3) mAbs to show the increase of LOS molecular weight for a greater proportion of the LOS in the blebs as reaction time goes on.
Yields of 99% blebs can be recovered using such techniques.
EDAC was found to be an excellent intra-bleb cross-linking agent in that it cross-linked LOS to OMP sufficiently for improved LOS T-dependent immunogenicity, but did not cross link it to such a high degree that problems such as poor filterability and inter-bleb cross-linking occurred. A too high cross-linking should also avoided to avoid any decrease in immunogenicity of protective OMPs naturally present on the surface of the bleb e.g. TbpA.

### 5.Integral outer membrane proteins

Other categories of Neisserial proteins may also be candidates for inclusion in the Neisserial vaccines of the invention and may be able to combine with other antigens in a surprisingly effective manner. Membrane associated proteins, particularly integral membrane proteins and most advantageously outer membrane proteins, especially integral outer membrane proteins may be used in the compositions of the present invention. An example of such a protein is PIdA also known as Omp1A (NMB 0464) (WO00/15801) which is a Neisserial phospholipase outer membrane protein. Further examples are TspA (NMB 0341) (Infect. Immun. 1999, 67; 3533-3541) and TspB (T-cell stimulating protein) (WO 00/03003; NMB 1548, NMB 1628 or NMB 1747). Further examples include PilQ (NMB 1812) (WO99/61620), OMP85 - also known as D15- (NMB 0182) (WO00/23593), NspA (U52066) (WO96/29412), FhaC (NMB 0496 or NMB 1780), PorB (NMB 2039) (Mol. Biol. Evol. 12; 363-370, 1995), HpuB (NC_003116.1), TdfH (NMB 1497) (Microbiology 2001, 147; 1277-1290), OstA (NMB 0280), M1tA also known as GNA33 and Lipo30 (NMB0033), HtrA (NMB 0532; WO 99/55872), HimD (NMB 1302), HisD (NMB 1581), GNA 1870 (NMB 1870), H1pA (NMB 1946), NMB 1124, NMB 1162, NMB 1220, NMB 1313, NMB 1953, HtrA, TbpA (NMB 0461) (WO92/03467) (see also above under iron acquisition proteins) and LbpA (NMB 1541).

### OMP85

Immunogenic compositions of the invention may comprise the full length OMP85, preferably as part of an OMV preparation. Fragments of OMP85 may also be used in immunogenic compositions of the invention, in particularly, the surface exposed domain of OMP85 made up of amino acid residues 1-475 or 50-475 is preferably incorporated into a subunit component of the immunogenic compositions of the invention. The above sequence for the surface exposed domain of OMP85 can be extended or truncated by up to 1, 3, 5, 7, 10, 15, 20, 25, or 30 amino acids at either or both N or C termini. It is preferred that the signal sequence is omitted from the OMP85 fragment.

### OstA

OstA functions in the synthesis of lipopolysaccharides and may be considered to be a regulator of toxicity. OstA may alternatively be included in the toxin category where the toxin category is broadened to contain regulators of toxicity as well as toxins.

### Immunogenic compositions

An immunogenic composition is a composition comprising at least one antigen which is capable of generating an immune response when administered to a host. Preferably, such immunogenic preparations are capable of generating a protective immune response against Neisserial, preferably *Neisseria meningitidis or Neisseria gonorrhoeae* infection.

The invention relates to immunogenic compositions comprising at least two antigens, which preferably elicit one or more of a synergistic bactericidal, protective, or adhesion blocking response.

### SBA bactericidal assays of the invention

Such a synergistic response may be characterised by the SBA elicited by the combination of antigens being at least 50%, two times, three times, preferably four times, five times, six times, seven times, eight times, nine times and most preferably ten times higher than the SBA elicited by each antigen separately. Preferably SBA is measured against a homologous strain from which the antigens are derived and preferably also against a panel of heterologous strains. (See below for a representative panel for instance BZ10 (B:2b:P1.2) belonging to the A-4 cluster; B16B6 (B:2a:P1.2) belonging to the ET-37 complex; and H44/76 (B:15:P1.7,16)). SBA is the most commonly agreed immunological marker to estimate the efficacy of a meningococcal vaccine (Perkins et al. J Infect Dis. 1998, 177:683-691). Satisfactory SBA can be acertained by any known method. SBA can be carried out using sera obtained from animal models (see examples 17-20), or from human subjects.

A preferred method of conducting SBA with human sera is the following. A blood sample is taken prior to the first vaccination, two months after the second vaccination and one month after the third vaccination (three vaccinations in one year being a typical human primary vaccination schedule administered at, for instance, 0, 2 and 4 months, or 0, 1 and 6 months). Such human primary vaccination schedules can be carried out on infants under 1 year old (for instance at the same time as Hib vaccinations are carried out) or 2-4 year olds or adolescents may also be vaccinated to test SBA with such a primary vaccination schedule. A further blood sample may be taken 6 to 12 months after primary vaccination and one month after a booster dose, if applicable.

SBA will be satisfactory for an antigen or bleb preparation with homologous bactericidal activity if one month after the third vaccine dose (of the primary vaccination schedule) (in 2-4 year olds or adolescents, but preferably in infants in the first year of life) the percentage of subjects with a four-fold increase in terms of SBA (antibody dilution) titre (compared with pre-vaccination titre) against the strain of meningococcus from which the antigens of the invention were derived is greater than 30%, preferably greater than 40%, more preferably greater than 50%, and most preferably greater than 60% of the subjects.

Of course an antigen or bleb preparation with heterologous bactericidal activity can also constitute bleb preparation with homologous bactericidal activity if it can also elicit satisfactory SBA against the meningococcal strain from which it is derived.

SBA will be satisfactory for an antigen or bleb preparation with heterologous bactericidal activity if one month after the third vaccine dose (of the primary vaccination schedule) (in 2-4 year olds or adolescents, but preferably in infants in the first year of life) the percentage of subjects with a four-fold increase in terms of SBA (antibody dilution) titre (compared with pre-vaccination titre) against three heterologous strains of meningococcus is greater than 20%, preferably greater than 30%, more preferably greater than 35%, and most preferably greater than 40% of the subjects. Such a test is a good indication of whether the antigen or bleb preparation with heterologous bactericidal activity can induce cross-bactericidal antibodies against various meningococcal strains. The three heterologous strains should preferably have different electrophoretic type (ET)-complex or multilocus sequence typing (MLST) pattern (see Maiden et al. PNAS USA 1998, 95:3140-5) to each other and preferably to the strain from which the antigen or bleb preparation with heterologous bactericidal activity is made or derived. A skilled person will readily be able to determine three strains with different ET-complex which reflect the genetic diversity observed amongst meningococci, particularly amongst meningococcus type B strains that are recognised as being the cause of significant disease burden and/or that represent recognised MenB hyper-virulent lineages (see Maiden et al. *supra*). For instance three strains that could be used are the following: BZ10 (B:2b:P1.2) belonging to the A-4 cluster; B16B6 (B:2a:P1.2) belonging to the ET-37 complex; and H44/76 (B:15:P1.7,16) belonging to the ET-5 complex, or any other strains belonging to the same ET/Cluster. Such strains may be used for testing an antigen or bleb preparation with heterologous bactericidal activity made or derived from, for instance, meningococcal strain CU385 (B:4:P1.15) which belongs to the ET-5 complex. Another sample strain that could be used is from the Lineage 3 epidemic clone (e.g. NZ124 [B:4:P1.7,4]). Another ET-37 strain is NGP165 (B:2a:P1.2).

Processes for measuring SBA activity are known in the art. For instance a method that might be used is described in WO 99/09176 in Example 10C. In general terms, a culture of the strain to be tested is grown (preferably in conditions of iron depletion - by addition of an iron chelator such as EDDA to the growth medium) in the log phase of growth. This can be suspended in a medium with BSA (such as Hanks medium with 0.3% BSA) in order to obtain a working cell suspension adjusted to approximately 20000 CFU/ml. A series of reaction mixes can be made mixing a series of two-fold dilutions of sera to be tested (preferably heat-inactivated at 56°C for 30 min) [for example in a 50µl/well volume] and the 20000 CFU/ml meningococcal strain suspension to be tested [for example in a 25µl/well volume]. The reaction vials should be incubated (e.g. 37°C for 15 minutes) and shaken (e.g. at 210 rpm). The final reaction mixture [for example in a 100µl volume] additionally contains a complement source [such as 25 % final volume of pretested baby rabbit serum], and is incubated as above [e.g. 37°C for 60 min]. A sterile polystyrene U-bottom 96-well microtiter plate can be used for this assay. A aliquot [e.g. 10 µl] can be taken from each well using a multichannel pipette, and dropped onto Mueller-Hinton agar plates (preferably containing 1 % Isovitalex and 1 % heat-inactivated Horse Serum) and incubated (for example for 18 hours at 37°C in 5 % CO₂). Preferably, individual colonies can be counted up to 80 CFU per aliquot. The following three test samples can be used as controls: buffer + bacteria + complement; buffer + bacteria + inactivated complement; serum + bacteria + inactivated complement. SBA titers can be straightforwardly calculated using a program which processes the data to give a measurement of the dilution which corresponds to 50 % of cell killing by a regression calculation.

### Animal protection assays

Alternatively, the synergistic response may be characterised by the efficacy of the combination of antigens in an animal protection assay. For instance, the assays described in example 12 or 13 may be used. Preferably the number of animals protected by the combination of antigens is significantly improved compared with using the antigens by themselves, particularly at suboptimal doses of antigen.

A successful vaccine for the prevention of infection by *N. gono* may require more than one of the following elements: generation of serum and/or mucosal antibodies to facilitate complement mediated killing of the gonococcus, and/or to enhance phagocytosis and microbial killing by leukocytes such as polymorphonuclear leukocytes, and/or to prevent attachment of the gonococci to the host tissues; induction of a cell mediated immune response may also participate to protection.

The improvement of efficacy of a bleb gono vaccine preparation of the invention can be evaluated by analyzing the induced immune response for serum and/or mucosal antibodies that have antiadherence, and/or opsonizing properties, and/or bactericidal activity, as described by others (McChesney D et al, Infect. Immun. 36: 1006, 1982; Boslego J et al: Efficacy trial of a purified gonococcl pilus vaccine, in Program and Abstracts of the 24th Interscience Conference on Antimicrobial Agents and Chemotherapy, Washington, American Society for Microbiology, 1984; Siegel M et al, J. Infect. Dis 145: 300, 1982; de 1a Pas, Microbiology, 141 (Pt4): 913-20, 1995).
A mouse model of genital infection by *N. gono* has recently been described (Plante M, J. Infect. Dis., 182: 848-55, 2000). The improvement of efficiency of a bleb gono vaccine of the invention could also be evaluated by its ability to prevent or to reduce colonization by *N. gono* in this mouse model of infection.

### Adhesion blocking assay

Alternatively, the synergisic response may be characterised by the efficacy of the combination of anigens in an adhesion blocking assay. For instance, the assay described in example 11 may be used. Preferably the extent of blocking induced by antisera raised against the combination of antigens is significantly improved compared with using antisera raised against the antigens by themselves, particularly at suboptimal doses of antibody.

### Subunit compositions

The immunogenic composition of the invention may be a subunit composition.

Subunit compositions are compositions in which the components have been isolated and purified to at least 50%, preferably at least 60%, 70%, 80%, 90% pure before mixing the components to form the antigenic composition.

The immunogenic subunit composition of the invention preferably comprises at least 2 antigens selected from the following list: FhaB, PilC, Hsf, Hap, NadA, OMP85, IgA protease, AspA, passenger domain of AspA, passenger domain of Hsf, passenger domain of Hap, FrpA, FrpC, TbpA, TbpB, LbpA, LbpB, HpuA, HpuB, TspA, TspB, P1dA, PilQ, FhaC, NspA, and either or both of LPS immunotype L2 and LPS immunotype L3.

Subunit compositions may be aqueous solutions of water soluble proteins. They may comprise detergent, preferably non-ionic, zwitterionic or ionic detergent in order to solubilise hydrophobic portions of the antigens. They may comprise lipids so that liposome structures could be formed, allowing presentation of antigens with a structure that spans a lipid membrane.

### Outer membrane vesicle preparations

*N. meningitidis* serogroup B (menB) excretes outer membrane blebs in sufficient quantities to allow their manufacture on an industrial scale. An outer membrane vesicles may also be prepared via the process of detergent extraction of the bacterial cells (see for example EP 11243).

The immunogenic composition of the invention may also comprise an outer membrane vesicle preparation having at least two antigens which have been upregulated, either recombinantly or by other means including growth under iron-depleted conditions. Examples of antigens which would be upregulated in such a outer membrane vesicle preparation include; NspA, Hsf, Hap, OMP85, TbpA (high), TbpA (low), LbpA, TbpB, LbpB, PilQ, AspA, TdfH, PorB, HpuB, P2086, NM-ADPRT, MafA, MafB and P1dA. Such preparations would optionally also comprise either or both of LPS immunotype L2 and LPS immunotype L3.

The manufacture of bleb preparations from Neisserial strains may be achieved by any of the methods well known to a skilled person. Preferably the methods disclosed in EP 301992, US 5,597,572, EP 11243 or US 4,271,147, Frederikson et al. (NIPH Annals [1991], 14:67-80), Zollinger et al. (J. Clin. Invest. [1979], 63:836-848), Saunders et al. (Infect. Immun. [1999], 67:113-119), Drabick et al. (Vaccine [2000], 18:160-172) or WO 01/09350 (Example 8) are used. In general, OMVs are extracted with a detergent, preferably deoxycholate, and nucleic acids are optionally removed enzymatically. Purification is achieved by ultracentrifugation optionally followed by size exclusion chromatography. If 2 or more different blebs of the invention are included, they may be combined in a single container to form a multivalent preparation of the invention (although a preparation is also considered multivalent if the different blebs of the invention are separate compositions in separate containers which are administered at the same time [the same visit to a practitioner] to a host). OMV preparations are usually sterilised by filtration through a 0.2 µm filter, and are preferably stored in a sucrose solution (e.g. 3%) which is known to stabilise the bleb preparations.

Upregulation of proteins within outer membrane vesicle preparations may be achieved by insertion of an extra copy of a gene into the Neisserial strain from which the OMV preparation is derived. Alternatively, the promoter of a gene can be exchanged for a stronger promoter in the Neisserial strain from which the OMV preparation is derived. Such techniques are described in WO01/09350. Upregulation of a protein will lead to a higher level of protein being present in OMV compared to the level of protein present in OMV derived from unmodified *N. meningitidis* (for instance strain H44/76). Preferably the level will be 1.5, 2, 3, 4, 5, 7, 10 or 20 times higher.

Where LPS is intended to be an additional antigen in the OMV, a protocol using a low concentration of extracting detergent ( for example deoxycholate or DOC) may preferably be used in the OMV preparation method so as to preserve high levels of bound LPS whilst removing particularly toxic, poorly bound LPS. The concentration of DOC used is preferably 0-0.5% DOC, 0.02-0.4% DOC, 0.04-0.3% DOC more preferably 0.06%-0.2% DOC or 0.08-0.15% DOC most preferably around or exactly 0.1% DOC.

"Stronger promoter sequence" refers to a regulatory control element that increases transcription for a gene encoding antigen of interest.
"Upregulating expression" refers to any means to enhance the expression of an antigen of interest, relative to that of the non-modified (i.e., naturally occurring) bleb. It is understood that the amount of 'upregulation' will vary depending on the particular antigen of interest but will not exceed an amount that will disrupt the membrane integrity of the bleb. Upregulation of an antigen refers to expression that is at least 10% higher than that of the non-modified bleb. Preferably it is at least 50% higher. More preferably it is at least 100% (2 fold) higher. Most preferably it is 3, 4, 5, 7, 10, 20 fold higher. Alternatively or additionally, upregulating expression may refer to rendering expression non-conditional on metabolic or nutritional changes, particularly in the case of TbpA, TbpB, LbpA and LbpB. Preferably the level of expression is assessed when blebs have been derived from bacteria grown in iron limited conditions (for instance in the presence of an iron chelator).

Again for the purpose of clarity, the terms 'engineering a bacterial strain to produce less of said antigen' or down regulation refers to any means to reduce the expression of an antigen (or the expression of a functional gene product) of interest, relative to that of the non-modified (i.e., naturally occurring bleb), preferably by deletion, such that expression is at least 10% lower than that of the non-modified bleb. Preferably it is at least 50% lower and most preferably completely absent. If the down regulated protein is an enzyme or a functional protein, the downregulation may be achieved by introducing one or more mutations resulting in a 10%, 20%, 50%, 80% or preferably a 100% reduction in enzymatic or functional activity.

The engineering steps required to modulate the expression of Neisserial proteins can be carried out in a variety of ways known to the skilled person. For instance, sequences (e.g. promoters or open reading frames) can be inserted, and promoters/genes can be disrupted by the technique of transposon insertion. For instance, for upregulating a gene's expression, a strong promoter could be inserted via a transposon up to 2 kb upstream of the gene's initiation codon (more preferably 200-600 bp upstream, most preferably approximately 400 bp upstream). Point mutation or deletion may also be used (particularly for down-regulating expression of a gene).

Such methods, however, may be quite unstable or uncertain, and therefore it is preferred that the engineering step is performed via a homologous recombination event. Preferably, the event takes place between a sequence (a recombinogenic region) of at least 30 nucleotides on the bacterial chromosome, and a sequence (a second recombinogenic region) of at least 30 nucleotides on a vector transformed within the strain. Preferably the regions are 40-1000 nucleotides, more preferably 100-800 nucleotides, most preferably 500 nucleotides). These recombinogenic regions should be sufficiently similar that they are capable of hybridising to one another under highly stringent conditions.

Methods used to carry out the genetic modification events herein described (such as the upregulation or downregulation of genes by recombination events and the introduction of further gene sequences into a Neisserial genome) are described in WO01/09350. Typical strong promoters that may be integrated in *Neisseria* are *porA, porB, lgtF,* Opa, *p110, lst,* and *hpuAB.* PorA and PorB are preferred as constitutive, strong promoters. It has been established that the PorB promoter activity is contained in a fragment corresponding to nucleotides -1 to -250 upstream of the initation codon of *porB.*

### Upregulation of expression of antigens by growth in iron limitation media

The upregulation of some antigens in an outer membrane vesicle preparation of the invention is preferably achieved by isolating outer membrane vesicles from a parental strain of Neisseria grown under iron limitation conditions. A low concentration of iron in the medium will result in increased expression of proteins involved in iron acquisition including TbpA, TbpB, LbpA, LbpB, HpuA, HpuB and P2086. The expression of these proteins is thereby upregulated without the need for recombinantly modifying the gene involved, for instance by inserting a stronger promoter or inserting an additional copy of the gene. The invention would also encompass upregulation of iron acquisition proteins by growth in iron limitation medium where the gene has also been recombinantly modified.

Iron limitation is achieved by the addition of an iron chelator to the culture medium. Suitable iron chelators include 2,2-Dipyridil, EDDHA (ethylenediamine-di(o-hydroxyphenylacetic acid) and Desferal (deferoxamine mesylate, Sigma). Desferal is the preferred iron chelator and is added to the culture medium at a concentration of between 10 and 100µM, preferably 25-75µM, more preferably 50-70µM, most preferably at 60µM. The iron content of medium comes primarily from the yeast extract and soy peptone constituents and the amount present may vary between batches. Therefore different concentrations of Desferal may be optimal to achieve upregulation of iron acquisition proteins in different batches of medium. The skilled artisan should easily be able to determine the optimal concentration. In basic terms, enough iron chelator should be added to the medium to upregulate the expression of the desired iron-regulated protein, but not so much so as to adversely affect the growth of the bacteria.

Preferably, upregulation of iron acquisition proteins by growth under iron limited conditions is combined with recombinant upregulation of other antigens so that the outer membrane vesicle of the invention is achieved.

### Down regulation/Removal of Variable and non-protective immunodominant antigens

Many surface antigens are variable among bacterial strains and as a consequence are protective only against a limited set of closely related strains. An aspect of this invention covers outer membrane vesicles of the invention in which the expression of other proteins is reduced, or, preferably, gene(s) encoding variable surface protein(s) are deleted. Such deletion results in a bacterial strain producing blebs which, when administered in a vaccine, have a stronger potential for cross-reactivity against various strains due to a higher influence exerted by conserved proteins (retained on the outer membranes) on the vaccinee's immune system. Examples of such variable antigens in Neisseria that may be downregulated in the bleb immunogenic compositions of the invention include PorA, PorB, Opa.

Other types of gene that could be down-regulated or switched off are genes which, *in vivo,* can easily be switched on (expressed) or off by the bacterium. As outer membrane proteins encoded by such genes are not always present on the bacteria, the presence of such proteins in the bleb preparations can also be detrimental to the effectiveness of the vaccine for the reasons stated above. A preferred example to down-regulate or delete is *Neisseria* Opc protein. Anti-Opc immunity induced by an Opc containing bleb vaccine would only have limited protective capacity as the infecting organism could easily become Opc⁻.

For example, these variable or non-protective genes may be down-regulated in expression, or terminally switched off. This has the advantage of concentrating the immune system on better antigens that are present in low amounts on the outer surface of blebs. By down-regulation it is also meant that surface exposed, variable immunodominant loops of the above outer membrane proteins may be altered or deleted in order to make the resulting outer membrane protein less immunodominant.

Methods for downregulation of expression are disclosed in WO01/09350. Preferred combinations of proteins to be downregulated in the bleb immunogenic compositions of the invention include PorA and OpA; PorA and OpC; OpA and OpC; PorA and OpA and OpC.

Four different Opa genes are known to exist in the meningococcal genome (Aho et al. 1991 Mol. Microbiol. 5:1429-37), therefore where Opa is said to be downregulated in expression it is meant that preferably 1, 2, 3 or (preferably) all 4 genes present in meningococcus are so downregulated. Such downregulation may be performed genetically as described in WO 01/09350 or by seeking readily-found, natural, stable meningococcal strains that have no or low expression from the Opa loci. Such strains can be found using the technique described in Poolman et al (1985 J. Med. Micro. 19:203-209) where cells that are Opa⁻ have a different phenotype to cells expressing Opa which can be seen looking at the appearance of the cells on plates or under a microscope. Once found, the strain can be shown to be stably Opa⁻ by performing a Western blot on cell contents after a fermentation run to establish the lack of Opa.

Where upregulation of some antigens in the outer membrane vesicle is achieved by growth under iron limitation conditions, the variable protein FrpB (Microbiology 142; 3269-3274, (1996); J. Bacteriol. 181; 2895-2901 (1999)) will also be upregulated. The inventors have found that it is advantageous to downregulate expression of FrpB under these circumstances by downregulating expression of the entire protein as described in WO01/09350 or by deleting variable region(s) of FrpB. This will ensure that the immune response elicited by the immunogenic composition is directed towards antigens that are present in a wide range of strains. Down regulation of FrpB is preferably combined with down regulation of PorA and OpA; PorA and OpC; OpA and OpC; PorA and OpA and OpC in the bleb immunogenic compositions of the invention.

In an alternative embodiment of the invention, FrpB is downregulated in outer membrane vesicles which have been prepared from Neisseria strains not grown under iron limitation conditions.

### Detoxification of LPS

The blebs in the immunogenic compositions of the invention may be detoxified via methods for detoxification of LPS which are disclosed in WO01/09350. In particular methods for detoxification of LPS of the invention involve the downregulation/deletion of htrB and/or msbB enzymes which are disclosed in WO01/09350. The msbB and htrB genes of Neisseria are also called 1pxL1 and lpxL2 , respectively (WO 00/26384) and deletion mutationsof these genes are characterised pnenoltypically by the msbB- mutant LOS losing one secondary acyl chain), and the htrB- mutatn LOS losing both secondary acyl chains. WO93/14155 and WO 95/03327 describe nontoxix peptide functional equivalents of polymycin B that may be used in compositions of the invention.

Such methods are preferably combined with methods of bleb extraction involving low levels of DOC, preferably 0-0.3% DOC, more preferably 0.05%-0.2% DOC, most preferably around or exactly 0.1 % DOC.

Further methods of LPS detoxification include adding to the bleb preparations a non-toxic peptide functional equivalent of polymyxin B (preferably SAEP 2) as described above.

### Cross-reactive polysaccharides

The isolation of bacterial outer-membrane blebs from encapsulated Gram-negative bacteria often results in the co-purification of capsular polysaccharide. In some cases, this "contaminant" material may prove useful since polysaccharide may enhance the immune response conferred by other bleb components. In other cases however, the presence of contaminating polysaccharide material in bacterial bleb preparations may prove detrimental to the use of the blebs in a vaccine. For instance, it has been shown at least in the case of *N. meningitidis* that the serogroup B capsular polysaccharide does not confer protective immunity and is susceptible to induce an adverse auto-immune response in humans. Consequently, outer membrane vesicles of the invention may be isolated from a bacterial strain for bleb production, which has been engineered such that it is free of capsular polysaccharide. The blebs will then be suitable for use in humans. A particularly preferred example of such a bleb preparation is one from *N. meningitidis* serogroup B devoid of capsular polysaccharide.

This may be achieved by using modified bleb production strains in which the genes necessary for capsular biosynthesis and/or export have been impaired. Inactivation of the gene coding for capsular polysaccharide biosynthesis or export can be achieved by mutating (point mutation, deletion or insertion) either the control region, the coding region or both (preferably using the homologous recombination techniques described above), or by any other way of decreasing the enzymatic function of such genes. Moreover, inactivation of capsular biosynthesis genes may also be achieved by antisense over-expression or transposon mutagenesis. A preferred method is the deletion of some or all of the *Neisseria meningitidis cps* genes required for polysaccharide biosynthesis and export. For this purpose, the replacement plasmid pMF121 (described in Frosh et a1.1990, Mol. Microbiol. 4:1215-1218) can be used to deliver a mutation deleting the *cpsCAD* (+ *galE*) gene cluster.

The safety of antibodies raised to L3 or L2 LPS has been questioned, due to the presence of a structure similar to the lacto-N-neotetraose oligosaccharide group (Ga1βl-4G1cNAcβ1-3Ga1β1-4G1cβ1-) present in human glycosphingolipids. Even if a large number of people has been safely vaccinated with deoxycholate extracted vesicle vaccines containing residual amount of L3 LPS (G. Bjune et al, Lancet (1991), 338, 1093-1096; GVG. Sierra et al, NIPH ann (1991), 14, 195-210), the deletion of the terminal part of the LOS saccharidic is advantageous in preventing any cross-reaction with structures present at the surface of human tissues. In a preferred embodiment, inactivation of the *lgtB* gene results in an intermediate LPS structure in which the terminal galactose residue and the sialic acid are absent ( the mutation leaves a 4G1cNAcβ1-3Ga1β1-4G1cβ1- structure in L2 and L3 LOS). Such intermediates could be obtained in an L3 and an L2 LPS strain. An alternative and less preferred (short) version of the LPS can be obtained by turning off the *lgtE* gene. A further alternative and less preferred version of the LPS can be obtained by turning off the lgtA gene. If such an lgtA⁻ mutation is selected it is preferred to also turn off lgtC expression to prevent the non-immunogenic L1 immunotype being formed.

LgtB⁻ mutants are most preferred as the inventors have found that this is the optimal truncation for resolving the safety issue whilst still retaining an LPS protective oligosaccharide epitope that can still induce a bactericidal antibody response.

Therefore, immunogenic compositions of the invention further comprising L2 or L3 preparations (whether purified or in an isolated bleb) or meningococcal bleb preparations in general are advantageously derived from a Neisserial strain (preferably meningococcal) that has been genetic engineered to permanently downregulate the expression of functional gene product from the lgtB, lgtA or lgtE gene, preferably by switching the gene off, most preferably by deleting all or part of the promoter and/or open-reading frame of the gene.

Where the above immunogenic compositions of the invention are derived from a meningococcus B strain, it is further preferred that the capsular polysaccharide (which also contains human-like saccharide structures) is also removed. Although many genes could be switched off to achieve this, the inventors have advantageously shown that it is preferred that the bleb production strain has been genetically engineered to permanently downregulate the expression of functional gene product from the siaD gene (i.e. downregulating α-2-8 polysialyltransferase activity), preferably by switching the gene off, most preferably by deleting all or part of the promoter and/or open-reading frame of the gene. Such an inactivation is described in WO 01/09350. The siaD (also known as synD) mutation is the most advantageous of many mutations that can result in removing the human-similar epitope from the capsular polysaccharide, because it one of the only mutations that has no effect on the biosynthesis of the protective epitopes of LOS, thus being advantageous in a process which aims at ultimately using LOS as a protective antigen, and has a minimal effect on the growth of the bacterium. A preferred aspect of the invention is therefore a bleb immunogenic preparation as described above which is derived from an lgtE⁻ siaD⁻, an lgtA⁻ siaD⁻ or, preferably, an lgtB⁻ siaD⁻ meningococcus B mutant strain. The strain itself is a further aspect of the invention.

Although siaD⁻ mutation is preferable for the above reasons, other mutations which switch off meningococcus B capsular polysaccharide synthesis may be used. Thus bleb production strain can be genetically engineered to permanently downregulate the expression of functional gene product from one or more of the following genes: ctrA, ctrB, ctrC, ctrD, synA (equivalent to synX and siaA), synB (equivalent to siaB) or synC (equivalent to siaC) genes, preferably by switching the gene off, most preferably by deleting all or part of the promoter and/or open-reading frame of the gene. The lgtE⁻ mutation may be combined with one or more of these mutations. Preferably the lgtB⁻ mutation is combined with one or more of these mutations. A further aspect of the invention is therefore a bleb immunogenic preparation as described above which is derived from such a combined mutant strain of meningococcus B. The strain itself is a further aspect of the invention.

A Neisserial locus containing various *lgt* genes, including lgtB and lgtE, and its sequence is known in the art (see M. P. Jennings et al, Microbiology 1999, 145, 3013-3021 and references cited therein, and J. Exp. Med. 180:2181-2190 [1994]).

Where full-length (non-truncated) LOS is to be used in the final product, it is desirable for LOS not to be sialyated (as such LOS generates an immune response against the most dangerous, invasive meningococcal B strains which are also unsialylated). In such case using a capsule negative strain which has a deleted synA (equivalent to synX and siaA), synB (equivalent to siaB) or synC (equivalent to siaC) gene is advantageous, as such a mutation also renders menB LOS incapable of being sialylated.

In bleb preparations, particularly in preparations extracted with low DOC concentrations LPS may be used as an antigen in the immunogenic composition of the invention. It is however advantageous to downregulate/delete/inactivate enzymatic function of either the lgtE, lgtA (particularly in combination with lgtC), or, preferably, lgtB genes/gene products in order to remove human like lacto-N-neotetraose structures. The Neisserial locus (and sequence thereof) comprising the lgt genes for the biosynthesis of LPS oligosaccharide structure is known in the art (Jennings et al Microbiology 1999 145; 3013-3021 and references cited therein, and J. Exp. Med. 180:2181-2190 [1994]). Downregulation/deletion of lgtB (or functional gene product) is preferred since it leaves the LPS protective epitope intact.

In *N. meningitidis* serogroup B bleb preparations of the invention, the downregulation/deletion of both siaD and lgtB is preferred, (although a combination of lgtB⁻ with any of ctrA⁻, ctrB⁻, ctrC⁻, ctrD⁻, synA⁻ (equivalent to synX⁻ and siaA⁻), synB⁻ (equivalent to siaB⁻) or synC⁻ (equivalent to siaC⁻) in a meningococcus B bleb production strain may also be used) leading to a bleb preparation with optimal safety and LPS protective epitope retention.

A further aspect of the invention is therefore a bleb immunogenic preparation as described above which is derived from such a combined mutant strain of meningococcus B. The strain itself is a further aspect of the invention.

Immunogenic composition of the invention may comprise at least, one, two, three, four or five different outer membrane vesicle preparations. Where two or more OMV preparations are included, at least one antigen of the invention is upregulated in each OMV. Such OMV preparations may be derived from Neisserial strains of the same species and serogroup or preferably from Neisserial strains of different class, serogroup, serotype, subserotype or immunotype. For example, an immunogenic composition may comprise one or more outer membrane vesicle preparation(s) which contains LPS of immunotype L2 and one or more outer membrane vesicle preparation which contains LPS of immunotype L3. L2 or L3 OMV preparations are preferably derived from a stable strain which has minimal phase variability in the LPS oligosaccharide synthesis gene locus.

### Outer membrane vesicles combined with subunit compositions

The immunogenic compositions of the invention may also comprise both a subunit composition and an outer membrane vesicle. There are several antigens that are particularly suitable for inclusion in a subunit composition due to their solubility. Examples of such proteins include; FhaB, NspA, passenger domain of Hsf, passenger domain of Hap, passenger domain of AspA, AspA, OMP85, FrpA, FrpC, TbpB, LbpB, PilQ. The outer membrane vesicle preparation would have at least one different antigen selected from the following list which has been recombinantly upregulated in the outer membrane vesicle: NspA, Hsf, Hap, OMP85, TbpA (high), TbpA (low), LbpA, TbpB, LbpB, NadA, TspA, TspB, PilC, PilQ, TdfH, PorB, HpuB, P2086, NM-ADPRT, MafA, MafB and P1dA; and optionally comprise either or both of LPS immunotype L2 and LPS immunotype L3.

### Specific immunogenic compositions of the invention

In the specific combinations listed below, where combinations of antigens are present in a bleb, such combinations of antigens should be upregulated as descibed above.

A particularly preferred embodiment of the invention comprises an autotransporter protein and an iron acquisition protein, more preferably Hsf and TbpA (high) and/or TbpA (low). Such immunogenic compositions may more preferably further comprise at least one of OMP 85, FrpA, FrpC, LbpA, LbpB, Lipo28, Sibp, NMB0964, NMB0293, TspA, NadA, TspB, PilQ, FhaC, NspA, PldA, HimD, HisD, GNA1870, OspA, HlpA, FhaB, PilC, Omp26, NMB0315, NMB0995, NMB 1119, TdfH, PorB, HpuB, P2086, NM-ADPRT, VapD and Hap. All the above immunogenic compositions may further comprise either or both of LPS immunotype L2 and LPS immunotype L3.

A further preferred embodiment of the invention comprises Hsf and at least one further antigen selected form the group consisting of FrpA, FrpC,NM-ADPRT, VapD, LbpB, LbpA, TbpB, TbpA, P2086, HpuA, HpuB, Lipo28, Sibp, Hap, AspA, IgA protease, OMP85, NspA, PilQ, HimD, HisD, GNA1870, OspA, HlpA, FhaC, NadA, PldA, TspA, TspB, TdfH, PorB and FhaB. All the above immunogenic compositions may further comprise either or both of LPS immunotype L2 and LPS immunotype L3. Preferred combinations comprise Hsf and OMP85 (optionally with one or more of Hap, FrpA or LbpB); Hsf and Hap(optionally with one or more of FrpA, LbpB or OMP85); Hsf and FrpA (optionally with one or more of Hap, LbpB or OMP85); Hsf and LbpB (optionally with one or more of Hap, OMP85 or FrpA). In as much as Hsf is an adhesin and an autotransporter protein, a particularly preferred combination comprises Hsf, OMP85, TbpA, LPS immunotype L2 and/or L3, preferably in a multivalent bleb preparation, which has members of all five groups of antigens represented. Preferably both TbpA(low) and TbpA(high) are present.

A further immunogenic composition of the invention comprises FhaB and at least one further antigen selected from the group consisting of FrpA, FrpC, NM-ADPRT, VapD, LbpB, LbpA, TbpB, HpuA, HpuB, P2086, Lipo28, Sibp, NMB0964, NMB0293, TdfH, PorB, PldA, Hap, IgA protease, AspA, PilQ, HimD, HisD, GNA1870, OspA, HlpA, OMP85, NspA, PilC, Omp26, NMB0315, NMB0995, NMB1119, NadA, PldA, TbpA, Hsf, TspA and TspB, and either or both of LPS immunotype L2 and LPS immunotype L3. Preferred combinations comprise FhaB and Hsf (optionally with one or more of OMP85, LbpB, Hap or FrpA); FhaB and OMP85 (optionally with one or more of LbpB, Hap or FrpA); FhaB and LbpB (optionally with one or more of Hap or FrpA); FhaB and Hap (optionally with FrpA). A prefered combination comprises FhaB, LbpB, Hsf (as an OMP) and FrpA which has members of all five groups of antigen represented.

A further immunogenic composition of the invention comprises NspA and at least one further antigen selected from the group consisting of FrpA, FrpC, NM-ADPRT, VapD, LbpB, LbpA, TbpB, TbpA, HpuA, HpuB, P2086, Lipo28, Sibp, NMB0964, NMB0293, Hap, OMP85, PilQ, AspA, IgA protease, NadA, PldA, Hsf, Hap, TspA, TspB, TdfH, PorB, and either or both of LPS immunotype L2 and LPS immunotype L3. Preferred combinations comprise NspA and Hsf (optionally with one or more of OMP85, Hap, LbpA or TbpA); NspA and OMP85 (optionally with one or more of Hap, LbpA or TbpA); NspA and Hap (optionally with one or more of LbpA or TbpA); NspA and LbpA (optionally with TbpA). A particularly preferred combination comprises NspA, Hsf, TbpA, LPS immunotype L2 and/or L3, preferably in a multivalent bleb preparation, which has members of all five groups of antigens represented. Preferably both TbpA(low) and TbpA(high) are present.

Immunogenic compositins with individualised combinatins of antigens disclosed in WO 00/25811 are not claimed in this invention. Preferably, immunogenic compositions or vaccines are not covered by the present invention if they have an antigen content consisting solely of transferrin binding protein and NspA (or in the case of a bleb vaccine, have an upregulated or enriched antigen content consisting solely of transferrin binding protein and NspA), however specific combinations of antigens (or upregulated antigens) consisting of or including NspA as well as both TbpA(high) and TbpA (low) may be included. Optionally, compositions or vaccines comprising a combination (subunit) or upregulation (bleb) of transferrin binding protein and NspA are not claimed.

A further immunogenic composition of the invention comprises NadA and at least one further antigen selected from the group consisting of FrpA, FrpC, NM-ADPRT, VapD, LbpB, LbpA, TbpB, TbpA, P2086, Lipo28, Sibp, NMB0964, NMB0293, Hap, OMP85, NspA, PilQ, HimD, HisD, GNA1870, OspA, HlpA, HpuA, HpuB, AspA, IgA protease, PldA, Hsf, TspA, TspB, TdfH, PorB, and either or both of LPS immunotype L2 and LPS immunotype L3.

A further immunogenic composition of the invention comprises TbpA (low) and at least one further antigen selected from the group consisting of FrpA, FrpC, NM-ADPRT, VapD, LbpB, LbpA, TbpB, IgA protease, NspA, HpuA, HpuB, Hap, OMP85, NspA (when further combined with TbpA(high)), PilQ, HimD, HisD, GNA1870, OspA, HlpA, PilC,, Omp26, NMB0315, NMB0995, NMB1119, MafA, MafB, AspA, NadA, PldA, Hsf, TspA, TspB, TdfH, PorB and FhaB, and either or both of LPS immunotype L2 and LPS immunotype L3. Preferred combinations comprise TbpA(low) and Hsf and LbpA; TbpA(low) and OMP85 (optionally with either or both of LbpA and Hap); TbpA(low) and LbpA and Hap.

A further immunogenic composition of the invention comprises TbpA (high) and at least one further antigen selected from the group consisting of FrpA, FrpC, NM-ADPRT, VapD, LbpB, LbpA, TbpB, Hap, OMP85, NspA (when further combined with TbpA(low)), PilC, Omp26, NMB0315, NMB0995, NMB 1119, PilQ, HimD, HisD, GNA1870, OspA, HlpA, MafA, MafB, AspA, IgA protease, PldA, FhaB, NadA, PldA, Hsf, TspA, TspB, TdfH, PorB and FhaB, and either or both of LPS immunotype L2 and LPS immunotype L3. Preferred combinations comprise TbpA(high) and Hsf and LbpA; TbpA(high) and OMP85 (optionally with either or both of LbpA and Hap); TbpA(high) and LbpA and Hap.

A further immunogenic composition of the invention comprises LbpA and at least one further antigen selected from the group consisting of FrpA, FrpC, NM-ADPRT, VapD, LbpB, TbpB, Hap, OMP85, NspA, PilC, Omp26, NMB0315, NMB0995, NMB1119, NadA, PldA, TbpA, Hsf, TspA, TspB, MafA, MafB, IgA protease, AspA, FhaB, PilQ, HimD, HisD, GNA1870, OspA, HlpA, TdfH, PorB and FhaB and either or both of LPS immunotype L2 and LPS immunotype L3. Preferred combinations comprise LbpA and Hsf (optionally with Hap).

A further immunogenic composition of the invention comprises LbpB and at least one further antigen selected from the group consisting of FrpA, FrpC, NM-ADPRT, VapD, LbpA, TbpB, Hap, OMP85, NspA, PilC,, Omp26, NMB0315, NMB0995, NMB1119, NadA, PldA, TbpA, Hsf, TspA, TspB, MafA, MafB, IgA protease, AspA, FhaB, PilQ, HimD, HisD, GNA1870, OspA, HlpA, TdfH, PorB and FhaB, and either or both of LPS immunotype L2 and LPS immunotype L3. Preferred combinations comprise LbpB and Hsf (optionally with one or more of OMP85, Hap or FrpA); LbpB and OMP85 (optionally with one or more of Hap or FrpA); LbpB and Hap (optionally with FrpA).

A further immunogenic composition of the invention comprises OMP85 and at least one further antigen selected from the group consisting of FrpA, FrpC, NM-ADPRT, VapD, LbpB, LbpA, TbpB, TbpA, HpuA, HpuB, P2086, Lipo28, Sibp, NMB0964, NMB0293, Hap, IgA protease, AspA, Hsf, NspA, PilC,, Omp26, NMB0315, NMB0995, NMB1119, MafA, MafB, NadA, PldA, Hsf, TspA, TspB, PilQ, TdfH, PorB and FhaB, and either or both of LPS immunotype L2 and LPS immunotype L3. Preferred combinations comprise OMP85 and Hsf (optionally with either or both of LbpA or NspA); OMP85 and LbpA (optionally with either or both of Hap and NspA); OMP85 and Hap (optionally with NspA).

A further immunogenic composition of the invention comprises Hap and at least one further antigen selected from the group consisting of FrpA, FrpC, NM-ADPRT, VapD, LbpB, LbpA, TbpB, TbpA, HpuA, HpuB, P2086, Lipo28, Sibp, NMB0964, NMB0293, PilQ, HimD, HisD, GNA1870, OspA, HlpA, NspA, IgA protease, AspA, OMP85, NspA, PilC, Omp26, NMB0315, NMB0995, NMB1119, MafA, MafB, NadA, PldA, Hsf, TspA, TspB, TdfH, PorB and FhaB, and either or both of LPS immunotype L2 and LPS immunotype L3.

A further immunogenic composition of the invention comprises FrpA and at least one further antigen selected from the group consisting of LbpB, LbpA, TbpA, TbpB, HpuA, HpuB, P2086, Lipo28, Sibp, NMB0964, NMB0293, PilQ, HimD, HisD, GNA1870, OspA, HlpA, TspA, TspB, Hap, IgA protease, AspA, NadA, FhaB, PilQ, HimD, HisD, GNA1870, OspA, HlpA, OMP85, NspA, PilC, Omp26, NMB0315, NMB0995, NMB1119, MafA, MafB, PldA, Hsf, TspA, TspB, TdfH, PorB and FhaB, and either or both of LPS immunotype L2 and LPS immunotype L3.

A further immunogenic composition of the invention comprises FrpC and at least one further antigen selected from the group consisting of LbpB, LbpA, TbpA, TbpB, HpuA, HpuB, P2086, Lipo28, Sibp, NMB0964, NMB0293, PilQ, HimD, HisD, GNA1870, OspA, HlpA, TspA, TspB, Hap, IgA protease, AspA, NadA, FhaB, OMP85, NspA, PilC, Omp26, NMB0315, NMB0995, NMB1119, MafA, MafB, PldA, Hsf, TspA, TspB, TdfH, PorB and FhaB, and either or both of LPS immunotype L2 and LPS immunotype L3.

A further immunogenic composition of the invention comprises either or both of LPS immunotype L2 and LPS immunotype L3 and at least one further antigen selected from the group consisting of LbpB, LbpA, TbpA, TbpB, HpuA, HpuB, P2086, Lipo28, Sibp, NMB0964, NMB0293, PilQ, HimD, HisD, GNA1870, OspA, HlpA, TspA, TspB, Hap, IgA protease, AspA, NadA, FhaB, OMP85, NspA, PilC, Omp26, NMB0315, NMB0995, NMB1119, MafA, MafB, PldA, Hsf, TspA, TspB, TdfH, PorB and FhaB.

Preferred combinations of antigens in an immunogenic composition of the invention include combinations comprising an iron acquisition protein, an autotransporter protein and FhaB; an iron acquisition protein, an autotransporter protein and PilC; an iron acquisition protein, an autotransporter protein and NadA; an iron acquisition protein, an autotransporter protein and FrpA; an iron acquisition protein, an autotransporter protein and PilQ; an iron acquisition protein, an autotransporter protein and TspA; an iron acquisition protein, an autotransporter protein and TspB; an iron acquisition protein, an autotransporter protein and NspA; an iron acquisition protein, an autotransporter protein and FrpC; more preferably comprising an iron acquisition protein, an autotransporter protein and Hap; an iron acquisition protein, an autotransporter protein and FrpA/C; an iron acquisition protein, an autotransporter protein and LbpB; an iron acquisition protein, an autotransporter protein and OMP85 (D15). Most preferably, OMP85 (D15) would be incorporated as part of an outer membrane vesicle preparation.

Immunogenic compositions of the invention which contain LPS will preferably have the LPS conjugated to a source of T-helper epitopes, preferably proteins, and in the case of LPS in OMVs, preferably outer membrane proteins. A particularly preferred embodiment contains LPS which have been (preferably intra-bleb) conjugated to OMP in situ in the outer membrane vesicle preparation (for instance as described above).

The immunogenic compositions of the invention may comprise antigens (proteins, LPS and polysaccharides) derived from *Neisseria meningitidis* serogroups A, B, C, Y, W-135 or *Neisseria gonorrhoeae.*

Preferably the immunogenic compositions or vaccines of the invention do not consist of and/or comprise the particular combinations of SEQ IDs listed in the table spanning from page 3, line 18 to page 52, line 2 of WO 00/71725 and/or any individual combination described in the examples 1-11 of WO 00/71725.

Preferably, any individualised combinations disclosed in WO 01/52885 are not claimed in this invention.

### Further combinations

The immunogenic composition of the invention may further comprise bacterial capsular polysaccharides or oligosaccharides. The capsular polysaccharides or oligosaccharides may be derived from one or more of: *Neisseria meningitidis* serogroup A, C, Y, and/or W-135, *Haemophilus influenzae* b, *Streptococcus pneumoniae,* Group A Streptococci, Group B Streptococci, *Staphylococcus aureus* and *Staphylococcus epidermidis.*

A further aspect of the invention are vaccine combinations comprising the antigenic composition of the invention with other antigens which are advantageously used against certain disease states including those associated with viral or Gram positive bacteria.

In one preferred combination, the antigenic compositions of the invention are formulated with 1, 2, 3 or preferably all 4 of the following meningococcal capsular polysaccharides or oligosaccharides which may be plain or conjugated to a protein carrier: A, C, Y or W-135. Preferably the immunogenic compositions of the invention are formulated with A and C; or C; or C and Y. Such a vaccine containing proteins from *N. meningitidis,* preferably serogroup B may be advantageously used as a global meningococcus vaccine.

In a further preferred embodiment, the antigenic compositions of the invention, preferably formulated with 1, 2, 3 or all 4 of the plain or conjugated meningococcal capsular polysaccharides or oligosaccharides A, C, Y or W-135 (as described above), are formulated with a conjugated *H. influenzae* b capsular polysaccharide or oligosaccharides, and/or one or more plain or conjugated pneumococcal capsular polysaccharides or oligosaccharides. Optionally, the vaccine may also comprise one or more protein antigens that can protect a host against *Streptococcus pneumoniae* infection. Such a vaccine may be advantageously used as a global meningitis vaccine.

In a still further preferred embodiment, the immunogenic composition of the invention is formulated with capsular polysaccharides or oligosaccharides derived from one or more of *Neisseria meningitidis, Haemophilus influenzae* b, *Streptococcus pneumoniae,* Group A Streptococci, Group B Streptococci, *Staphylococcus aureus* or *Staphylococcus epidermidis.* The pneumococcal capsular polysaccharide antigens are preferably selected from serotypes 1, 2, 3, 4, 5, 6B, 7F, 8, 9N, 9V, 10A, 11A, 12F, 14, 15B, 17F, 18C, 19A, 19F, 20, 22F, 23F and 33F (most preferably from serotypes 1, 3, 4, 5, 6B, 7F, 9V, 14, 18C, 19F and 23F). A further preferred embodiment would contain the PRP capsular polysaccharides of *Haemophilus influenzae.* A further preferred embodiment would contain the Type 5, Type 8 or 336 capsular polysaccharides of *Staphylococcus aureus.* A further preferred embodiment would contain the Type I, Type II or Type III capsular polysaccharides of *Staphylococcus epidermidis.* A further preferred embodiment would contain the Type Ia, Type Ic, Type II or Type III capsular polysaccharides of Group B streptocoocus. A further preferred embodiment would contain the capsular polysaccharides of Group A streptococcus, preferably further comprising at least one M protein and more preferably multiple types of M protein.

Such capsular polysaccharides of the invention may be unconjugated or conjugated to a carrier protein such as tetatus toxoid, tetanus toxoid fragment C, diphtheria toxoid, CRM197, pneumolysin, Protein D (US6342224). The polysaccharide conjugate may be prepared by any known coupling technique. For example the polysaccharide can be coupled via a thioether linkage. This conjugation method relies on activation of the polysaccharide with 1-cyano-4-dimethylamino pyridinium tetrafluoroborate (CDAP) to form a cyanate ester. The activated polysaccharide may thus be coupled directly or via a spacer group to an amino group on the carrier protein. Preferably, the cyanate ester is coupled with hexane diamine and the amino-derivatised polysaccharide is conjugated to the carrier protein using heteroligation chemistry involving the formation of the thioether linkage. Such conjugates are described in PCT published application WO93/15760 Uniformed Services University.

The conjugates can also be prepared by direct reductive amination methods as described in US 4365170 (Jennings) and US 4673574 (Anderson). Other methods are described in EP-0-161-188, EP-208375 and EP-0-477508. A further method involves the coupling of a cyanogen bromide activated polysaccharide derivatised with adipic acid hydrazide (ADH) to the protein carrier by Carbodiimide condensation (Chu C. et al Infect. Immunity, 1983 245 256). Where oligosaccharides are included, it is preferred that they be conjugated.

Preferred pneumococcal proteins antigens are those pneumococcal proteins which are exposed on the outer surface of the pneumococcus (capable of being recognised by a host's immune system during at least part of the life cycle of the pneumococcus), or are proteins which are secreted or released by the pneumococcus. Most preferably, the protein is a toxin, adhesin, 2-component signal tranducer, or lipoprotein of *Streptococcus pneumoniae,* or fragments thereof. Particularly preferred proteins include, but are not limited to: pneumolysin (preferably detoxified by chemical treatment or mutation) [Mitchell et al. Nucleic Acids Res. 1990 Jul 11; 18(13): 4010 "Comparison of pneumolysin genes and proteins from Streptococcus pneumoniae types 1 and 2.", Mitchell et al. Biochim Biophys Acta 1989 Jan 23; 1007(1): 67-72 "Expression of the pneumolysin gene in *Escherichia coli*: rapid purification and biological properties.", WO 96/05859 (A. Cyanamid), WO 90/06951 (Paton et al), WO 99/03884 (NAVA)]; PspA and transmembrane deletion variants thereof (US 5804193 - Briles et al.); PspC and transmembrane deletion variants thereof (WO 97/09994 - Briles et al); PsaA and transmembrane deletion variants thereof (Berry & Paton, Infect Immun 1996 Dec;64(12):5255-62 "Sequence heterogeneity of PsaA, a 37-kilodalton putative adhesin essential for virulence of *Streptococcus pneumoniae*"); pneumococcal choline binding proteins and transmembrane deletion variants thereof; CbpA and transmembrane deletion variants thereof (WO 97/41151; WO 99/51266); Glyceraldehyde-3-phosphate - dehydrogenase (Infect. Immun. 1996 64:3544); HSP70 (WO 96/40928); PcpA (Sanchez-Beato et al. FEMS Microbiol Lett 1998, 164:207-14); M like protein, (EP 0837130) and adhesin 18627, (EP 0834568). Further preferred pneumococcal protein antigens are those disclosed in WO 98/18931, particularly those selected in WO 98/18930 and PCT/US99/30390.

The immunogenic composition/vaccine of the invention may also optionally comprise outer membrane vesicle preparations made from other Gram negative bacteria, for example *Moraxella catarrhalis* or *Haemophilus influenzae.*

### Moraxella catarrhalis bleb preparations

Immunogenic compositions of the invention may further comprise OMV preparations derived from *Moraxella catarrhalis.* Engineered OMV preparations can be derived from *Moraxella catarrhalis* as described in WO01/09350. One or more of the following genes (encoding protective antigens) are preferred for upregulation: OMP106 (WO 97/41731 & WO 96/34960), HasR (PCT/EP99/03824), PilQ (PCT/EP99/03823), OMP85 (PCT/EP00/01468), lipo06 (GB 9917977.2), lipo10 (GB 9918208.1), lipo11 (GB 9918302.2), lipo18 (GB 9918038.2), P6 (PCT/EP99/03038), ompCD, CopB (Helminen ME, et al (1993) Infect. Immun. 61:2003-2010), D15 (PCT/EP99/03822), OmplA1 (PCT/EP99/06781), Hly3 (PCT/EP99/03257), LbpA and LbpB (WO 98/55606), TbpA and TbpB (WO 97/13785 & WO 97/32980), OmpE, UspA1 and UspA2 (WO 93/03761), and Omp21. They are also preferred as genes which may be heterologously introduced into other Gram-negative bacteria.

One or more of the following genes are preferred for downregulation: CopB, OMP106, OmpB1, TbpA, TbpB, LbpA, and LbpB.

One or more of the following genes are preferred for downregulation: htrB, msbB and 1pxK.

One or more of the following genes are preferred for upregulation: pmrA, pmrB, pmrE, and pmrF.

### Haemophilus influenzae bleb preparations

Immunogenic compositions of the invention may further comprise OMV preparations derived from *Haemophilus influenzae.* Engineered OMV preparations can be derived from *Haemophilus influenzae* as described in WO01/09350. One or more of the following genes (encoding protective antigens) are preferred for upregulation: D15 (WO 94/12641), P6 (EP 281673), TbpA (WO96/40929; WO95/13370), TbpB (WO96/40929; WO95/13370), P2, P5 (WO 94/26304), OMP26 (WO 97/01638), HMW1, HMW2, HMW3, HMW4, Hia, Hsf, Hap, Hin47, and Hif (all genes in this operon should be upregulated in order to upregulate pilin). They are also preferred as genes which may be heterologously introduced into other Gram-negative bacteria.

One or more of the following genes are preferred for downregulation: P2, P5, Hif, IgA1-protease, HgpA, HgpB, HMW1, HMW2, Hxu, htrB, msbB and IpxK.

One or more of the following genes are preferred for upregulation: pmrA, pmrB, pmrE, and pmrF.

The immunogenic composition/vaccine of the invention may also optionally comprise antigens providing protection against one or more of Diphtheria, tetanus and *Bordetella pertussis* infections. The pertussis component may be killed whole cell *B. pertussis* (Pw) or acellular pertussis (Pa) which contains at least one antigen (preferably 2 or all 3) from PT, FHA and 69kDa pertactin. Typically, the antigens providing protection against Diphtheria and tetanus would be Diphtheria toxoid and tetanus toxoid. The toxoids may chemically inactivated toxins or toxins inactivated by the introduction of point mutations.

The immunogenic composition/vaccine may also optionally comprise one or more antigens that can protect a host against non-typeable *Haemophillus influenzae,* RSV and/or one or more antigens that can protect a host against influenza virus. Such a vaccine may be advantageously used as a global otitis media vaccine.

Preferred non-typeable *H. influenzae* protein antigens include Fimbrin protein (US 5766608) and fusions comprising peptides therefrom (eg LB1 Fusion) (US 5843464 - Ohio State Research Foundation), OMP26, P6, protein D, TbpA, TbpB, Hia, Hmw1, Hmw2, Hap, and D15.

Preferred influenza virus antigens include whole, live or inactivated virus, split influenza virus, grown in eggs or MDCK cells, or Vero cells or whole flu virosomes (as described by R. Gluck, Vaccine, 1992, 10, 915-920) or purified or recombinant proteins thereof, such as HA, NP, NA, or M proteins, or combinations thereof.

Preferred RSV (Respiratory Syncytial Virus) antigens include the F glycoprotein, the G glycoprotein, the HN protein, the M protein or derivatives thereof.

Immunogenic compositions of the invention may include proteins of *Moraxella catarrhalis* include TbpA (WO97/13785; WO99/52947), TbpB (WO97/13785; WO99/52947; Mathers et al FEMS Immunol Med Microbiol 1997 19; 231-236; Myers et al Infect Immun 1998 66; 4183-4192), LbpA, LbpB (Du et al Infect Immun 1998 66; 3656-3665), UspA1, UspA2 (Aebi et al Infect Immun. 1997 65; 4367-4377), OMP106 (US6214981), Ton-B dependent receptor (WO00/78968), CopB (Sethi et al Infect. Immun. 1997 65; 3666-3671), and HasR receptor (WO00/78968); proteins of *Haemophilus influenzae* include HMW (St Geme et al Infect Immun 1998 66; 364-368), Hia (St Geme et al J. Bacteriol. 2000 182; 6005-6013), Tbp1 (WO96/40929; WO95/13370), Tbp2 (WO96/40929; WO95/13370; Gray-Owen et al Infect Immun 1995 63; 1201-1210), LbpA, LbpB (Schryvers et al 1989, 29:121-130), HasR, TonB-dependent receptor (Fleishmann et al Science 1995 269; 496-512), hemoglobin-binding protein, HhuA (Cope et al Infect Immun 2000 68; 4092-4101), HgpA (Maciver et al Infect Immun 1996 64; 3703-3712), HgbA, HgbB and HgbC (Jin et al Infect Immun 1996 64; 3134-3141), HxuA (Cope et al Mol Microbiol 1994 13; 863-873), HxuC (Cope et al Infect Immun 2001 69; 2353-2363); proteins from *Neisseria meningitidis* include Tbp1, Tbp2, FbpA, FbpB, BfrA, BfrB (Tettelin et al Science 2000 287; 1809-1815), LbpA, LbpB and HmbR.

### Vaccine Formulations

A preferred embodiment of the invention is the formulation of the immunogenic composition of the invention in a vaccine which may also comprise a pharmaceutically acceptable excipient or carrier.

The manufacture of outer membrane vesicle preparations from any of the aforementioned modified strains may be achieved by any of the methods well known to a skilled person. Preferably the methods disclosed in EP 301992, US 5,597,572, EP 11243 or US 4,271,147 are used. Most preferably, the method described in WO 01/09350 is used.

Vaccine preparation is generally described in Vaccine Design ("The subunit and adjuvant approach" (eds Powell M.F. & Newman M.J.) (1995) Plenum Press New York).

The antigenic compositions of the present invention may be adjuvanted in the vaccine formulation of the invention. Suitable adjuvants include an aluminium salt such as aluminum hydroxide gel (alum) or aluminium phosphate, but may also be a salt of calcium (particularly calcium carbonate), iron or zinc, or may be an insoluble suspension of acylated tyrosine, or acylated sugars, cationically or anionically derivatised polysaccharides, or polyphosphazenes.

Suitable Th1 adjuvant systems that may be used include, Monophosphoryl lipid A, particularly 3-de-O-acylated monophosphoryl lipid A, and a combination of monophosphoryl lipid A, preferably 3-de-O-acylated monophosphoryl lipid A (3D-MPL) together with an aluminium salt (preferably aluminium phosphate). An enhanced system involves the combination of a monophosphoryl lipid A and a saponin derivative particularly the combination of QS21 and 3D-MPL as disclosed in WO 94/00153, or a less reactogenic composition where the QS21 is quenched with cholesterol as disclosed in WO96/33739. A particularly potent adjuvant formulation involving QS21 3D-MPL and tocopherol in an oil in water emulsion is described in WO95/17210 and is a preferred formulation.

The vaccine may comprise a saponin, more preferably QS21. It may also comprise an oil in water emulsion and tocopherol. Unmethylated CpG containing oligo nucleotides (WO 96/02555) are also preferential inducers of a TH1 response and are suitable for use in the present invention.

The vaccine preparation of the present invention may be used to protect or treat a mammal susceptible to infection, by means of administering said vaccine via systemic or mucosal route. These administrations may include injection *via* the intramuscular, intraperitoneal, intradermal or subcutaneous routes; or *via* mucosal administration to the oral/alimentary, respiratory, genitourinary tracts. Thus one aspect of the present invention is a method of immunizing a human host against a disease caused by infection of a gram-negative bacteria, which method comprises administering to the host an immunoprotective dose of the OMV preparation of the present invention.

The amount of antigen in each vaccine dose is selected as an amount which induces an immunoprotective response without significant, adverse side effects in typical vaccinees. Such amount will vary depending upon which specific immunogen is employed and how it is presented. Generally, it is expected that each dose will comprise 1-100µg of protein antigen or OMV preparation, preferably 5-50µg, and most typically in the range 5 - 25µg.

An optimal amount for a particular vaccine can be ascertained by standard studies involving observation of appropriate immune responses in subjects. Following an initial vaccination, subjects may receive one or several booster immunisations adequately spaced.

The vaccines of the invention are preferably immunoprotective and non-toxic and suitable for paediatric or adolescent use.

By paediatric use it is meant use in infants less than 4 years old.

By immunoprotective it is meant that the SBA and/or animal protection model and/or adhesion blocking assay described above are satisfactorily met.

By non-toxic it is meant that there is no more than a satisfactory level of endotoxin activity in the vaccine as measured by the well-known LAL and pyrogenicity assays.

### Polynucleotides of the invention

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

Another aspect of the invention relates to an immunological/vaccine formulation which comprises one or more polynucleotide(s). Such techniques are known in the art, see for example Wolff et al., Science, (1990) 247: 1465-8.

Such vaccines comprise one or more polynucleotide(s) encoding a plurality of proteins corresponding to protein combinations of the invention described above.

The expression of proteins from such polynucleotides would be under the control of a eukaryotic promoter capable of driving expression within a mammalian cell. The polynucleotide may additionally comprise sequence encoding other antigens. Examples of eukaryotic promoters that could drive the expression include viral promoters from viruses including adenoviral promoters, retroviral promoters. Alternatively, mammalian promoters could be used to drive expression.

### Further aspects of the invention

Another aspect of the invention involves a method for treatment or prevention of Neisserial disease comprising administering a protective dose (or effective amount) of the vaccine of the invention to a host in need thereof. *Neisseria meningitidis* serogroups A, B, C, Y or W135 and/or *Neisseria gonorrhoeae* infection could be advantageously prevented or treated.

The invention also includes a use of the vaccine of the invention in the preparation of a medicament for treatment of prevention of Neisserial infection. Again Neisserial infection encompasses infection by *Neisseria meningitidis* serogroups A, B, C, Y, W-135 and/or *Neisseria gonorrhoeae.*

Another aspect of the invention is a genetically engineered Neisserial strain from which an outer membrane vesicle of the inventions (having at least two proteins of the invention recombinantly upregulated, as described above) may be derived. Such Neisserial strains may be *Neisseria meningitidis* or *Neisseria gonorrhoeae .*

The strain may also have been engineered (as described above) to downregulate the expression of other Neisserial proteins including the expression of one, two, three, four, five, six, seven or eight of LgtB, LgtE, SiaD, OpC, OpA, PorA, FrpB, msbB and HtrB. Preferred combinations for downregulation include down regulation (preferably deletion) of at least LgtB and SiaD, downregulation of at least PorA and OpC, downregulation of at least PorA and OpA and downregulation of at least PorA, OpA and OpC.

Further aspects of the invention are methods of making the immunogenic composition or vaccine of the invention. These include a method comprising a step of mixing together at least two isolated antigens or proteins from Neisseria, which may be present in the form of blebs derived from the Neisserial strains of the invention, to make an immunogenic composition of the invention, and a method of making the vaccine of the invention comprising a step of combining the immunogenic composition of the invention with a pharmaceutically acceptable carrier.

Also included in the invention are methods of making the immunogenic composition of the invention comprising a step of isolating outer membrane vesicles of the invention from a Neisserial culture. Such a method may involve a further step of combining at least two outer membrane vesicle preparations, preferably wherein at least one outer membrane vesicle preparation contains LPS of immunotype L2 and at least one outer membrane vesicle preparation contains LPS of immunotype L3. The invention also includes such methods wherein the outer membrane vesicles are isolated by extracting with a concentration of DOC of 0 - 0.5%. DOC concentrations of 0.3%-0.5% are used to minimise LPS content. In OMV preparations where LPS is to be conserved as an antigen, DOC concentrations of 0-0.3%, preferably 0.05%-0.2%, most preferably of about 0.1 % are used for extraction.

### Ghost or Killed Whole cell vaccines

The inventors envisage that the above improvements to bleb preparations and vaccines can be easily extended to ghost or killed whole cell preparations and vaccines (with identical advantages). The modified Gram-negative strains of the invention from which the bleb preparations are made can also be used to made ghost and killed whole cell preparations. Methods of making ghost preparations (empty cells with intact envelopes) from Gram-negative strains are well known in the art (see for example WO 92/01791). Methods of killing whole cells to make inactivated cell preparations for use in vaccines are also well known. The terms 'bleb [or OMV] preparations' and 'bleb [or OMV] vaccines' as well as the processes described throughout this document are therefore applicable to the terms 'ghost preparation' and 'ghost vaccine', and 'killed whole cell preparation' and 'killed whole cell vaccine', respectively, for the purposes of this invention.

### Antibodies and passive immunisation

Another aspect of the invention is a method of preparing an immune globulin for use in prevention or treatment of Neisserial infection comprising the steps of immunising a recipient with the vaccine of the invention and isolating immune globulin from the recipient. An immune globulin prepared by this method is a further aspect of the invention. A pharmaceutical composition comprising the immune globulin of the invention and a pharmaceutically acceptable carrier is a further aspect of the invention which could be used in the manufacture of a medicament for the treatment or prevention of Neisserial disease. A method for treatment or prevention of Neisserial infection comprising a step of administering to a patient an effective amount of the pharmaceutical preparation of the invention is a further aspect of the invention.

Inocula for polyclonal antibody production are typically prepared by dispersing the antigenic composition in a physiologically tolerable diluent such as saline or other adjuvants suitable for human use to form an aqueous composition. An immunostimulatory amount of inoculum is administered to a mammal and the inoculated mammal is then maintained for a time sufficient for the antigenic composition to induce protective antibodies.

The antibodies can be isolated to the extent desired by well known techniques such as affinity chromatography (Harlow and Lane Antibodies; a laboratory manual 1988).

Antibodies can include antiserum preparations from a variety of commonly used animals e.g. goats, primates, donkeys, swine, horses, guinea pigs, rats or man. The animals are bled and serum recovered.

An immune globulin produced in accordance with the present invention can include whole antibodies, antibody fragments or subfragments. Antibodies can be whole immunoglobulins of any class e.g. IgG, IgM, IgA, IgD or IgE, chimeric antibodies or hybrid antibodies with dual specificity to two or more antigens of the invention. They may also be fragments e.g. F(ab')2, Fab', Fab, Fv and the like including hybrid fragments. An immune globulin also includes natural, synthetic or genetically engineered proteins that act like an antibody by binding to specific antigens to form a complex.

A vaccine of the present invention can be administered to a recipient who then acts as a source of immune globulin, produced in response to challenge from the specific vaccine. A subj ect thus treated would donate plasma from which hyperimmune globulin would be obtained via conventional plasma fractionation methodology. The hyperimmune globulin would be administered to another subject in order to impart resistance against or treat Neisserial infection. Hyperimmune globulins of the invention are particularly useful for treatment or prevention of Neisserial disease in infants, immune compromised individuals or where treatment is required and there is no time for the individual to produce antibodies in response to vaccination.

An additional aspect of the invention is a pharmaceutical composition comprising two of more monoclonal antibodies (or fragments thereof; preferably human or humanised) reactive against at least two constituents of the immunogenic composition of the invention, which could be used to treat or prevent infection by Gram negative bacteria, preferably Neisseria, more preferably *Neisseria meningitidis* or *Neisseria gonorrhoeae* and most preferably *Neisseria meningitidis* serogroup B.

Such pharmaceutical compositions comprise monoclonal antibodies that can be whole immunoglobulins of any class e.g. IgG, IgM, IgA, IgD or IgE, chimeric antibodies or hybrid antibodies with specificity to two or more antigens of the invention. They may also be fragments e.g. F(ab')2, Fab', Fab, Fv and the like including hybrid fragments.

Methods of making monoclonal antibodies are well known in the art and can include the fusion of splenocytes with myeloma cells (Kohler and Milstein 1975 Nature 256; 495; Antibodies - a laboratory manual Harlow and Lane 1988). Alternatively, monoclonal Fv fragments can be obtained by screening a suitable phage display library (Vaughan TJ et al 1998 Nature Biotechnology 16; 535). Monoclonal antibodies may be humanised or part humanised by known methods.

All references or patent applications cited within this patent specification are incorporated by reference herein.

The terms "comprising", "comprise" and "comprises" herein is intended by the inventors to be optionally substitutable with the terms "consisting of", "consist of", and "consists of", respectively, in every instance.

### Method of Industrial Application of the Invention

The examples below are carried our using standard techniques, which are well known and routine to those of skill in the art, except where otherwise described in detail. The examples are illustrative, but do not limit the invention.

### Example 1: Methods for constructing strains of Neisseria meningitidis serogroup B used in outer membrane vesicle preparations

WO01/09350 provides detailed methods for preparing outer membrane vesicles and manipulating the bacterial strains from which the outer membrane vesicles are derived. Where the outer membrane vesicles are to retain lipoproteins such as TbpB and or lipopolysaccharides, methods of isolation with low levels or no deoxycholate are preferred.

### Example 2: Up-regulation of the Hsf protein antigen in a recombinant Neisseria meningitidis serogroup B strain lacking functional cps genes but expressing PorA.

As described in WO01/09350 examples, in certain countries, the presence of PorA in outer membrane vesicles may be advantageous, and can strengthen the vaccine efficacy of recombinant improved blebs. In the following example, we have used a modified pCMK(+) vector to up-regulate the expression of the Hsf protein antigen in a strain lacking functional *cps* genes but expressing PorA. The original pCMK(+) vector contains a chimeric *porA*/*lacO* promoter repressed in *E. coli* host expressing *lacI^{q}* but transcriptionally active in *Neisseria meningitidis.* In the modified pCMK(+), the native *porA* promoter was used to drive the transcription of the *hsf* gene. The gene coding for Hsf was PCR amplified using the HSF 01*-Nde*I and HSF 02-*Nhe*I oligonucleotide primers, presented in the table below. Because of the sequence of the HSF 01*-Nde*I primer the Hsf protein expressed will contain two methionine residues at the 5' end. The conditions used for PCR amplification were those described by the supplier (HiFi DNA polymerase, Boehringer Mannheim, GmbH). Thermal cycling was the following: 25 times (94°C 1min., 48°C 1min., 72°C 3min.) and 1 time (72°C 10min., 4°C up to recovery). The corresponding amplicon was subsequently cloned in the corresponding restriction sites of pCMK(+) delivery vector. In this recombinant plasmid, designed pCMK(+)-Hsf, we deleted the *lacO* present in the chimeric *porA*/*lacO* promoter by a recombinant PCR strategy. The pCMK(+)-Hsf plasmid was used as a template to PCR amplify 2 separate DNA fragments:
- fragment 1 contains the *porA 5'* recombinogenic region, the Kanamycin resistance gene and the *porA* promoter. Oligonucleotide primers used, RP1(*Sac*II) and RP2, are presented in the table below. RP1 primer is homologous to the sequence just upstream of the *lac* operator.
- fragment 2 contains the Shine-Dalgarno sequence from the *porA* gene, the *hsf* gene and the *porA 3'* recombinogenic region. Oligonucleotide primers used, RP3 and RP4(*Apa*I), are presented in the table below. RP3 primer is homologous to the sequence just downstream of the *lac* operator. The 3' end of fragment 1 and the 5'end of fragment 2 have 48 bases overlapping. 500ng of each PCR (1 and 2) were used for a final PCR reaction using primers RP1 and RP4. The final amplicon obtained was subcloned in pSL1180 vector restricted with *Sac*II and *Apa*I*.* The modified plasmid pCMK(+)-Hsf was purified at a large scale using the QIAGEN maxiprep kit and 2 µg of this material was used to transform a *Neisseiria meningitidis* serogroup B strain lacking functional *cps* genes. In order to preserve the expression of *porA,* integration resulting from a single crossing-over was selected by a combination of PCR and Western blot screening procedures. Kanamycin resistant clones testing positive by *porA*-specific PCR and western blot were stored at -70°C as glycerol stocks and used for further studies. Bacteria (corresponding to about 5.10⁸ bacteria) were re-suspended in 50 µl of PAGE-SDS buffer, frozen (-20°C) / boiled (100°C) three times and then were separated by PAGE-SDS electrophoresis on a 12.5 % gel. The expression of Hsf was examined in Whole-cell bacterial lysates (WCBL) derived from *NmB* [Cps-, PorA+] or *NmB* [Cps-, PorA+, Hsf+]. Coomassie staining detected a significant increase in the expression of Hsf (with respect to the endogenous Hsf level). This result confirms that the modified pCMK(+)-Hsf vector is functional and can be used successfully to up-regulate the expression of outer membrane proteins, without abolishing the production of the major PorA outer membrane protein antigen.

Oligonucleotides used in this work

| **Oligonucleotides** | Sequence | **Remark(s)** |
|---|---|---|
| Hsf 01-Nde | 5'- GGA ATT CCA TAT GAT GAA CAA AAT ATA CCG C-3' | *Nde*I **cloning site** |
| Hsf02-Nhe | 5'-GTA GCT AGC TAG CTT ACC ACT GAT AAC CGA C -3' | *Nhe* I **cloning site** |
| GFP-mut-Asn | 5'-AAC TGC AGA ATT AAT ATG AAA GGA GAA GAA CTT TTC-3' | *Asn*I **cloning site** Compatible with *Nde*I |
| GFP-Spe | 5'-GAC ATA CTA GTT TAT TTG TAG AGC TCA TCC ATG -3' | *Spe*I **cloning site** Compatible with *Nhe*I |
| RP1 (SacII) | 5'- TCC CCG CGG GCC GTC TGA ATA CAT CCC GTC-3' | *Sac*II **cloning site** |
| RP2 | 5'-CAT ATG GGC TTC CTT TTG TAA ATT TGA GGG CAA ACA CCC GAT ACG TCT TCA-3' | |
| RP3 | 5'-AGA CGT ATC GGG TGT TTG CCC TCA AAT TTA CAA AAG GAA GCC CAT ATG -3' | |
| RP4(ApaI) | 5'-GGG TAT TCC GGG CCC TTC AGA CGG CGC AGC AGG -3' | *Apa*I **cloning site** |

### Example 3: Up-regulation of the N. meningitidis serogroup B tbpA gene by promoter replacement.

The aim of the experiment was to replace the endogenous promoter region of the *tbpA* gene by the strong *porA* promoter, in order to up-regulate the production of the TbpA antigen. For that purpose, a promoter replacement plasmid was constructed using *E. coli* cloning methodologies. A DNA region (731bp) located upstream from the *tbpA* coding sequence was discovered in the private Incyte PathoSeq data base of the *Neisseria meningitidis* strain ATCC 13090. This DNA contains the sequence coding for TbpB antigen. The genes are organized in an operon. The *tbpB* gene will be deleted and replaced by the *CmR*/porA promoter cassette. For that purpose, a DNA fragment of 3218bp corresponding to the 509bp 5' flanking region of *tbpB* gene, the 2139bp *tbpB* coding sequence, the 87bp intergenic sequence and the 483 first nucleotides of *tbpA* coding sequence was PCR amplified from *Neisseria meningitidis* serogroup B genomic DNA using oligonucleotides **BAD16** (5'- GGC CTA GCT AGC CGT CTG AAG CGA TTA GAG TTT CAA AAT TTA TTC-3') and **BAD17** (5'-GGC CAA GCT TCA GAC GGC GTT CGA CCG AGT TTG AGC CTT TGC-3') containing uptake sequences and *Nhe*I and *Hind*III restriction sites (underlined). This PCR fragment was cleaned with a High Pure Kit ( Boerhinger Mannheim, Germany) and directly cloned in a pGemT vector (Promega, USA). This plasmid was submitted to circle PCR mutagenesis (Jones & Winistofer (1992)) in order to (i) insert suitable restriction sites allowing cloning of a *CmR*/PorA promoter cassette and (ii) to delete 209bp of the 5' flanking sequence of *tbpB* and the *tbpB* coding sequence. The circle PCR was performed using the **BAD 18** (5'-TCC CCC GGG AAG ATC TGG ACG AAA AAT CTC AAG AAA CCG-3') & the **BAD 19** (5'-GGA AGA TCT CCG CTC GAG CAA ATT TAC AAA AGG AAG CCG ATA TGC AAC AGC AAC ATT TGT TCC G -3') oligonucleotides containing suitable restriction sites *Xma*I, *Bgl*II and *Xho*I (underlined). The *CmR*/PorA promoter cassette was amplified from the pUC D15/Omp85 plasmid previously described, using primers **BAD21** (5'- GGA AGA TCT CCG CTC GAG ACA TCG GGC AAA CAC CCG-3') & **BAD20** (5'- TCC CCC GGG AGA TCT CAC TAG TAT TAC CCT GTT ATC CC-3') containing suitable restriction sites *Xma*I, *Spe*I, *Bgl*II and *Xho*I (underlined). This PCR fragment was cloned in the circle PCR plasmid. This plasmid will be used to transform *Neisseria meningitidis* serogroup B □*cps-*□ and □*cps- porA-*□ strains. Integration by double crossing-over in the upstream region of *tbpA* will direct the insertion of the *porA* promoter directly upstream of the *tbpA* ATG.

### Example 4: Construction of a N. meningitidis serogroup B strain up-regulated for the expression of two antigens: TbpA and Hsf.

The aim of the experiment was to up-regulate the expression of TbpA and Hsf simultaneously in the same *N. meningitidis* serogroup B strain. The production of TbpA was up-regulated by replacing its endogenous promoter region by the strong *porA* promoter (promoter replacement). In this context, the *tbpB* gene, located upstream of *tbpA* is deleted, and the TbpB protein no longer present in the outer-membrane. The expression of Hsf was up-regulated by insertion (homologous recombination) of a second copy of the corresponding gene at the *porA* locus (gene delivery). Both strains have been described in a separate patent referred to as WO01/09350. The selection markers used in both strategies (Cm^{R} or Kan^{R}) allowed the combination of both integrations into the same chromosome.

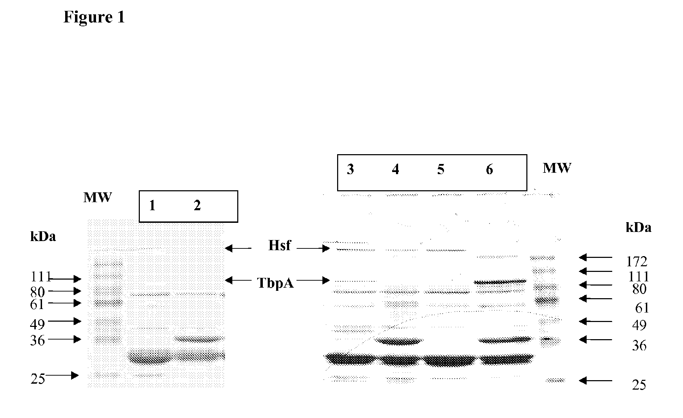

Total genomic DNA was extracted from the recombinant *Nm*.B *cps*-/TbpA+/PorA+ strain by the Qiagen Genomic tip 500-G protocol. Ten µg of DNA was restricted o/n with *Dra*III restriction enzyme and used to transform *Neisseria meningitidis* serogroup B by the classical transformation protocol. Cells used for transformation were either recombinant *Nm*B *cps*-/Hsf+/PorA+ (homologous recombination by 1 crossing over into the *porA* locus) or recombinant *NmB* cps-/Hsf+/PorA- (Allelic exchange/homologous recombination by 2 crossing over into the *porA* locus). They were plated over-night on GC agar containing 200µg/ml kanamycine, diluted to DO₆₅₀= 0.1 in GC liquid medium 10mM MgCl₂, and incubated 6 hours at 37°C under vigorous agitation with 10µg of *Dra*III restricted genomic DNA. Recombinant *Neisseria meningitidis* resulting from a double crossing over event (PCR screening) were selected on GC medium containing 200µg/ml kanamycin and 5µg/ml chloramphenicol and analyzed for TbpA and Hsf expression in OMV preparations. As represented in Figure 1, the production of both TbpA and Hsf was significantly increased in the OMV prepared from the TbpA/Hsf recombinant *NmB* strain when compared to the OMV prepared from the control *Nm*B *cps-* strains. The level of over expression of each protein in the dual recombinant is comparable with the level of expression obtained in the corresponding single recombinants. The level of over expression of TbpA and Hsf was comparable in PorA+ and PorA- strains (data not shown). All together, these data demonstrate that: (i) expression of TbpA and Hsf can be jointly and concomitantly up-regulated into *N. meningitidis* and (ii) recombinant blebs enriched for TbpA and Hsf can be obtained and used for immunization.

### Example 5: Construction of a N. meningitidis serogroup B strain up-regulated for the expression of two antigens: TbpA and NspA.

The aim of the experiment was to up-regulate the expression of TbpA and NspA simultaneously in the same *N. meningitidis* serogroup B strain. The production of TbpA was up-regulated by replacing its endogenous promoter region by the strong *porA* promoter (promoter replacement). The expression of NspA was up-regulated by insertion (homologous recombination) of a second copy of the corresponding gene at the *porA* locus (gene delivery). Both individual strains have been described in a separate patent WO01/09350. The selection markers used in both strategies (Cm^{R} or Kan^{R}) allowed the combination of both integrations into the same chromosome. Total genomic DNA was extracted from the recombinant *Nm*B *cps*-/TbpA+/PorA+ strain by the Qiagen Genomic tip 500-G protocol. Ten µg of DNA was restricted o/n with *Aat*II restriction enzyme and used to transform *Neisseria meningitidis* seregroup B by the classical transformation protocol. Cells used for transformation were recombinant *Nm*B *cps*-/NspA+/PorA-. They were plated over-night on GC agar containing 200µg/ml kanamycine, diluted to DO₆₅₀=0.1 in GC liquid medium 10mM MgCl₂, and incubated 6 hours at 37°C under vigorous agitation with 10µg of *Aat*II restricted genomic DNA. Recombinant *Neisseria meningitidis* resulting from a double crossing over event (PCR screening) were selected on GC medium containing 200µg/ml kanamycine and 5µg/ml chloramphenicol and analyzed for TbpA and NspA expression in OMV preparations. The production of both TbpA and NspA was significantly increased in the OMV prepared from the TbpA/NspA recombinant *Nm*B strain when compared to the OMV prepared from the control *Nm*B *cps-* strains. The level of over-expression of each protein in the dual recombinant is comparable with the level of expression obtained in the corresponding single recombinants. All together, these data demonstrate that: (i) expression of TbpA and NspA can be jointly and concomitantly up-regulated into *N. meningitidis* and (ii) recombinant blebs enriched for TbpA and NspA can be obtained and used for immunization.

### Example 6: Construction of a N. meningitidis serogroup B strain up-regulated for the expression of two antigens: NspA and D15/Omp85.

The aim of the experiment was to up-regulate the expression of NspA and D15/Omp85 simultaneously in the same *N. meningitidis* serogroup B strain. The production of D15/Omp85 was up-regulated by replacing its endogenous promoter region by the strong *porA* promoter (promoter replacement). The expression of NspA was up-regulated by insertion (homologous recombination) of a second copy of the corresponding gene at the *porA* locus (gene delivery). Both strains have been described in a separate patent WO01/09350. The selection markers used in both strategies (Cm^{R} or Kan^{R}) allowed the combination of both integrations into the same chromosome.

Total genomic DNA was extracted from the recombinant *Nm*B *cps-*/D15-Omp85/PorA+ strain by the Qiagen Genomic tip 500-G protocol. Ten µg of DNA was restricted o/n with *Aat*II restriction enzyme and used to transform *Neisseria, meningitidis* seregroup B by the classical transformation protocol. Cells used for transformation were recombinant *NmB cps*-/NspA+/PorA-. They were plated o/n on GC agar containing 200µg/ml kanamycine, diluted to DO₆₅₀= 0.1 in GC liquid medium 10mM MgCl₂, and incubated 6 hours at 37°C under vigorous agitation with 10µg of *Aat*II restricted genomic DNA. Recombinant *Neisseria meningitidis* resulting from a double crossing over event (PCR screening) were selected on GC medium containing 200µg/ml kanamycine and 5µg/ml chloramphenicol and analyzed for NspA and D15/Omp85 expression in OMV preparations. The production of both NspA and D15/Omp85 was significantly increased in the OMV prepared from the NspA/D15-Omp85 recombinant *NmB* strain when compared to the OMV prepared from the control *Nm*B *cps-* strains. The level of over expression of each proteins in the dual recombinant is comparable with the level of expression obtained in the corresponding single recombinants. All together, these data demonstrate that: (i) expression of NspA and Omp85 can be jointly and concomitantly up-regulated into N. meningitidis and (ii) recombinant blebs enriched for NspA and Omp85 can be obtained and used for immunization.

### Example 7: Production and purification of recombinant Hsf forms in E. coli

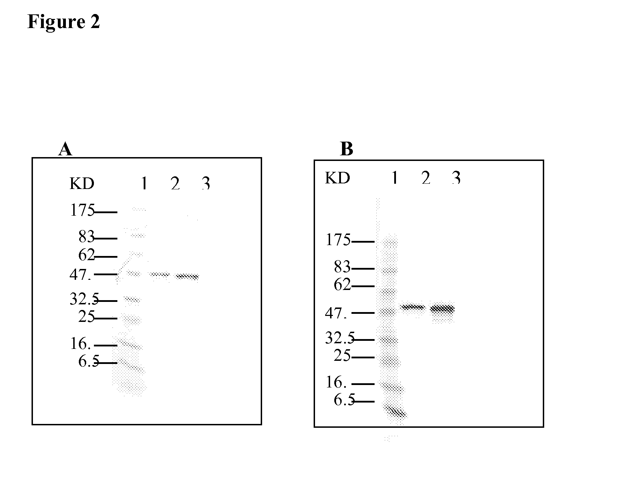

Computer analysis of the Hsf-like protein from *Neisseria meningitidis* reveals at least four structural domains. Considering the Hsf sequence from strain H44/76 as a reference, Domain 1, comprising amino-acid 1 to51, encodes a sec-dependant signal peptide characteristic of the auto-transporter family, Domain 2, comprising amino-acids 52 to 473, encode the passenger domain likely to be surface exposed and accessible to the immune system, Domain 3, comprising amino-acids 474 to 534, encodes a putative coiled-coil domain required for protein oligomerisation and a hinge (neck), Domain 4, comprising residues 535 to the C-terminus, is predicted to encode a beta-strands likely to assemble into a barrel-like structure and to be anchored into the outer-membrane (Henderson et al. (1998), Trends Microbiol. 6: 370-378; Hoiczyk et al. (2000), EMBO 22: 5989-5999). Since domains 2 and 3 are likely to be surface-exposed, are well conserved (more than 80% in all strain tested; as described in Pizza et al. (2000), Science 287: 1816-1820), they represent interesting vaccine candidates. For that purpose, domain 2 (referred to as Hsf passenger domain) and domain 2 + 3 (referred to as Hsf neck + coiled-coil domain) were expressed in and purified from *E. coli.* DNA fragments encoding amino-acids 52-473 (Hsf passenger) and 52-534 (Hsf n+cc) were PCR amplified using oligonucleotides adding terminal *Rca*I (forward primer) and *Xho*I (reverse primer) restriction sites. Purified amplicons were digested with *RcaI*/*XhoI* in the conditions recommended by the supplier, and were subsequently cloned into the *Nco*I (compatible with rcaI) / *Xho*I sites of the pET24d (Novagen Inc., Madison WI) *E. coli* expression vector. Recombinant plasmids were selected and used to prepare purified recombinant plasmids. For expression study, these vectors (pET-Hsf pas & pET-Hsf ncc) were introduced into the *Escherichia coli* strain B121DE3 (Novagen), in which, the gene for the T7 polymerase is placed under the control of the isopropyl-beta-D thiogalactoside (IPTG)-regulatable *lac* promoter. Liquid cultures (700 ml) of the Novablue (DE3) [pET-24b/BASB029] *E. coli* recombinant strain were grown at 37°C under agitation until the optical density at 600nm (OD600) reached 0.6. At that time-point, IPTG was added at a final concentration of 1mM and the culture was grown for 4 additional hours. The culture was then centrifuged at 10,000 rpm and the pellet was frozen at - 20°C for at least 10 hours. After thawing, the pellet (680 ml culture) was resuspended during 30 minutes at 22°C in 20 mM phosphate buffer pH 7.0 prior cell lysis by two passes through a Rannie disruptor. Lysed cells were pelleted 30 min at 15,000 rpm (Beckman J2-HS centrifuge, JA-20 rotor) at 4°C. The supernatant was loaded on a Q-Sepharose fast flow column (Pharmacia) equilibrated in 20 mM Tris-HCl buffer ph 8.0. After passage of the flowthrough, the column was washed with 5 column volumes of 20 mM Tris-HCl buffer pH 8.0. The recombinant protein was eluted from the column by 250 mM NaCl in 20 mM Tris-HCl buffer pH 8.0. Antigen positive fractions were pooled and dialyzed overnight against 20 mM phosphate buffer pH 7.0. 0.5M NaCl and 20 mM Imidazole were added to the dialyzed sample. Sample was then applied onto Ni-NTA Agarose column (Qiagen) equilibrated in 20 mM phosphate buffer pH 7.0 containing 500 mM NaCl and 20 mM Imidazole. After passage of the flowthrough, the column was washed with 5 column volumes of 20 mM phosphate buffer pH 7.0 containing 500 mM NaCl and 20 mM Imidazole. Contaminants were eluted by 100 mM Imidazole in 20 mM phosphate buffer pH 7.0. The recombinant protein was eluted from the column by 250 mM Imidazole in 20 mM phosphate buffer pH 7.0. Antigen positive fractions were pooled and dialyzed versus 10 mM phosphate buffer pH 6.8 containing 150 mM NaCl. As shown in figure 2, an enriched (purity estimated to more than 90 % pure in CBB stained SDS-PAGE) Hsf-like passenger protein, migrating at around 47 kDa (estimated relative molecular mass), was eluted from the column. This polypeptide was reactive against a mouse monoclonal antibody raised against the 5-histidine motif. Taken together, these data indicate that the both Hsf passenger and Hsf ncc gene can be expressed and purified under a recombinant form in *E.coli.*

### Example 8: Production and purification of recombinant Hap passenger in E. coli

Computer analysis of the Hap-like protein from *Neisseria meningitidis* reveals at least three structural domains. Considering the Hap-like sequence from strain H44/76 as a reference, Domain 1, comprising amino-acid 1 to 42, encodes a sec-dependant signal peptide characteristic of the auto-transporter family, Domain 2, comprising amino-acids 43 to 950, encode the passenger domain likely to be surface exposed and accessible to the immune system, Domain 3, comprising residues 951 to the C-terminus (1457), is predicted to encode a beta-strands likely to assemble into a barrel-like structure and to be anchored into the outer-membrane. Since domains 2 is likely to be surface-exposed, well conserved (more than 80% in all strain tested) and could be produced as subunit antigens in *E. coli,* it represents an interesting vaccine candidates. Since domains 2 and 3 are likely to be surface-exposed, are well conserved (more than 80% in all strain tested; as described in Pizza et al. (2000), Science 287: 1816-1820), they represent interesting vaccine candidates. For that purpose, domain 2 (referred to as Hap passenger domain was expressed in and purified from *E. coli.* A DNA fragment encoding amino-acids 43-950 (Hap passenger) was PCR amplified using oligonucleotides adding terminal *Nco*I (forward primer) and *Xho*I (reverse primer) restriction sites. Purified amplicons were digested with *NcoI*/*XhoI* in the conditions recommended by the supplier, and were subsequently cloned into the *Nco*I / *Xho*I sites of the pET24d (Novagen Inc., Madison WI) *E. coli* expression vector. Recombinant plasmids were selected and purified to large scale. For expression study, these vectors (pET-Hap pass) were introduced into the *Escherichia coli* strain B121DE3 (Novagen), in which, the gene for the T7 polymerase is placed under the control of the isopropyl-beta-D thiogalactoside (IPTG)-regulatable *lac* promoter.

**Cultivation of *E. coli* BL21[pET-Hap pass] in fermentor:** An aliquote fraction (100µl) from the master seed was spread on FEC013AA plates (Soja peptone A3 20g/L, yeast extract 5g/L, NaCl 5g/L, Agar 18g/L, distillated H2O up to 1L) and grown 20hours at 37°C. The bacterial lawn was harvested and resuspended in sterile water containing NaCl 0.9%. This solution was used to inoculate a 20L fermentor used in the batch mode in FEC011AC medium (Soja peptone 24g/L, Yeast extract 48g/L, MgSO4/7H2O 0.5 g/L, K2HPO4 2g/L, NaH2PO4/2H2O 0.45g/L, Glycerol (87%) 40g and distilated H20 up to 1 L). Temperature (30°C ), pH (6.8, NaOH 25% /H₃PO₄ 25%), pressure (500mbar), were maintained constant and aeration was set to 20L/min. In these conditions dissolved oxygen pressure was maitained to 20% by tuning agitation (100 to 1000rpm). Inducer (IPTG, 1mM) was added after 8 hours of growth (OD = 27.8). Samples (6L) were collected after 6 hours (OD = 49.2) and 16H30 (OD = 48.6), biomass was harvested by centrifugation and corresponding pellets stored at -20°C.

### Purification of Hap passenger:

HAP passenger was purified from a fermentor in batch mode. A purification scheme was developed (see below).

| | |
|---|---|
| | French Press |
| | (Tris 50 mM/EDTA 5 mM, pH 8.0) |
| | ↓ |
| | 30' at 10000 rpm (JA10) |
| | ↓ |
| | solubilize pellet in Pi 20 mM/urea 8M, pH7.0 |
| *1 H stirring at RT°* | |
| | ↓ |
| | 30' at 10000 rpm (JA10) |
| | ↓ |
| | **SP-Sepharose-XL** |
| | Pi 20mM/Urea 8M; elution +/-50 mM NaCl |
| | ↓ |
| | **Chelating Sepharose-FF - Cu⁺⁺** |
| | Pi 20mM/NaCl 0.5M/Urea 8M; elution +/- 100 mM imidazole |
| | ↓ |
| | **Dialysis** |
| | (PBS pH6.8/arginine 0.5M) |
| | ↓ |
| | sterile filtration |

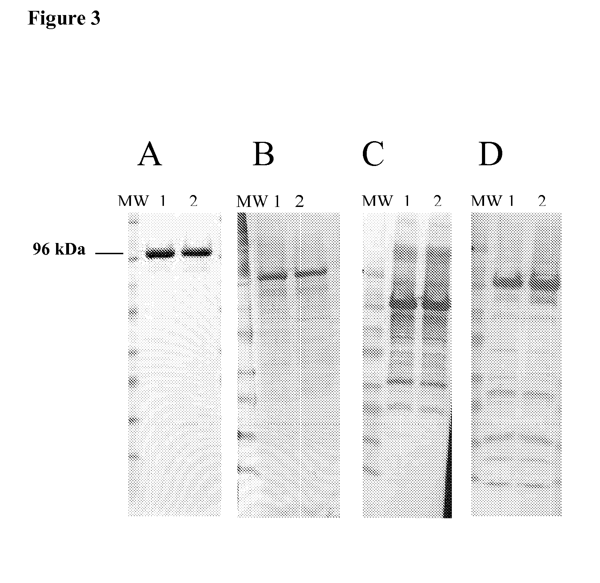

The majority of Hap passenger is recovered in the centrifugation pellet after cell breakage. Solubilization was made possible by 8M urea. Despite N-term His-tail, IMAC was not operative as 1 st step, but well after a first step on SP-XL cation-exchanger. On this SP-Sepharose-XL, the protein is eluted quantitatively in the middle of a linear NaCl (0-250 mM) gradient. IMAC was done with Cu⁺⁺-loaded Chelating Sepharose FF, as for FHAb. This time, contrary to FHAb, IMAC shows a significant purification factor. On SDS-PAGE, HAP2/3 seems pure after IMAC. The HAP2/3 peak was however very broad on 0-200 mM imidazole gradient, so we tried elution by imidazole steps (10 mM-100 mM); gradient mode seems however more efficient in terms of purity.As final step, we tried the urea-to-arginine buffer exchange by gel permeation however in this case the protein eluted on two peaks. These two peaks show a comparable profile on SDS-PAGE; so it can be hypothetized that it is due to a partial refolding of HAP passenger.We then went back to the classical dialysis as final step for buffer exchange. SDS-PAGE analysis shows good purity of the final material (see in figure 3). HAP passneger purity is further confirmed by WB anti-his. It is recognized by anti-*E*. *coli.* A Molecular weight of 96.1 KD is found.

### Example 9: Production and purification of recombinant FrpA/C forms in E. coli

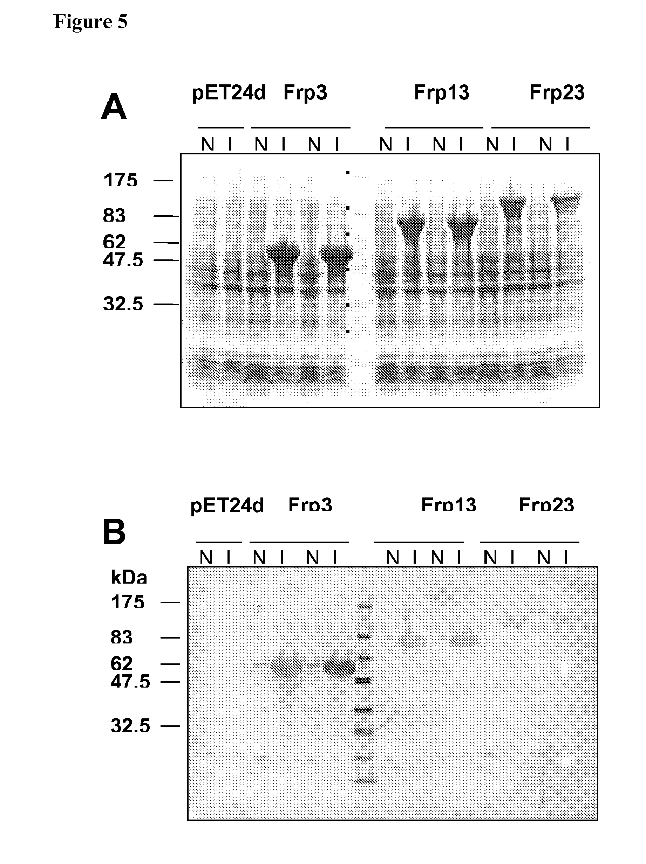

*Neisseria meningitidis* encodes two RTX proteins, referred to as FrpA & FrpC secreted upon iron limitation (Thompson et al., (1993) J. Bacteriol. 175:811-818; Thompson et al., (1993) Infect. Immun.. 61:2906-2911). The RTX (Repeat ToXin) protein family have in common a series of 9 amino acid repeat near their C-termini with the consensus: Leu Xaa Gly Gly Xaa Gly (Asn/Asp) Asp Xaa. (LXGGXGN_{/D}DX). The repeats in *E. coli* HlyA are thought to be the site of Ca2+ binding. As represented in Figure 4, meningococcal FrpA and FrpC proteins, as characterized in strain FAM20, share extensive amino-acid similarity in their central and C-terminal regions but very limited similarity (if any) at the N-terminus. Moreover, the region conserved between FrpA and FrpC exhibit some polymorphism due to repetition (13 times in FrpA and 43 times in FrpC) of a 9 amino acid motif. To evaluate the vaccine potential of FrpA & FrpC, we produced recombinantly in *E. coli* protein regions conserved between FrpA and FrpC. For that purpose, a DNA segment covering aminoacids 277 to 1007 (with regard to the *N. meningitidis* FAM20 peptide sequence) was PCR amplified from the *N. meningitidis* serogroupB H44/76 genome using forward primers FrpA-19 (5'-CTCGAGACCATGGGCAAA TATCATGTCTACGACCCCCTCGC-3') and reverse primer FrpA-18 (3'-GTG CATAGTGTCAGAGTTTTTGTCGACGTCGTAATTATAGACC-3'). Three amplicons of respectively ∼1530 bp (3 repeats), ∼2130 bp (13 repeats) and 2732 bp (23 repeats) were obtained and digested with *Nco*I and *Sal*I restriction endonucleases. These fragments were then inserted into the *Nco*I/*Xho*I (compatible with *Sal*I) sites of pET24d and recombinant plasmids (pET-Frp3, pET-Frp13 and pET-Frp23 respectively) were selected and used to transform *E. coli* BL21DE3 cells. As represented in figure 5, all three constructs produced recombinant FrpA/C conserved domains upon induction. Moreover, increasing the number of repeats increased the solubility of the recombinant protein, as determined by cell fractionation analysis (data not shown).

**Purification of FrpA/C conserved domain containing 23 repeats of the nonapeptide LXGGXGN/DDX (911 aa in total):** 3.5 liters of *E. coli* B121DE3[pET-Frp23] were cultivated and induced for 4 hours by addition of 2 mM IPTG when OD reached 0.6. Cell were harvested by centrifugation and the correspondeing pellet was pressure-disrupted, clarified by centrifugation and the corresponding supernatent loaded on a Ni2+-ion metal affinity column (Ni-NTA-agarose, Qiagen GmBh). Imidazole ()was used for elution and was finally removed by the extensive dialysis against 10 mM Na phosphate pH6.8, 150 mM NaCl .

### Example 10: Production and purification of recombinant FHA forms in E. coli Cloning of a truncated FhaB from N.meningitidis

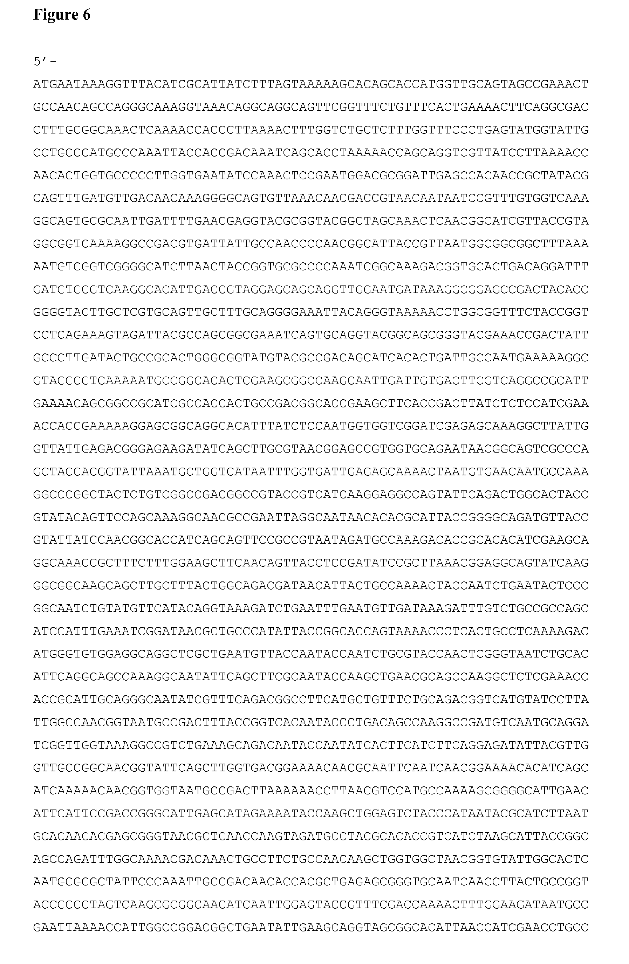

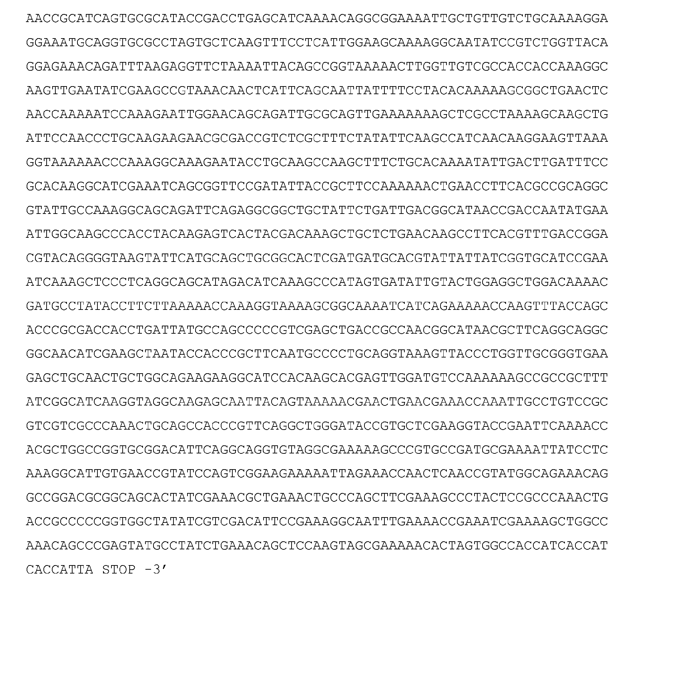

Genomic DNA was extracted from from 10¹⁰ cells of *N. meningitidis* serogroup B strain H44/76 using the QIAGEN genomic DNA extraction kit (Qiagen Gmbh). This material (1µg) was then submitted to Polymerase Chain Reaction DNA amplification using the following primers specific of the FhaB gene: JKP: 5'AAT GGA ATA CAT ATG AAT AAA GGT TTA CAT CGC ATT ATC3' and 57JKP 5'CCA ACT AGT GTT TTT CGC TAC TTG GAG CTG T3'. A DNA fragment of about 4200 bp, encoding the first 1433 N-terminal amino acids of the protein, was obtained, digested by the *Nde*I/*SpeI* restriction endonucleases and inserted into the corresponding sites of the pMG MCS (pMG derivative, Proc Natl Acad Sci U S A 1985 Jan;82(1):88-92) using standard molecular biology techniques (Molecular Cloning,a Laboratory Manual, Second Edition, Eds: Sambrook, Fritsch & Maniatis, Cold Spring Harbor press 1989). ). The DNA sequence of the cloned FhaB fragment was determined using the Big Dye Cycle Sequencing kit (Perkin-Elmer) and an ABI 373A/PRIS DNA sequencer (see fig. 1). The recombinant pMG-FhaB plasmid (1µg) was then submitted to Polymerase Chain Reaction DNA amplification using primers specific FhaB (XJKP03 5'AATGGAATA*CATATG*AATAAAGGTTTACATCGCATTATCTTTAG3' and XJKP5702 5'GGGGCCA*CTCGA*GGTTTTTCGCTACTTGGAGCTGTTTCAG ATAGG3'). A 4214 bp DNA fragment was obtained, digested by the *Nde*I/*Xho*I restriction endonucleases and inserted into the corresponding sites of the pET-24b cloning/expression vector (Novagen) using standard molecular biology techniques (Molecular Cloning,a Laboratory Manual, Second Edition, Eds: Sambrook, Fritsch & Maniatis, Cold Spring Harbor press 1989). Confirmatory sequencing of the recombinant pET-24b containing the truncated FhaB (pET24b/FhaB2/3) was performed using using the Big Dyes kit (Applied biosystems) and analysis on a ABI 373/A DNA sequencer in the conditions described by the supplier. The resulting nucleotide sequence is presentedin Figure 6.

### Expression and purification of recombinant truncated FhaB protein in Escherichia coli.

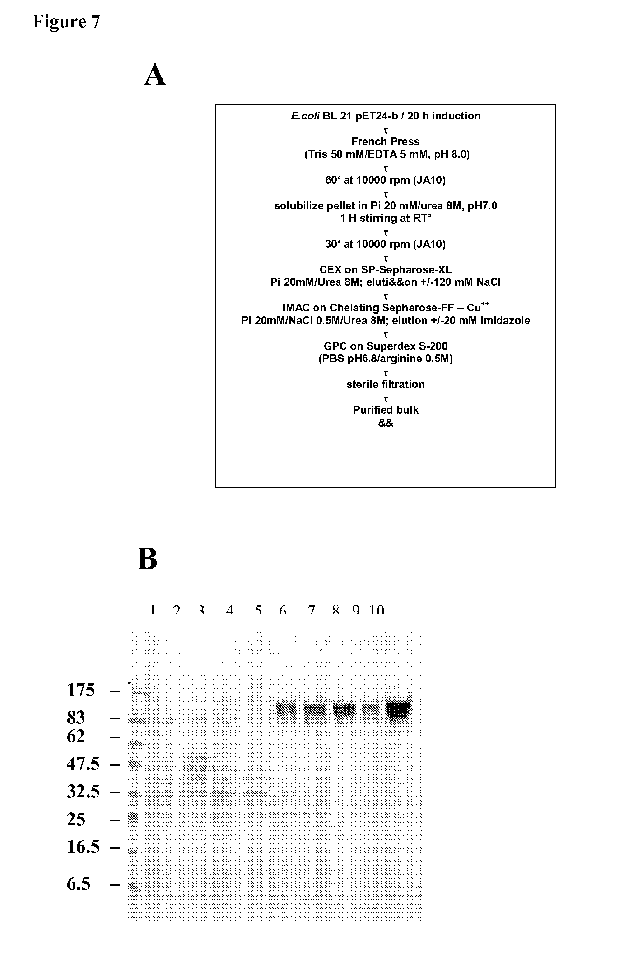

The construction of the pET24b/FhaB2/3 cloning/expression vector was described above. This vector harbours the truncated FhaB gene isolated from the strain H44/76 in fusion with a stretch of 6 Histidine residues (at the C-terminus of the recombinant product), placed under the control of the strong bacteriophage T7 gene 10 promoter. For expression study, this vector was introduced into the *Escherichia coli* strain Novablue (DE3) (Novagen), in which, the gene for the T7 polymerase is placed under the control of the isopropyl-beta-D thiogalactoside (IPTG)-regulatable *lac* promoter. Liquid cultures (100 ml) of the Novablue (DE3) [pET24b/FhaB2/3] *E. coli* recombinant strain were grown at 37°C under agitation until the optical density at 600nm (OD600) reached 0.6. At that time-point, IPTG was added at a final concentration of 1mM and the culture was grown for 4 additional hours. The culture was then centrifuged at 10,000 rpm and the pellet was frozen at -20°C for at least 10 hours. After thawing, the pellet was resuspended during 30 min at 25°C in buffer A (6M guanidine hydrochloride, 0.1M NaH2PO4, 0.01M Tris, pH 8.0), passed three-times through a needle and clarified by centrifugation (20000rpm, 15 min). The sample was then loaded at a flow-rate of 1ml/min on a Ni2+ -loaded Hitrap column (Pharmacia Biotech). After passsage of the flowthrough, the column was washed succesively with 40ml of buffer B (8M Urea, 0.1MNaH2PO4, 0.01M Tris, pH 8.0), 40ml of buffer C (8M Urea, 0.1MNaH2PO4, 0.01M Tris, pH 6.3). The recombinant protein FhaB2/3/His6 was then eluted from the column with 30ml of buffer D (8M Urea, 0.1MNaH2PO4, 0.01M Tris, pH 6.3) containing 500mM of imidazole and 3ml-size fractions were collected. As presented in figure 7, a highly enriched FhaB-2/3/His6 protein, migrating at around 154 kDa (estimated relative molecular mass), was eluted from the column. This polypeptide was reactive against a mouse monoclonal antibody raised against the 5-histidine motif. Taken together, these data indicate that the FhaB2/3 can be expressed and purified under a recombinant form in *E.coli.*

### Immunization of mice with recombinant FhaB2/3/His

Partially purified recombinant FhaB2/3/His6 protein expressed in E. coli was injected three times in Balb/C mice on days 0, 14 and 29 (10 animals/group). Animals were injected by the subcutaneous route with around 5µg of antigen in two different formulations : either adsorbed on 100µg AlPO₄ or formulated in SBAS2 emulsion (SB62 emulsion containing 5µg MPL and 5µg QS21 per dose). A negative control group consisting of mice immunized with the SBAS2 emulsion only has also been added in the experiment. Mice were bled on days 29 (15 days Post II) and 35 (6 days Post III) in order to detect specific anti-FhaB antibodies. Specific anti-FhaB antibodies were measured on pooled sera (from 10 mice/group) by ELISA on purified recombinant FhaB2/3/His.

### Example 11: Adhesion blocking activities of mouse and rabbit sera raised against

### FHA, Hap and Hsf antigens

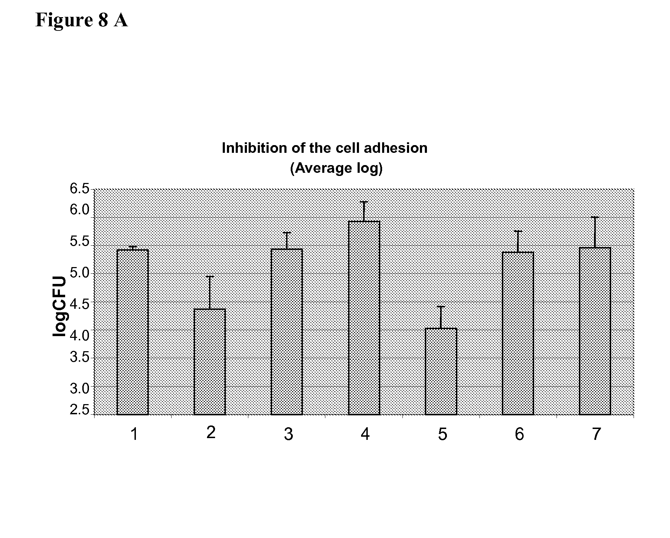

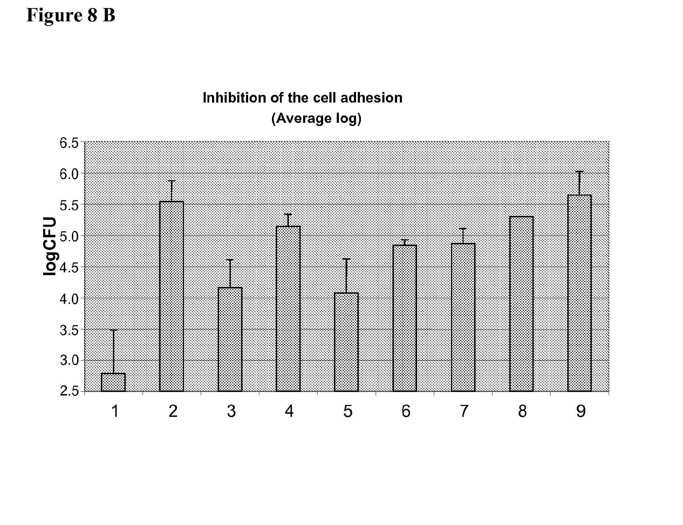

Proteins homologous to the meningococcal FHAB-like, Hsf-like and Hap-like have been described previously to be important virulence determinant and to mediate bacterial adhesion of *Bordetella pertussis* (FHA) and *Haemophilus influenzae* (Hap and Hsf). Adhesion to epithelial and endothelial cells is known to be crucial for colonization of the nasopharynges and crossing of the blood-brain barrier by the meningococcus. Thus interfering with the adhesion of *N. meningitidis* represent a valuable approach to controle meningococcal colonization and infection. Here we tested if anti-sera directed against the meningococcal FHAB 2/3^{rd}, Hap-like and Hsf-like antigens were able to interfer the adhesion of *Neisseria meningitidis* to endothelial cells. The following experimental procedure was used:
**Inhibition of adhesion to HUVEC's:** the meningococcal test strain used in this study was a non-capsulated, non-piliated, Opa- and Opc- derivative of strain NmA8013. Meningococcal cells (2.10E5 colony forming units (CFU) of the NmA8013 derivative) were incubated during 30 minutes at 37°C in a medium composed of 400µl of RPMI, 50µl of fetale bovine serum and 50 µl of the serum to be tested for adhesion blocking properties. This mixture was then placed in a well containing confluent monolayers of human umbilical vein endothelial cells (HUVEC's) whose culture medium has been previously removed. Bacteria and HUVEC's cells were incubated during 4 hours at 37°C, under 5% CO2. Cell monolayers were then washed three times with fresh RPMI serum and subsequently scrapped off the plate. CFU associated to HUVEC's cells was then determined serial dilution and plating of the cell lysate onto GC plates. Plates were incubated during 48 hours at 37°C to allow the recovery and growth of cell-associated meningococci.
**Adhesion-blocking activities of mouse and rabbit sera raised agains recombinant FHAB2/3^{rd}**, **Hap & hsf antigens:** anti-FHA 2/3, anti-Hsf full-length (described in WO99/58683) and anti-Hap full-length (WO99/55873) antibodies, as well as anti-sera directed against corresponding Hsf & Hap passenger domains, interfere with meningococcal adhesion to endothelial HUVEC's cells. Figure 8 illustrates that specific antibodies induced by FHA 2/3 formulated in AlPO₄ was able to inhibit *Neisseria meningitidis B* adhesion to the HUVEC cells compared to the adjuvant only. When compared to the SBAS2 adjuvant only (without antigen, group 4), the anti-FHA 2/3 abs (SBAS2 formulation) is still effective, but less potent than AlPO₄. The SBAS2 adjuvant only (without antigen) does not induce antibodies able to interfere with the adhesion. Compared to group 4, anti-Hap antibodies (group 1) may have a slight inhibition effect. In group 5, when a mixture of anti-FHA 2/3, anti-Hsf and anti-Hap antibodies is tested, inhibition of the adhesion is stronger than with anti-FHA 2/3 only, suggesting a synergetic effect given by anti-Hap and anti-Hsf antibodies. In a second inhibition experiment (Fig 2), a specific rabbit antiserum directed against anti OMVs over-expressing Hsf (as a candidate protein) was able to inhibit partially the fixation of *Neisseria meningitidis* B to the endothelial cells compared to the negative control (group 3 vs 4). This rabbit antiserum has been demonstrated to contain a very high specific anti-Hsf antibody titer. Antibodies against rec Hsf (Hsf passenger and Hsf full length) are also able to inhibit adhesion of bacteria on the HUVEC cells. This is true both with mice sera (groups 5 - 6) as well as with rabbit sera (in a laser extend) (groups 7 -8). In this second experiment, specific anti-rec FHA 2/3 antibodies (group 1) already tested in the first experiment confirm their very high inhibitory effect. These results indicate that these specific antigens (Hap, FHA2/3 and Hsf), isolated or in combination, are interesting vaccine antigens.

### Example 12: Protective effect of recombinant OMV's in the mouse challenge model

Several recombinant OMVs have been evaluated in Balb/C mice for their protective effect after lethal challenge. This active immunisation model involved intraperitoneal injection of meningococci from several strains (suspended in iron depleted TSB medium) into adult Balb/C or OF1 mice (6 - 8 weeks old), after a series of immunization by the subcutaneous route. The iron dextran, used as an external iron source seems to be needed to maintain bacteraemia and induce mortality in infected animal. Although this IP model in mice has been shown to be effective for assessing virulence, immune protection and the role of iron in infection, they do not incorporate the pharyngeal carriage phase, which precedes bacteraemia and meningitis in humans. This model has been used to screen our several OMV candidates over-expressing NspA, TbpA, or Hsf. In the following experiments, Balb/C (inbred) or OF 1 (outbred) mice were immunized three times on days 0, 14 and 28 by the subcutaneous route with 3 (PV00N049) to 5µg (PV00N035 and PV00N043 experiments) of rec. OMV over-expressing Hsf, NspA or TbpA formulated on Al(OH)₃ (100 µg Al(OH)₃ /animal) (PV00N035 and PV00N043) or on Al PO₄ (100 µg Al PO₄/animal Then, animals are bled on days 28 (day 14 past II) and 35 (day 7 past III) for specific Ab evaluation. On day 35, 10 mg of iron dextran are injected intraperitaneally one hour before the challenge. The challenges were done with H44/76 (B:15:P1.7,16) or CU-385 (B:4:P1.19,15) strains, with around 1.10 e7 CFU/animal (see the table of results for the exact challenge doses). The heterologous strain done with the CU-385 strain is more stringent than when using the homologous strain. Mortalities were recorded from days 1 to 5. The table 1 hereafter illustrates that when compared to OMV porA (-) and with OMV porA (+) in a lesser extend, there is already a better protection observed with OMV TbpA (+) (1/10 and 3/5 for porA(-) and 9/10 and 3/5 for porA (+)), with OMV NspA (+) ( 4/10 and 4/5) and with OMV Hsf (+) (3/10, 2/10 and 3/5). This is the global observation we can make in these three experiments. These data support that TbpA, Hsf and NspA antigens, expressed at the bleb surface, are of interest for a future *men*B vaccine.

**Table 1: Protective activity in the mouse model of recombinant outer-membrane vesicles. The table summarizes the results obtained during three experiments (PV00N35, PV00N043 & PV00N049)**

| OMVs (blebs) | | | | |
|---|---|---|---|---|
| Rec OMVs H44/76 background= porA P1.17, 16) | Survival rate (on day Active mouse protection | | | Immuno Specific Abs by Elisa Mean - PV00N049 only |
| | PV00N035 in OF1 mice | PV00N04 in Balb/C mice | PV00N049 in OF1 mice | |
| | Challenge strain (+ | | | |
| | (H44/76 | H44/76 | CU-385 | |
| | (B:15:P1.7, 1.27 | (B:15:P1.7, 1.0 | (B:4:P1.19,1 1.1 | |
| OMV porA(-) | 1/10 | 0/10 | 1/5 | / |
| OMV porA(+) | 2/10 | 9/10 | 4/5 | / |
| OMV TbpA porA(+) | NT | 9/10 | 3/5 | < |
| OMV TbpA porA (-) | NT | 1/10 | 3/5 | < |
| OMV NspA porA (-) | 1/10 | 4/10 | 4/5 | 155 - (< |
| OMV Hsf PorA(-) | 3/10 | 2/10 | 3/5 | 7802 - (5496) |
| OMV Hsf PorA(+) | NT | 9/9 | NT | / |
| No antigen | 0/10 | 0/10 | 1/5 | / |

| | | | | |
|---|---|---|---|---|
| NT : Not tested. | | | | |

### Example 13: Protective effect of recombinant subunit antigens in the mouse challenge model

Several recombinant purified proteins have been evaluated in Balb/C mice for their protective effect after lethal challenge. This active immunization model involved introperitoneal injection of meningococci from several strains (suspended in iron depleted TSB medium) into adult Balb/C or OF 1 mice (6 - 8 weeks old) after a series of immunization by the subcutaneous route.

The iron dextran, used as an external iron source seems to be needed to maintain bacteraemia and induce mortality in infected animal. Although this IP model in mice has been shown to be effective for assessing virulence, immune protection and the role of iron in infection, they do not incorporate the pharyngeal carriage phase, which precedes bacteraemia and meningitis in humans. This model has been used to screen several *men*B sub-unit vaccine candidates like recombinant FrpC, TbpA, FHA2/3 and Hap molecules.

In this experiment, OF 1 (outbred) mice were immunized three times on days 0, 14 and 28 by the subcutaneous route with 5 µg (PV00N050) of these proteins formulated on Al PO₄ (100 µg) in presence of 10 µg MPL (per animal).

Then, animals are bled on days 28 (day 14 past II) and 35 (day 7 past III) for specific Ab evaluation, while they are challenged on day 35. The day of challenge, 10 mg of iron dextran are injected intraperitaneally one hour before the challenge. The challenges were done with CU-385 strains (B:4:P1.19,15), which is heterologous in this case, indeed, the antigens sequence coming from the H44/76 (B:15:P1.7,16), except for the TbpA for which the sequence comes from the B16B6 strain (B:2a:P1.2).

The results illustrated in table 2 indicate that FrpC, TbpA, FHAB2/3^{rd}, Hap induced significant protection in this model : from 2 to 4 out of 5 mice survived after challenge, compared to only 1/5 with the adjuvant only. In all groups but one, the specific antibody titer were high (specific anti-TbpA titer was moderate). All these data support that FrpC, FrpA, FrpA/C conserved domain, TbpA, FHAB2/3^{rd}, Hap presented as sub-unit antigens, isolated or in combination, are of interest for the development of a *men*B vaccine.

**Table 2: Protective activity in the mouse model of recombinant outer-membrane vesicles. The table summarizes the results obtained during one experiment (PV00N050).**

| **Sub-unit antigens** | | |
|---|---|---|
| **Rec sub-unit** **Antigens** **(from H44/76** **Ag sequence)** | **Survival rate (on day 5) Active mouse protection model** | Immuno Specific Abs by Elisa Mean - (GMT) |
| | PV00N050 (in OF1 mice) | |
| | Challenge strain (+ dose) | |
| | CU-385 (B:4:P1.19,15) 1.4 10e7 | |
| **FrpC Ca2++** treated | **3/5** | 31477 - (27068) |
| **FHAB** 2/3 **refolded** | **2/5** | 98200 - (73220) |
| **FHAB** 2/3 non refolded | **3/5** | 55939 - (35347) |
| **Hap N-ter** | **4/5** | 9960 - (12811) |
| rec**TbpA** on **SBAS4** | **3/5** | 875-(520) |
| SBAS4 | 1/5 | / |

### Example 14: Method to show synergetic effect of vaccine antigens combinations.

Different recombinant OMVs available (OMVs porA (+) rmp-LbpB, OMVs porA (-) TbpA (+) Hsf(+), OMVs porA (-) TbpA (+), OMVs porA (-) NspA (+), OMVs porA (-) Hsf (+), OMVs porA (-) TbpA (+) NspA (+)) can be tested alone or in combination to determine statistically the best combinations, in terms of detecting a synergetic effect of such combinations of vaccine candidates. This work can also be performed with combinations of subunit antigens, as well as combination of subunit antigens + recombinant OMV's. 32 groups of 5 OF1 mice/group can be injected and tested for serum bactericidal & opsonic activity, active and passive protection in the mouse model (if needbe using suboptimal amounts of individual antigens). An indication of synergistic antigen combinations is if the level of protective conferred after combined immunization is higher that the sum of individual antigens.

### Example 15: Analysis of Hsf and TbpA content of Outer Membrane Vesicles Coommassie blue stained SDS-PAGE

15µg of protein in outer membrane vesicle preparations with up-regulation of Hsf or TbpA or both Hsf and TbpA, were diluted in a sample buffer containing β-mercaptoethanol and heated at 95°C for 10 minutes. The samples were then run on SDS-PAGE polyacrylamide gel (Novex 4-20% Tris-glycine 1.5 mm 2Dwell SDS Page), stained in Coomassie blue for one hour and destained in several washes of destain. Results are shown in Figure 9, which shows that the level of Hsf and TbpA are considerably higher in outer membrane vesicle preparations, derived from *N*. *meningitidis* where their level of expression had been enhanced.

### Example 16: Immunogenicity of OMVs with upregulation of Hsf and/or TbpA

Groups of 20 mice were immunised three times with OMV by the intra-muscular route on days 0, 21 and 28. Each innoculation was made up of 5µg (protein content) of OMVs formulated on A1PO4 with MPL. The OMVs were derived from *N. meningitidis* strain H44/76, engineered so that capsular polysaccharides and PorA were down regulated. A comparison was made of OMVs in which Hsf, TbpA, both Hsf and TbpA or neither were upregulated. On day 41, blood samples were taken for analysis by ELISA or by serum bactericidal assay.

### ELISA to detect antibodies against Hsf

96 well microplates (Nunc, Maxisorb) were coated overnight at 4°C with 100 µl of 1 µg/ml of specific antigen in PBS. After washing with NaCl 150 mM Tween 20 0.05%, plates were saturated with 100 µl of PBS-BSA 1% under shaking at room temperature for 30 minutes. Between each step (performed under shaking at room temperature during 30 min and with PBS-BSA 0.2% as diluant buffer), reagents in excess were removed by washing with NaCl-Tween 20. One hundred micro-liters of diluted serum samples were added per micro-well. Bound antibodies were recognized by a biotinylated anti-mouse Ig (Prosan) (1/2000). The antigen-antibody complex was revealed by incubation with streptavidin-biotinylated peroxidase conjugate (Amersham) (1/4000). OrthoPhenileneDiamine/H₂O₂ (4 mg/10 ml citrate buffer 0.1M pH 4.5 + 5 µl H₂O₂) is used to reveal the assay. Plates were incubated for 15 min at room temperature in the dark before stoping the reaction by addition of 50 µl of 1N HCl. The absorbance was read at 490nm.

| | Titre Mid-Point (on pooled sera) |
|---|---|
| g1, blebs TbpA-HSF, IM | 15471 |
| g2, blebs TbpA, IM | 15.41 |
| g3, blebs HSF, IM | 14508 |
| g4, blebs CPS(-)PorA(-), IM | - |
| g5, MPL/AIPO4, IM | - |

The results shown in the table above, show that high and equivalent antibody titres against Hsf were raised by immunisation with OMVs with upregulation of Hsf or both Hsf and TbpA. Virtually no antibody against Hsf could be detected in sera raised after inoculation with adjuvant alone or OMV in which neither Hsf nor TbpA had been upregulated or OMV in which only TbpA had been upregulated.

### Example 17: Serum Bactericidal Activity of antisera raised against OMVs with up-regulation of Hsf and/or TbpA

The serum bactericidal activity of antisera from the mice inoculated with OMVs with upregulation of Hsf, TbpA, both Hsf and TbpA or without upregulation were compared in assays using either the homologous strain H44/76 or the heterologous strain Cu385. The serum bactericidal assay has been shown to show good correlation with the protection and is therefore a good indication of how effective a candidate composition will be in eliciting a protective immune response.

*Neisseria meningitidis* serogroup B wild type strains (H44/76 strain =B:15 P1.7,16 L3,7,9 and CU385 strain =B: 4 P1.19,15 L3,7,9) were cultured overnight on MH + 1% Polyvitex + 1% horse serum Petri dishes at 37°C + 5% CO2. They were subcultured for 3 hours in a liquid TSB medium supplemented with 50 µM of Desferal (Iron chelator) at 37°C under shaking to reach an optical density of approximately 0.5 at 470 nm.

Pooled or individual serum were inactivated for 40 min at 56°C. Serum samples were diluted 1/100 in HBSS-BSA 0.3% and then serially diluted two fold (8 dilutions) in a volume of 50 µl in round bottom microplates.

Bacteria, at the appropriate OD, were diluted in HBSS-BSA 0.3% to yield 1.3 10e4 CFU per ml. 37.5 µl of this dilution was added to the serum dilutions and microplates were incubated for 15 minutes at 37°C under shaking. Then, 12.5 µl of rabbit complement were added to each well. After 1 hour of incubation at 37°C and under shaking, the microplates were placed on ice to stop the killing.

Using the tilt method, 20µl of each well were platted on MH + 1% Polyvitex + 1% horse serum Petri dishes and incubated overnight at 37°C +CO2. The CFU's were counted and the percent of killing calculated. The serum bactericidal titer is the last dilution yielding ≥ 50% killing.

| | **H44/76** | | **CU385** | |
|---|---|---|---|---|
| OMV | GMT | % responders | GMT | % responders |
| CPS(-) PorA (-) | 93 | 30% | 58 | 5% |
| CPS(-) PorA (-) Hsf | 158 | 40% | 108 | 20% |
| CPS(-) PorA (-) TbpA | 327 | 60% | 147 | 30% |
| CPS(-) PorA (-) Hsf - TbpA | **3355** | **100%** | **1174** | **80%** |

Similar results to those shown in the above table were obtained in two other similar experiments.

A dramatic increase in the bactericidal titres (GMT) against the homologous strain and a heterologous strain were seen after vaccination with OMV in which both Hsf and TbpA were upregulated. By comparison, bactericidal GMTs measured on mice vaccinated with Hsf or TbpA upregulated OMVs were similar to those obtained with mice vaccinated with control OMVs.

The benefit of double up-regulation was also clearly observed in the percentage of mice producing a significant level of bactericidal antibodies (titres greater than 1/100), particularly in experiments using the heterologous strain.

### Example 18: Effect of mixing anti-Hsf and anti-TbpA sera on bactericidal activity

Groups of 20 mice were immunised three times with OMV by the intra-muscular route on days 0, 21 and 28. Each inoculation was made up of 5µg (protein content) of OMVs formulated on AlPO4 with MPL. The OMVs were derived *from N. meningitidis* strain H44/76, engineered so that capsular polysaccharides and PorA were down regulated. One group of mice was immunised with control OMVs in which there was no up-regulation of proteins. In a second group, Hsf expression was up-regulated, in a third group TbpA expression was up-regulated and in a fourth group, the expression of both Hsf and TbpA was up-regulated.

The sera were pooled, either using sera from mice in the same group or by mixing sera isolated from the group in with Hsf alone or TbpA alone had been up-regulated. Serum bactericidal activity was measured for each of the pooled sera and the results are shown in the table below.

| SBA done on pooled sera from mice immunized with | SBA titer |
|---|---|
| TbpA-Hsf blebs | 774 |
| TbpA blebs | 200 |
| Hsf blebs | 50 |
| CPS(-) PorA(-) blebs | 50 |
| Mix anti-TbpA + anti-Hsf sera | 1162 |

The results in the above table show that mixing of anti-Hsf and anti-TbpA antisera resulted in a much higher serum bactericidal activity than was achieved by either antisera individually. The synergistic effect seems to be achieved by the presence of antibodies against both Hsf and TbpA.

### Example 19: Truncated Hsf proteins may combine synergistically with TbpA

A series of truncated Hsf constructs were made using standard molecular biology procedures. These include a construct that encodes amino acids 1 to 54 which contains the signal sequence of Hsf and amino acids 134 to 592 of Hsf (Tr1Hsf). A second truncated Hsf contained amino acids 1-53 of the signal sequence of Hsf followed by amino acids 238-592 of Hsf (Tr2Hsf). These two truncated Hsf constructs and full length Hsf were introduced into *N. Meningitidis* B strain MC58 siaD-, Opc-, PorA- so that their expression would be up-regulated and outer membrane vesicles were produced using the methods described above.

The outer membrane vesicle preparations were adsorbed onto Al(OH)3 and injected into mice on days 0, 21 and 28. On day 42, the mice were bled and sera prepared. The sera were mixed with sera from mice vaccinated with up-regulated TbpA OMVs and serum bactericidal assays were performed as described above.

### Results

| | Serum Bactericidal titres | |
|---|---|---|
| Group | H44/76 | CU385 |
| MC58 PorA+ siaD+ | 25600 | 25600 |
| MC58 PorA- siaD- Hsf | 1530 | 800 |
| MC58 PorA- siaD- Tr1Hsf | 1015 | 1360 |
| MC58 PorA- siaD- Tr2Hsf | 50 | 50 |
| Negative control | 50 | 50 |
| | | |
| TbpA + MC58 PorA+ siaD+ | 25600 | 24182 |
| TbpA + MC58 PorA- siaD- Hsf | 2595 | 1438 |
| TbpA + MC58 PorA- siaD- Tr1Hsf | **4383** | **2891** |
| TbpA + MC58 PorA- siaD- Tr2Hsf | 1568 | 742 |
| TbpA + Negative control | 778 | 532 |

The results shown in the above table reveal that the first truncation (Tr1Hsf) elicits an immune response which is capable of combining with antisera against TbpA to produce a larger serum bactericidal activity than when full length Hsf is used. However, the extent of the truncation is important and the truncation produced in Tr2 has a deleterious effect compared to the full length Hsf. The enhanced bactericidal activity of Tr1Hsf was seen against both the strains used.

### Example 20: Serum bactericidal activity of antibodies against TbpA, Hsf and a third meningococcal protein

*N. meningitidis* strain H66/76 in which PorA and capsular polysaccharides were down regulated as described above, was used as the background strain for upregulating TbpA and Hsf, LbpB, D15, PilQ or NspA using the procedure described above. Outer membrane vesicles were prepared from each strain as described above. Recombinant FHAb, FrpC, FrpA/C and Hap were made using techniques hereinbefore described and known in the art (as described in PCT/EP99/02766, WO92/01460 and WO98/02547).

The outer membrane vesicle preparations and recombinant proteins were adsorbed onto Al(OH)3 and injected into mice on days 0, 21 and 28. On day 42, the mice were bled and sera prepared. The sera against TbpA and Hsf up-regulated OMVs were mixed with sera from mice vaccinated with OMVs containing up-regulated LbpB, D15, PilQ or NspA OMVs or recombinant FHAb, FrpC, FrpA/C or Hap and serum bactericidal assays were performed as described above.

### Results

Results are shown in the table below. In assays using the homologous H44/76 stain, the addition of antibodies against a third meningococcal antigen, with the exception of FrpC, did not produce a serum bactericidal titre higher than that produced using antibodies against TbpA and Hsf alone.

However, the addition of antibodies against a third antigen was advantageous in serum bactericidal assays using a heterologous strain. Antibodies against D15 (OMP85), Hap, FrpA/C and LbpB were particularly effective at increasing the serum bactericidal titre against the CU385 strain.

| | Serum Bactericidal Titre | |
|---|---|---|
| Antisera Mix | H44/76 | CU385 |
| anti-TbpA-Hsf and nonimmune sera | 5378 | 2141 |
| anti-TbpA-Hsf and anti-FHA | 5260 | 2563 |
| anti-TbpA-Hsf and anti-Hap | 4577 | 5150 |
| anti-TbpA-Hsf and anti-FrpA/C | 5034 | 4358 |
| anti-TbpA-Hsf and anti-LbpB | 5400 | 4834 |
| anti-TbpA-Hsf and anti-D15 | 4823 | 4657 |
| anti-TbpA-Hsf and anti-PilQ | 4708 | 2242 |
| anti-TbpA-Hsf and anti-NspA | 4738 | 2518 |
| anti-TbpA-Hsf and anti-FrpC | 6082 | 2300 |

### Example 21: Effect of FrpB KO in outer membrane vesicles on their ability to elicit a bactericidal immune response in homologous and heterologous strains

Two strains of H44/76 N. meningitidis were used to prepare outer membrane vesicle preparations as described in WO01/09350, using a 0.1% DOC extraction so that the LOS content was around 20%. Strain B1733 is siaD(-), PorA(-), has upregulation of Tr1 Hsf (example 19) and IgtB is knocked out. Strain B1820 B1733 is siaD(-), PorA(-), has upregulation of Trl Hsf , lgtB is knocked out and FrpB is also knocked out. Both strains were cultured in media supplemented with 60µM Desferal so that iron regulated proteins such as LbpA/B and TbpA/B are upregulated.

The bleb preparations were adsorbed onto Al(OH)3 and 5µg were injected intramuscularly into groups of 30 mice on day 0 and day 21. Blood samples were taken on day 28.

Serum bactericidal assays were carried out on three L3 strains (the homologous wild type strain H44/76 and two heterologous L3 strains; NZ124 and M97250687), as described in example 17.

### Results

| Blebs used for inoculation | **H44/76** | | **M97250687** | | **NZ124** | |
|---|---|---|---|---|---|---|
| | GMT | SC | **GMT** | **SC** | **GMT** | **SC** |
| B1733 | 1518 | 30/30 | 151 | 11/30 | 70 | 4/29 |
| B1820 | 781 | 19/30 | 1316 | 24/30 | 276 | 19/30 |

| | | | | | | |
|---|---|---|---|---|---|---|
| GMT indicates the geometric mean titre of the sera in the SBA. SC indicates the number of mice seroconverting (SBA titre >1/100). | | | | | | |

The results clearly show that FrpB KO (B1820) blebs induce a better heterologous cross-bactericidal response than FrpB(+) blebs (B1733). The SBA titres were higher and a higher proportion of mice seroconverted in strains M97250687 and NZ124. The results in the homologous strain was not quite as good when FrpB was deleted. These data suggest that FrpB drives the immune response, but since this outer membrane protein is highly variable, antibodies against this protein are only able to induce killing of the homologous strain.

### Preferred Embodiments

The invention also relates to the following preferred embodiments:
1. An immunogenic composition comprising two or more different antigens,
   wherein the antigens are selected from at least two of the following categories:
   a) at least one Neisserial adhesin;
   b) at least one Neisserial autotransporter;
   c) at least one Neisserial toxin;
   d) at least one Neisserial Fe acquisition protein; or
   e) at least one Neisserial membrane associated protein, preferably integral outer membrane protein.
2. The immunogenic composition of embodiment 1, wherein the antigens are selected from at least two of the following categories:
   a) at least one Neisserial adhesin selected from the group consisting of FhaB, NspA, PilC, Hsf, Hap, MafA, MafB, Omp26, NMB0315, NMB0995, NMB1119 and NadA;
   b) at least one Neisserial autotransporter selected from the group consisting of Hsf, Hap, IgA protease, AspA and NadA;
   c) at least one Neisserial toxin selected from the group consisting of FrpA, FrpC, FrpA/C, VapD, NM-ADPRT, and either or both of LPS immunotype L2 and LPS immunotype L3;
   d) at least one Neisserial Fe acquisition protein selected from the group consisting of TbpA high, TbpA low, TbpB high, TbpB low, LbpA, LbpB, P2086, HpuA, HpuB, Lipo28, Sibp, FbpA, BfrA, BfrB, Bcp, NMB0964 and NMB0293; or
   e) at least one Neisserial membrane associated protein, preferably integral outer membrane protein selected from the group consisting of PilQ, OMP85, FhaC, NspA, TbpA(high), TbpA(low), LbpA, HpuB, TspA, TspB, TdfH, PorB, HimD, HisD, GNA1870, OstA, HlpA, MltA, NMB 1124, NMB 1162, NMB 1220, NMB 1313, NMB 1953, HtrA and PldA.
3. The immunogenic composition of embodiment 1 or 2 which is a subunit composition
4. The immunogenic composition of embodiment 3 comprising at least 2 antigens selected from the following list: FhaB, NspA, passenger domain of Hsf, passenger domain of Hap, surface exposed domain of OMP85, FrpA, FrpC, TbpB, LbpB, PldA, PilC, Lipo28 and either or both of LPS immunotype L2 and LPS immunotype L3.
5. The immunogenic composition of embodiment 1 or 2 comprising an outer membrane vesicle preparation, wherein the antigens have been upregulated (preferably recombinantly) in the outer membrane vesicle.
6. The immunogenic composition of embodiment 5 comprising at least two antigens selected from the following list which have been upregulated in the outer membrane vesicle: NspA, Hsf, Hap, OMP85, AspA, HpuA, HpuB, TspA, TspB, FhaC, TbpA (high), TbpA (low), LbpA, TbpB, LbpB, PilQ, NM-ADPRT, P2086, TdfH, PorB, MafA, MafB, HimD, HisD, GNA1870, OstA, HlpA, MltA and PldA; and optionally comprising either or both of LPS immunotype L2 and LPS immunotype L3.
7. The immunogenic composition of embodiment 1 or 2 comprising a subunit composition having one or more of the antigens, and an outer membrane vesicle preparation having at least one antigen which has been upregulated in the outer membrane vesicle.
8. The immunogenic composition of embodiment 7 comprising a subunit composition and an outer membrane vesicle preparation wherein the subunit composition comprises at least one antigen selected from the following list:
   FhaB, NspA, passenger domain of Hsf, passenger domain of Hap, surface exposed domain of OMP85, FrpA, FrpC, TbpB, LbpB, PilC, Lipo28 and the outer membrane vesicle preparation having at least one different antigen selected from the following list, which has been recombinantly upregulated in the outer membrane vesicle: NspA, Hsf, Hap, OMP85, AspA, HpuA, HpuB, TspA, TspB, FhaC, TbpA (high), TbpA (low), LbpA, TbpB, LbpB, PilQ, NM-ADPRT, P2086, TdfH, PorB, MafA, MafB, HimD, HisD, GNA1870, OstA, HlpA, MltA and PldA; and optionally comprising either or both of LPS immunotype L2 and LPS immunotype L3, preferably within the outer membrane vesicle preparation.
9. The immunogenic composition of embodiments 5-8 comprising at least two different outer membrane vesicle preparations of embodiment 5 or 6.
10. The immunogenic composition of embodiment 9 wherein one outer membrane vesicle preparation is immunotype L2 and one outer membrane vesicle preparation is immunotype L3.
11. The immunogenic composition of embodiments 1, 2, 5, 6 , 7, 8, 9 or 10 wherein Hsf and TbpA (high) are selected.
12. The immunogenic composition of embodiments 1-2 or 5-11, wherein Hsf and TbpA (low) are selected.
13. The immunogenic composition of embodiments 11 or 12 wherein one or more additional antigens from a list consisting of Hap, LbpB, OMP 85 and FrpA are further selected.
14. The immunogenic composition of embodiments 11-13 wherein LPS immunotype L2 is further selected.
15. The immunogenic composition of embodiments 11-14 wherein LPS immunotype L3 is further selected.
16. The immunogenic composition of embodiments 1-15 wherein FhaB is selected together with at least one further antigen selected from the group consisting of :
   PilC, MafA, MafB, Omp26, NMB0995, FhaC, FbpA, Bcp, NMB 1124, NMB 1162, NMB 1220, NMB 1313, NMB 1953, HtrA, Hsf, LbpB, FrpA, FrpC, FrpA/C, NadA, OMP85, PldA, LbpA, TbpA (low), TbpA(high), TbpB(low), TbpB(high), HpuA, HpuB, Hap, IgA protease, AspA, PilQ, MltA, HimD, HisD, GNA1870, OstA, HlpA, NspA, TspA, TspB, P2086, Lipo28, Sibp, NMB0964, NMB0293, NMB0315, NMB1119, TdfH, PorB, NM-ADPRT, VapD and either or both of LPS immunotype L2 and LPS immunotype L3.
17. The immunogenic composition of embodiments 1-16 wherein NspA is selected together with at least one further antigen selected from the group consisting of :
   PilC, MafA, MafB, Omp26, NMB0995, FhaC, FbpA, Bcp, NMB 1124, NMB 1162, NMB 1220, NMB 1313, NMB 1953, HtrA, Hsf, LbpB, FrpA, FrpC, FrpA/C, NadA, OMP85, PldA, LbpA, TbpA (low), TbpA(high), TbpB(low), TbpB(high), HpuA, HpuB, Hap, IgA protease, AspA, PilQ, MltA, HimD, HisD, GNA1870, OstA, HlpA, TspA, TspB, P2086, Lipo28, Sibp, NMB0964, NMB0293, NMB0315, NMB1119, TdfH, PorB, NM-ADPRT, VapD and either or both of LPS immunotype L2 and LPS immunotype L3.
18. The immunogenic composition of embodiments 1-17 wherein NadA is selected together with at least one further antigen selected from the group consisting of :
   PilC, MafA, MafB, Omp26, NMB0995, FhaC, FbpA, Bcp, NMB 1124, NMB 1162, NMB 1220, NMB 1313, NMB 1953, HtrA, Hsf, LbpB, FrpA, FrpC, FrpA/C, OMP85, PldA, LbpA, TbpA (low), TbpA(high), TbpB(low), TbpB(high), HpuA, HpuB, Hap, IgA protease, AspA, PilQ, MltA, HimD, HisD, GNA1870, OstA, HlpA, TspA, TspB, P2086, Lipo28, Sibp, NMB0964, NMB0293, NMB0315, NMB1119, TdfH, PorB, NM-ADPRT, VapD and either or both of LPS immunotype L2 and LPS immunotype L3.
19. The immunogenic composition of embodiments 1-18 wherein TbpA (low) is selected together with at least one further antigen selected from the group consisting of: PilC, MafA, MafB, Omp26, NMB0995, FhaC, FbpA, Bcp, NMB 1124, NMB 1162, NMB 1220, NMB 1313, NMB 1953, HtrA, Hsf, LbpB, FrpA, FrpC, FrpA/C, OMP85, PldA, LbpA, TbpA(high), TbpB(low), TbpB(high), HpuA, HpuB, Hap, IgA protease, AspA, PilQ, MltA, HimD, HisD, GNA1870, OstA, HlpA, TspA, TspB, P2086, Lipo28, Sibp, NMB0964, NMB0293, NMB0315, NMB1119, TdfH, PorB, NM-ADPRT, VapD and either or both of LPS immunotype L2 and LPS immunotype L3.
20. The immunogenic composition of embodiments 1-19 wherein TbpA (high) is selected together with at least one further antigen selected from the group consisting of: PilC, MafA, MafB, Omp26, NMB0995, FhaC, FbpA, Bcp, NMB 1124, NMB 1162, NMB 1220, NMB 1313, NMB 1953, HtrA, Hsf, LbpB, FrpA, FrpC, FrpA/C, OMP85, PldA, LbpA, TbpB(low), TbpB(high), HpuA, HpuB, Hap, IgA protease, AspA, PilQ, MltA, HimD, HisD, GNA1870, OstA, HlpA, TspA, TspB, P2086, Lipo28, Sibp, NMB0964, NMB0293, NMB0315, NMB1119, TdfH, PorB, NM-ADPRT, VapD and either or both of LPS immunotype L2 and LPS immunotype L3.
21. The immunogenic composition of embodiments 1-20 wherein LbpB is selected together with at least one further antigen selected from the group consisting of:
   PilC, MafA, MafB, Omp26, NMB0995, FhaC, FbpA, Bcp, NMB 1124, NMB 1162, NMB 1220, NMB 1313, NMB 1953, HtrA, Hsf, FrpA, FrpC, FrpA/C, OMP85, PldA, LbpA, TbpB(low), TbpB(high), HpuA, HpuB, Hap, IgA protease, AspA, PilQ, MltA, HimD, HisD, GNA1870, OstA, HlpA, TspA, TspB, P2086, Lipo28, Sibp, NMB0964, NMB0293, NMB0315, NMB1119, TdfH, PorB, NM-ADPRT, VapD and either or both of LPS immunotype L2 and LPS immunotype L3.
22. The immunogenic composition of embodiments 1-21 wherein OMP85 is selected together with at least one further antigen selected from the group consisting of:
   PilC, MafA, MafB, Omp26, NMB0995, FhaC, FbpA, Bcp, NMB 1124, NMB 1162, NMB 1220, NMB 1313, NMB 1953, HtrA, Hsf, FrpA, FrpC, FrpA/C, PldA, LbpA, TbpB(low), TbpB(high), HpuA, HpuB, Hap, IgA protease, AspA, PilQ, MltA, HimD, HisD, GNA1870, OstA, HlpA, TspA, TspB, P2086, Lipo28, Sibp, NMB0964, NMB0293, NMB0315, NMB1119, TdfH, PorB, NM-ADPRT, VapD and either or both of LPS immunotype L2 and LPS immunotype L3.
23. The immunogenic composition of embodiments 1-22 wherein Hap is selected together with at least one further antigen selected from the group consisting of:
   PilC, MafA, MafB, Omp26, NMB0995, FhaC, FbpA, Bcp, NMB 1124, NMB 1162, NMB 1220, NMB 1313, NMB 1953, HtrA, Hsf, FrpA, FrpC, FrpA/C, PldA, LbpA, TbpB(low), TbpB(high), HpuA, HpuB, IgA protease, AspA, PilQ, MltA, HimD, HisD, GNA1870, OstA, HlpA, TspA, TspB, P2086, Lipo28, Sibp, NMB0964, NMB0293, NMB0315, NMB1119, TdfH, PorB, NM-ADPRT, VapD and either or both of LPS immunotype L2 and LPS immunotype L3.
24. The immunogenic composition of embodiments 1-23 wherein Hsf is selected together with at least one further antigen selected from the group consisting of:
   PilC, MafA, MafB, Omp26, NMB0995, FhaC, FbpA, Bcp, NMB 1124, NMB 1162, NMB 1220, NMB 1313, NMB 1953, HtrA, FrpA, FrpC, FrpA/C, PldA, LbpA, TbpB(low), TbpB(high), HpuA, HpuB, IgA protease, AspA, PilQ, MltA, HimD, HisD, GNA1870, OstA, HlpA, TspA, TspB, P2086, Lipo28, Sibp, NMB0964, NMB0293, NMB0315, NMB1119, TdfH, PorB, NM-ADPRT, VapD and either or both of LPS immunotype L2 and LPS immunotype L3.
25. The immunogenic composition of embodiments 1-24 wherein Frp A is selected together with at least one further antigen selected from the group consisting of:
   PilC, MafA, MafB, Omp26, NMB0995, FhaC, FbpA, Bcp, NMB 1124, NMB 1162, NMB 1220, NMB 1313, NMB 1953, HtrA, FrpC, FrpA/C, PldA, LbpA, TbpB(low), TbpB(high), HpuA, HpuB, IgA protease, AspA, PilQ, MltA, HimD, HisD, GNA1870, OstA, HlpA, TspA, TspB, P2086, Lipo28, Sibp, NMB0964, NMB0293, NMB0315, NMB1119, TdfH, PorB, NM-ADPRT, VapD and either or both of LPS immunotype L2 and LPS immunotype L3.
26. The immunogenic composition of embodiments 1-25 wherein FrpC is selected together with at least one further antigen selected from the group consisting of:
   PilC, MafA, MafB, Omp26, NMB0995, FhaC, FbpA, Bcp, NMB 1124, NMB 1162, NMB 1220, NMB 1313, NMB 1953, HtrA, PldA, LbpA, TbpB(low), TbpB(high), HpuA, HpuB, IgA protease, AspA, PilQ, MltA, HimD, HisD, GNA1870, OstA, HlpA, TspA, TspB, P2086, Lipo28, Sibp, NMB0964, NMB0293, NMB0315, NMB1119, TdfH, PorB, NM-ADPRT, VapD and either or both of LPS immunotype L2 and LPS immunotype L3.
27. The immunogenic composition of embodiments 1-26 wherein LPS immunotype L2 is selected together with at least one further antigen selected from the group consisting of: PilC, MafA, MafB, Omp26, NMB0995, FhaC, FbpA, Bcp, NMB 1124, NMB 1162, NMB 1220, NMB 1313, NMB 1953, HtrA, PldA, LbpA, TbpB(low), TbpB(high), HpuA, HpuB, IgA protease, AspA, PilQ, MltA, HimD, HisD, GNA1870, OstA, HlpA, TspA, TspB, P2086, Lipo28, Sibp, NMB0964, NMB0293, NMB0315, NMB1119, TdfH, PorB, NM-ADPRT , VapD and LPS immunotype L3.
28. The immunogenic composition of embodiments 1-27 wherein LPS immunotype L3 is selected together with at least one further antigen selected from the group consisting of: PilC, MafA, MafB, Omp26, NMB0995, FhaC, FbpA, Bcp, NMB 1124, NMB 1162, NMB 1220, NMB 1313, NMB 1953, HtrA, PldA, LbpA, TbpB(low), TbpB(high), HpuA, HpuB, IgA protease, AspA, PilQ, MltA, HimD, HisD, GNA1870, OstA, HlpA, TspA, TspB, P2086, Lipo28, Sibp, NMB0964, NMB0293, NMB0315, NMB1119, TdfH, PorB, NM-ADPRT and VapD.
29. The immunogenic composition of embodiments 1-28 wherein PilQ is selected together with at least one further antigen selected from the group consisting of:
   PilC, MafA, MafB, Omp26, NMB0995, FhaC, FbpA, Bcp, NMB 1124, NMB 1162, NMB 1220, NMB 1313, NMB 1953, HtrA, PldA, LbpA, TbpB(low), TbpB(high), HpuA, HpuB, IgA protease, AspA, MltA, HimD, HisD, GNA1870, OstA, HlpA, TspA, TspB, P2086, Lipo28, Sibp, NMB0964, NMB0293, NMB0315, NMB1119, TdfH, PorB, NM-ADPRT and VapD.
30. The immunogenic composition of embodiments 1-29 wherein HlpA is selected together with at least one further antigen selected from the group consisting of:
   PilC, MafA, MafB, Omp26, NMB0995, FhaC, FbpA, Bcp, NMB 1124, NMB 1162, NMB 1220, NMB 1313, NMB 1953, HtrA, PldA, LbpA, TbpB(low), TbpB(high), HpuA, HpuB, IgA protease, AspA, MltA, HimD, HisD, GNA1870, OstA, TspA, TspB, P2086, Lipo28, Sibp, NMB0964, NMB0293, NMB0315, NMB1119, TdfH, PorB, NM-ADPRT and VapD.
31. The immunogenic composition of embodiments 1-30 wherein MltA is selected together with at least one further antigen selected from the group consisting of:
   PilC, MafA, MafB, Omp26, NMB0995, FhaC, FbpA, Bcp, NMB 1124, NMB 1162, NMB 1220, NMB 1313, NMB 1953, HtrA, PldA, LbpA, TbpB(low), TbpB(high), HpuA, HpuB, IgA protease, AspA, HimD, HisD, GNA1870, OstA, TspA, TspB, P2086, Lipo28, Sibp, NMB0964, NMB0293, NMB0315, NMB1119, TdfH, PorB, NM-ADPRT and VapD.
32. The immunogenic composition of embodiments 1-31 wherein GNA1870 is selected together with at least one further antigen selected from the group consisting of: PilC, MafA, MafB, Omp26, NMB0995, FhaC, FbpA, Bcp, NMB 1124, NMB 1162, NMB 1220, NMB 1313, NMB 1953, HtrA, PldA, LbpA, TbpB(low), TbpB(high), HpuA, HpuB, IgA protease, AspA, HimD, HisD, OstA, TspA, TspB, P2086, Lipo28, Sibp, NMB0964, NMB0293, NMB0315, NMB1119, TdfH, PorB, NM-ADPRT and VapD.
33. The immunogenic composition of embodiments 1-32 wherein NM-ADPRT is selected together with at least one further antigen selected from the group consisting of: PilC, MafA, MafB, Omp26, NMB0995, FhaC, FbpA, Bcp, NMB 1124, NMB 1162, NMB 1220, NMB 1313, NMB 1953, HtrA, PldA, LbpA, TbpB(low), TbpB(high), HpuA, HpuB, IgA protease, AspA, HimD, HisD, OstA, TspA, TspB, P2086, Lipo28, Sibp, NMB0964, NMB0293, NMB0315, NMB1119, TdfH, PorB, and VapD.
34. The immunogenic composition of embodiments 1-33 wherein MafA is selected together with at least one further antigen selected from the group consisting of:
   PilC, MafB, Omp26, NMB0995, FhaC, FbpA, Bcp, NMB 1124, NMB 1162, NMB 1220, NMB 1313, NMB 1953, HtrA, PldA, LbpA, TbpB(low), TbpB(high), HpuA, HpuB, IgA protease, AspA, HimD, HisD, OstA, TspA, TspB, P2086, Lipo28, Sibp, NMB0964, NMB0293, NMB0315, NMB1119, TdfH, PorB, and VapD.
35. The immunogenic composition of embodiments 1-34 wherein MafB is selected together with at least one further antigen selected from the group consisting of:
   PilC, MafB, Omp26, NMB0995, FhaC, FbpA, Bcp, NMB 1124, NMB 1162, NMB 1220, NMB 1313, NMB 1953, HtrA, PldA, LbpA, TbpB(low), TbpB(high), HpuA, HpuB, IgA protease, AspA, HimD, HisD, OstA, TspA, TspB, P2086, Lipo28, Sibp, NMB0964, NMB0293, NMB0315, NMB1119, TdfH, PorB, and VapD.
36. The immunogenic composition of embodiments 1-35 wherein NMB0315 is selected together with at least one further antigen selected from the group consisting of: PilC, MafB, Omp26, NMB0995, FhaC, FbpA, Bcp, NMB 1124, NMB 1162, NMB 1220, NMB 1313, NMB 1953, HtrA, PldA, LbpA, TbpB(low), TbpB(high), HpuA, HpuB, IgA protease, AspA, HimD, HisD, OstA, TspA, TspB, P2086, Lipo28, Sibp, NMB0964, NMB0293, NMB1119, TdfH, PorB, and VapD.
37. The immunogenic composition of embodiments 1-36 wherein NMB1119 is selected together with at least one further antigen selected from the group consisting of: PilC, MafB, Omp26, NMB0995, FhaC, FbpA, Bcp, NMB 1124, NMB 1162, NMB 1220, NMB 1313, NMB 1953, HtrA, PldA, LbpA, TbpB(low), TbpB(high), HpuA, HpuB, IgA protease, AspA, HimD, HisD, OstA, TspA, TspB, P2086, Lipo28, Sibp, NMB0964, NMB0293, NMB1119, TdfH, PorB, and VapD.
38. The immunogenic composition of embodiments 1-37 wherein HisD is selected together with at least one further antigen selected from the group consisting of:
   PilC, MafB, Omp26, NMB0995, FhaC, FbpA, Bcp, NMB 1124, NMB 1162, NMB 1220, NMB 1313, NMB 1953, HtrA, PldA, LbpA, TbpB(low), TbpB(high), HpuA, HpuB, IgA protease, AspA, HimD, OstA, TspA, TspB, P2086, Lipo28, Sibp, NMB0964, NMB0293, NMB1119, TdfH, PorB, and VapD.
39. The immunogenic composition of embodiments 1-38 wherein LbpA is selected together with at least one further antigen selected from the group consisting of:
   PilC, MafB, Omp26, NMB0995, FhaC, FbpA, Bcp, NMB 1124, NMB 1162, NMB 1220, NMB 1313, NMB 1953, HtrA, PldA, TbpB(low), TbpB(high), HpuA, HpuB, IgA protease, AspA, HimD, OstA, TspA, TspB, P2086, Lipo28, Sibp, NMB0964, NMB0293, NMB1119, TdfH, PorB, and VapD.
40. The immunogenic composition of embodiments 1-39 wherein NMB 0995 is selected together with at least one further antigen selected from the group consisting of: PilC, MafB, Omp26, FhaC, FbpA, Bcp, NMB 1124, NMB 1162, NMB 1220, NMB 1313, NMB 1953, HtrA, PldA, TbpB(low), TbpB(high), HpuA, HpuB, IgA protease, AspA, HimD, OstA, TspA, TspB, P2086, Lipo28, Sibp, NMB0964, NMB0293, NMB1119, TdfH, PorB, and VapD.
41. The immunogenic composition of embodiments 1-40 wherein Lipo28 is selected together with at least one further antigen selected from the group consisting of:
   PilC, MafB, Omp26, FhaC, FbpA, Bcp, NMB 1124, NMB 1162, NMB 1220, NMB 1313, NMB 1953, HtrA, PldA, TbpB(low), TbpB(high), HpuA, HpuB, IgA protease, AspA, HimD, OstA, TspA, TspB, P2086, Sibp, NMB0964, NMB0293, NMB1119, TdfH, PorB, and VapD.
42. The immunogenic composition of embodiments 1-41 wherein HimD is selected together with at least one further antigen selected from the group consisting of:
   PilC, MafB, Omp26, FhaC, FbpA, Bcp, NMB 1124, NMB 1162, NMB 1220, NMB 1313, NMB 1953, HtrA, PldA, TbpB(low), TbpB(high), HpuA, HpuB, IgA protease, AspA, OstA, TspA, TspB, P2086, Sibp, NMB0964, NMB0293, NMB1119, TdfH, PorB, and VapD.
43. The immunogenic composition of embodiments 1-42 wherein NMB1313 is selected together with at least one further antigen selected from the group consisting of: PilC, MafB, Omp26, FhaC, FbpA, Bcp, NMB 1124, NMB 1162, NMB 1220, NMB 1953, HtrA, PldA, TbpB(low), TbpB(high), HpuA, HpuB, IgA protease, AspA, OstA, TspA, TspB, P2086, Sibp, NMB0964, NMB0293, NMB1119, TdfH, PorB, and VapD.
44. The immunogenic composition of embodiments 1-43 wherein NMB1953 is selected together with at least one further antigen selected from the group consisting of: PilC, MafB, Omp26, FhaC, FbpA, Bcp, NMB 1124, NMB 1162, NMB 1220, HtrA, PldA, TbpB(low), TbpB(high), HpuA, HpuB, IgA protease, AspA, OstA, TspA, TspB, P2086, Sibp, NMB0964, NMB0293, NMB1119, TdfH, PorB, and VapD.
45. The immunogenic composition of embodiments 5-44 wherein a host cell from which the outer membrane vesicle preparation is derived has been engineered so as to down-regulate the expression from one or more of lgtB or lgtE, preferably the former.
46. The immunogenic composition of embodiments 5-45 wherein a host cell from which the outer membrane vesicle preparation is derived is unable to synthesize capsular polysaccharide and has preferably been engineered so as to down-regulate the expression from one or more of siaD, ctrA, ctrB, ctrC, ctrD, synA (equivalent to synX and siaA) or synB (equivalent to siaB and synC (equivalent to siaC), preferably siaD.
47. The immunogenic composition of embodiments 5-46 wherein a host cell from which the outer membrane vesicle preparation is derived has been engineered so as to down-regulate the expression of one or more of OpC, OpA or PorA, preferably PorA.
48. The immunogenic composition of embodiments 5-47 wherein a host cell from which the outer membrane vesicle preparation is derived has been engineered so as to down-regulate the expression of FrpB.
49. The immunogenic composition of embodiments 5-48 wherein a host cell from which the outer membrane vesicle preparation is derived has been engineered so as to down-regulate the expression from msbB and/or htrB, preferably msbB.
50. The immunogenic composition of embodiments 5-49 wherein the outer membrane vesicle preparation contains LPS which is conjugated to an outer membrane protein (OMP).
51. The immunogenic composition of embodiment 50 wherein LPS is conjugated (preferably intra-bleb ) to OMP in situ in the outer membrane vesicle preparation.
52. The immunogenic composition of embodiments 1-51 comprising an antigen derived from *Neisseria meningitidis,* preferably serogroup B.
53. The immunogenic compositions of embodiments 1-52 comprising an antigen derived from *Neisseria gonorrhoeae.*
54. The immunogenic composition of embodiments 1-52 wherein all neisserial antigens are derived from N.meningitidis, preerably serogroup B.
55. The immunogenic composition of embodiments 1-54 further comprising one or more bacterial capsular polysaccharides or oligosaccharides.
56. The immunogenic composition of embodiment 55 wherein the capsular polysaccharides or oligosaccharides are derived from bacteria selected from the group consisting of: *Neisseria meningitidis* serogroup A, C, Y and W-135, *Haemophilus influenzae b, Streptococcus pneumoniae,* Group A Streptococci, Group B Streptococci, *Staphylococcus aureus* and *Staphylococcus epidermidis.*
57. The immunogenic composition of embodiments 55-56 wherein the capsular polysaccharide or oligosaccharide is conjugated to a protein.
58. The immunogenic composition of embodiments 1-57 comprising an adjuvant.
59. The immunogenic composition of embodiment 58 comprising aluminium salts, preferably aluminium phosphate.
60. The immunogenic composition of embodiment 58 or 59 comprising 3D-MPL.
61. A vaccine comprising the immunogenic composition of embodiments 1-60 and a pharmaceutically acceptable carrier.
62. A vaccine comprising one or more polynucleotide(s) encoding two or more different proteins whose expression is driven by a eukaryotic promoter, wherein the proteins are selected from at least two of the following categories:
   a) at least one Neisserial adhesin selected from the group consisting of FhaB, NspA, PilC, Hsf, Hap, MafA, MafB, Omp26, NMB0315, NMB0995, NMB1119 and NadA;
   b) at least one Neisserial autotransporter selected from the group consisting of Hsf, Hap, IgA protease, AspA and NadA;
   c) at least one Neisserial toxin selected from the group consisting of FrpA, FrpC, FrpA/C, VapD and NM-ADPRT;
   d) at least one Neisserial Fe acquisition protein selected from the group consisting of TbpA high, TbpA low, TbpB high, TbpB low, LbpA, LbpB, P2086, HpuA, HpuB, Lipo28, Sibp, FbpA, BfrA, BfrB, Bcp, NMB0964 and NMB0293; or
   e) at least one Neisserial membrane associated protein, preferably integral outer membrane protein selected from the group consisting of PilQ, OMP85, FhaC, NspA, TbpA(high), TbpA(low), LbpA, HpuB, TspA, TspB, TdfH, PorB, HimD, HisD, GNA1870, OstA, HlpA, MltA, NMB 1124, NMB 1162, NMB 1220, NMB 1313, NMB 1953, HtrA and PldA.
63. A method for treatment or prevention of Neisserial disease comprising administering a protective dose of the vaccine of embodiments 61-62 to a host in need thereof.
64. The method of embodiment 63 in which *Neisseria meningitidis* infection is prevented or treated.
65. The method of embodiment 63 in which *Neisseria gonorrhoeae* infection is prevented or treated.
66. A use of the vaccine of embodiments 61-62 in the preparation of a medicament for treatment or prevention of Neisserial infection.
67. The use of embodiment 66 in which *Neisseria meningitidis* infection is prevented or treated.
68. The use of embodiment 66 in which *Neisseria gonorrhoeae* infection is prevented or treated.
69. A genetically engineered Neisserial strain from which the outer membrane vesicle preparation of embodiments 5-60 is derived.
70. A method of making the immunogenic composition of embodiment 1-60 comprising a step of mixing together at least two antigens from Neisseria.
71. A method of making the immunogenic composition of embodiments 5-60 comprising a step of isolating outer membrane vesicles from a Neisserial culture.
72. The method of embodiment 71 comprising a further step of combining at least two outer membrane vesicle preparations.
73. The method of embodiment 72 wherein at least one outer membrane vesicle preparation contains LPS of immunotype L2 and at least one outer membrane vesicle preparation contains LPS of immunotype L3.
74. The method of embodiments 71-73 wherein the outer membrane vesicles are isolated by extracting with a concentration of DOC of 0 - 0.5%.
75. The method of embodiment 74 wherein the outer membrane vesicles are isolated by extracting with a concentration of DOC of 0.02%-0.4%, 0.04%-0.3%, 0.06%-0.2%, 0.08%-0.15% or preferably around or exactly 0.1%.
76. A method of making the vaccine of embodiment 61 comprising a step of combining the immunogenic composition of embodiments 1-60 with a pharmaceutically acceptable carrier.
77. A method of preparing an immune globulin for use in prevention or treatment of Neisserial infection comprising the steps of immunising a recipient with the vaccine of embodiment 61 and isolating immune globulin from the recipient.
78. An immune globulin prepared by the method of embodiment 77.
79. A pharmaceutical composition comprising the immune globulin of embodiment 78 and a pharmaceutically acceptable carrier.
80. A method for treatment or prevention of Neisserial infection comprising a step of administering to a patient an effective amount of the pharmaceutical preparation of embodiment 79.
81. A use of the pharmaceutical preparation of embodiment 79 in the manufacture of a medicament for the treatment or prevention of Neisserial disease.
82. The immunogenic composition of embodiment 5-60, comprising a meningococcal bleb of immunotype L2 and a meningococcal bleb of immunotype L3.
83. The immunogenic composition of embodiment 82 wherein TbpA(high) is upregulated in one of the blebs.
84. The immunogenic composition of embodiment 82 or 83 wherein TbpA(low) is upregulated in one of the blebs.
85. The immunogenic composition of embodiments 82-84 wherein Hsf is upregulated in one of the blebs.
86. The immunogenic composition of embodiments 82-85 wherein OMP85 is upregulated in one of the blebs.
87. The immunogenic composition of embodiments 82-86 wherein the blebs are isolated from meningococcal strains incapable of making capsular polysaccharide, preferably siaD⁻.
88. The immunogenic composition of embodiments 82-87 wherein the L2 and/or L3 LPS oligosaccharide structures are truncated consistent with the blebs having been isolated from meningococcal strains that are lgtB⁻.
89. The immiunogenic composition of embodiments 82-88 wherein the blebs are isolated from meningococcal strains that have downregulated expression of msbB.
90. The immunogenic composition of embodiments 82-89 wherein the L2 and/or L3 LPS oligosaccharide moieties are intra-bleb conjugated to outer-membrane proteins integral to the bleb.
91. The immunogenic composition of embodiments 82-90 wherein the blebs are derived from meningococcal strains which have downregulated expression of one or more of: FrpB, PorA, Opa or Opc.

## Claims

1. An immunogenic composition comprising at least one Neisserial Fe acquisition protein antigen and at least one different antigen, wherein the at least one different antigen is selected from at least one of the following categories:
a) at least one Neisserial adhesin;
b) at least one Neisserial autotransporter;
c) at least one Neisserial toxin; or
d) at least one Neisserial membrane associated protein, preferably integral outer membrane protein,
and wherein the antigens, where present in an outer membrane vesicle, have been upregulated in the outer membrane vesicle.

2. The immunogenic composition of claim 1, wherein the at least one Neisserial Fe acquisition protein antigen is selected from the group consisting of NMB0964, TbpA (high), TbpA (low), TbpB (high), TbpB (low), LbpA, LbpB, P2086, HpuA, HpuB, Lipo28, Sibp, FbpA, BfrA, BfrB, Bcp and NMB0293, and the at least one different antigen is selected from at least one of the following categories:
a) at least one Neisserial adhesin selected from the group consisting of FhaB, NspA, PilC, Hsf, Hap, MafA, MafB, Omp26, NMB0315, NMB0995, NMB1119 and NadA;
b) at least one Neisserial autotransporter selected from the group consisting of Hsf, Hap, IgA protease, AspA and NadA;
c) at least one Neisserial toxin selected from the group consisting of FrpA, FrpC, FrpA/C, VapD, NM-ADPRT; or
d) at least one Neisserial membrane associated protein, preferably integral outer membrane protein selected from the group consisting of PilQ, OMP85, FhaC, NspA, TbpA(high), TbpA(low), LbpA, HpuB, TspA, TspB, TdfH, PorB, HimD, HisD, GNA1870, OstA, HlpA, MltA, NMB 1124, NMB 1162, NMB 1220, NMB 1313, NMB 1953, HtrA and PldA.

3. The immunogenic composition of claim 1 or claim 2, comprising at least or exactly two, three, four, five, six, seven, eight, nine or ten different antigens selected from at least or exactly two, three, four or all five of the categories of antigens as defined in claim 1.

4. The immunogenic composition of any one of claims 1 to 3, wherein NMB0964 and Hsf, or NMB0964 and Hap, or NMB0964 and NadA, or NMB0964 and GNA1870, or Lipo28 and Hsf, or Lipo28 and Hap, or Lipo28 and NadA, or Lipo28 and GNA1870, or TbpA and NspA, or TbpA and Hsf, are selected.

5. The immunogenic composition of claim 4, wherein:
a) NMB0964 and Hsf are selected together with at least one further different antigen selected from the group consisting of FhaB, PilC, MafA, MafB, Omp26, NMB0995, FhaC, FbpA, Bcp, NMB 1124, NMB 1162, NMB 1220, NMB 1313, NMB 1953, HtrA, LbpB, FrpA, FrpC, FrpA/C, NadA, OMP85, PldA, LbpA, TbpA (low), TbpA (high), TbpB (low), TbpB (high), HpuA, HpuB, Hap, IgA protease, AspA, PilQ, MltA, HimD, HisD, GNA1870, OstA, HlpA, NspA, TspA, TspB, P2086, Lipo28, Sibp, NMB0293, NMB0315, NMB1119, TdfH, PorB, NM-ADPRT and VapD; or
b) NMB0964 and GNA1870 are selected together with at least one further different antigen selected from the group consisting of FhaB, PilC, MafA, MafB, Omp26, NMB0995, FhaC, FbpA, Bcp, NMB 1124, NMB 1162, NMB 1220, NMB 1313, NMB 1953, HtrA, Hsf, LbpB, FrpA, FrpC, FrpA/C, NadA, OMP85, PldA, LbpA, TbpA (low), TbpA(high), TbpB(low), TbpB(high), HpuA, HpuB, Hap, IgA protease, AspA, PilQ, MltA, HimD, HisD, OstA, HlpA, NspA, TspA, TspB, P2086, Lipo28, Sibp, NMB0293, NMB0315, NMB1119, TdfH, PorB, NM-ADPRT, and VapD; or
c) NMB0964 and Hap are selected together with at least one further different antigen selected from the group consisting of FhaB, PilC, MafA, MafB, Omp26, NMB0995, FhaC, FbpA, Bcp, NMB 1124, NMB 1162, NMB 1220, NMB 1313, NMB 1953, HtrA, Hsf, LbpB, FrpA, FrpC, FrpA/C, NadA, OMP85, PldA, LbpA, TbpA (low), TbpA(high), TbpB(low), TbpB(high), HpuA, HpuB, GNA1870, IgA protease, AspA, PilQ, MltA, HimD, HisD, OstA, HlpA, NspA, TspA, TspB, P2086, Lipo28, Sibp, NMB0293, NMB0315, NMB1119, TdfH, PorB, NM-ADPRT, and VapD; or
d) Lipo28 and NadA are selected together with at least one further different antigen selected from the group consisting of FhaB, PilC, MafA, MafB, Omp26, NMB0995, FhaC, FbpA, Bcp, NMB 1124, NMB 1162, NMB 1220, NMB 1313, NMB 1953, HtrA, Hsf, LbpB, FrpA, FrpC, FrpA/C, OMP85, PldA, LbpA, TbpA (low), TbpA(high), TbpB(low), TbpB(high), HpuA, HpuB, Hap, IgA protease, AspA, PilQ, MltA, HimD, HisD, GNA1870, OstA, HlpA, NspA, TspA, TspB, P2086, Sibp, NMB0964, NMB0293, NMB0315, NMB1119, TdfH, PorB, NM-ADPRT, and VapD; or
e) Lipo28 and GNA1870 are selected together with at least one further different antigen selected from the group consisting of FhaB, PilC, MafA, MafB, Omp26, NMB0995, FhaC, FbpA, Bcp, NMB 1124, NMB 1162, NMB 1220, NMB 1313, NMB 1953, HtrA, Hsf, LbpB, FrpA, FrpC, FrpA/C, NadA, OMP85, PldA, LbpA, TbpA (low), TbpA(high), TbpB(low), TbpB(high), HpuA, HpuB, Hap, IgA protease, AspA, PilQ, MltA, HimD, HisD, OstA, HlpA, NspA, TspA, TspB, P2086, Sibp, NMB0964, NMB0293, NMB0315, NMB1119, TdfH, PorB, NM-ADPRT, and VapD; or
f) TbpA and Hsf are selected together with at least one further different antigen selected from the group consisting of FhaB, Hap, FrpA/C, LbpB, OMP85, PilQ, NspA and FrpC.

6. The immunogenic composition of any one of claims 1 to 5, wherein said composition:
a) is a subunit composition;
b) comprises an outer membrane vesicle preparation (preferably at least two different outer membrane vesicle preparations), wherein the antigens have been upregulated (preferably recombinantly) in the outer membrane vesicle; or
c) comprises a subunit composition having one or more of the antigens, and an outer membrane vesicle preparation (preferably at least two different outer membrane vesicle preparations) having at least one antigen which has been upregulated in the outer membrane vesicle.

7. The immunogenic composition of claim 6(b) or (c) wherein a host cell from which the outer membrane vesicle preparation is derived:
a) has been engineered so as to down-regulate the expression from one or more of lgtB or lgtE (preferably the former);
b) is unable to synthesize capsular polysaccharide and has preferably been engineered so as to down-regulate the expression from one or more of siaD, ctrA, ctrB, ctrC, ctrD, synA (equivalent to synX and siaA) or synB (equivalent to siaB) and synC (equivalent to siac), preferably siaD;
c) has been engineered so as to down-regulate the expression of one or more of OpC, OpA or PorA, preferably PorA;
d) has been engineered so as to down-regulate the expression of FrpB; and/or
e) has been engineered so as to down-regulate the expression from msbB and/or htrB, preferably msbB.

8. The immunogenic composition of claims 6(b) or (c) or 7 wherein the outer membrane vesicle preparation contains LPS which is conjugated (preferably intra-bleb) to an outer membrane protein (OMP), preferably in situ in the outer membrane vesicle preparation.

9. The immunogenic composition of claims 1-8 comprising an antigen derived from *Neisseria meningitidis* (preferably serogroup B) or *Neisseria gonorrhoeae,* wherein preferably all Neisserial antigens are derived from *N. meningitidis* (preferably serogroup B).

10. A vaccine comprising the immunogenic composition of claims 1-9 and a pharmaceutically acceptable carrier.

11. A vaccine comprising one or more polynucleotides whose expression is driven by a eukaryotic promoter, encoding at least one Neisserial Fe acquisition protein selected from the group consisting ofNMB0964, TbpA (high), TbpA (low), TbpB (high), TbpB (low), LbpA, LbpB, P2086, HpuA, HpuB, Lipo28, Sibp, FbpA, BfrA, BfrB, Bcp and NMB0293, and at least one different protein selected from at least one of the following categories:
a) at least one Neisserial adhesin selected from the group consisting of FhaB, NspA, PilC, Hsf, Hap, MafA, MafB, Omp26, NMB0315, NMB0995, NMB1119 and NadA;
b) at least one Neisserial autotransporter selected from the group consisting of Hsf, Hap, IgA protease, AspA and NadA;
c) at least one Neisserial toxin selected from the group consisting of FrpA, FrpC, FrpA/C, VapD and NM-ADPRT; or
d) at least one Neisserial membrane associated protein, preferably integral outer membrane protein selected from the group consisting of PilQ, OMP85, FhaC, NspA, TbpA(high), TbpA(low), LbpA, HpuB, TspA, TspB, TdfH, PorB, HimD, HisD, GNA1870, OstA, HlpA, MltA, NMB 1124, NMB 1162, NMB 1220, NMB 1313, NMB 1953, HtrA and PldA.

12. A use of the vaccine of claim 10 or claim 11 in the preparation of a medicament for treatment or prevention of Neisserial, preferably *Neisseria meningitidis,* infection.

13. The vaccine of claim 10 or claim 11 for use in the treatment or prevention of Neisserial, preferably *Neisseria meningitidis*, infection.

14. A genetically engineered Neisserial strain from which the outer membrane vesicle preparation of claim 6(b) or (c) is derived.

15. A method of making the immunogenic composition of claims 1-9 comprising a step of mixing together at least two antigens from Neisseria.

16. A method of making the immunogenic composition of claims 6(b) or (c) or 7-9 comprising a step of isolating outer membrane vesicles from a Neisserial culture, optionally comprising a further step of combining at least two outer membrane vesicle preparations, wherein preferably the outer membrane vesicles are isolated by extracting with a concentration of DOC of 0-0.5%.

17. A method of making the vaccine of claim 10 comprising a step of combining the immunogenic composition of claims 1-9 with a pharmaceutically acceptable carrier.


SEQUENCE LISTING

<110> GlaxoSmithKline Biologicals s.a.

<120> NEISSERIAL VACCINE COMPOSITION COMPRISING A COMBINATION OF ANTIGENS

<130> B45315D3

<160> 24

<150> GB 0218037.0
<151> 2002-08-02

<150> GB 0218036.2
<151> 2002-08-02

<150> GB 0218035.4
<151> 2002-08-02

<150> GB 0218051.1
<151> 2002-08-02

<150> GB 0220197.8
<151> 2002-08-30

<150> GB 0220199.4
<151> 2002-08-30

<150> GB 0225524.8
<151> 2002-11-01

<150> GB 0225531.3
<151> 2002-11-01

<150> GB 0230164.6
<151> 2002-12-24

<150> GB 0230168.7
<151> 2002-12-24

<150> GB 0230170.3
<151> 2002-12-24

<150> GB 0305028.3
<151> 2003-03-05

<170> FastSEQ for Windows Version 4.0

<210> 1
<211> 9
<212> PRT
<213> Neisseria meningitidis

<220>
<221> VARIANT
<222> (1)...(9)
<223> Xaa = Any Amino Acid

<400> 1

Leu Xaa Gly Gly Xaa Gly Asn Asp Xaa
 1               5

<210> 2
<211> 9
<212> PRT
<213> Neisseria meningitidis

<220>
<221> VARIANT
<222> (1)...(9)
<223> Xaa = Any Amino Acid

<400> 2
Leu Xaa Gly Gly Xaa Gly Asp Asp Xaa
 1               5

<210> 3
<211> 10
<212> PRT
<213> Neisseria meningitidis

<400> 3
Lys Thr Lys Cys Lys Phe Leu Lys Lys Cys
 1               5                   10

<210> 4
<211> 31
<212> DNA
<213> Artificial Sequence

<220>
<223> primer

<400> 4
ggaattccat atgatgaaca aaatataccg c                                    31

<210> 5
<211> 31
<212> DNA
<213> Artificial Sequence

<220>
<223> primer

<400> 5
gtagctagct agcttaccac tgataaccga c                                    31

<210> 6
<211> 36
<212> DNA
<213> Artificial Sequence

<220>
<223> primer

<400> 6
aactgcagaa ttaatatgaa aggagaagaa cttttc                               36

<210> 7
<211> 33
<212> DNA
<213> Artificial Sequence

<220>
<223> primer

<400> 7
gacatactag tttatttgta gagctcatcc atg                                33

<210> 8
<211> 30
<212> DNA
<213> Artificial Sequence

<220>
<223> primer

<400> 8
tccccgcggg ccgtctgaat acatcccgtc                                    30

<210> 9
<211> 51
<212> DNA
<213> Artificial Sequence

<220>
<223> primer

<400> 9
catatgggct tccttttgta aatttgaggg caaacacccg atacgtcttc a            51

<210> 10
<211> 48
<212> DNA
<213> Artificial Sequence

<220>
<223> primer

<400> 10
agacgtatcg ggtgtttgcc ctcaaattta caaaaggaag cccatatg                48

<210> 11
<211> 33
<212> DNA
<213> Artificial Sequence

<220>
<223> primer

<400> 11
gggtattccg ggcccttcag acggcgcagc agg                                33

<210> 12
<211> 45
<212> DNA
<213> Artificial Sequence

<220>
<223> primer

<400> 12
ggcctagcta gccgtctgaa gcgattagag tttcaaaatt tattc                45

<210> 13
<211> 42
<212> DNA
<213> Artificial Sequence

<220>
<223> primer

<400> 13
ggccaagctt cagacggcgt tcgaccgagt ttgagccttt gc                   42

<210> 14
<211> 39
<212> DNA
<213> Artificial Sequence

<220>
<223> primer

<400> 14
tcccccggga agatctggac gaaaaatctc aagaaaccg                       39

<210> 15
<211> 64
<212> DNA
<213> Artificial Sequence

<220>
<223> primer

<400> 15
ggaagatctc cgctcgagca aatttacaaa aggaagccga tatgcaacag caacatttgt 60
tccg                                                              64

<210> 16
<211> 36
<212> DNA
<213> Artificial Sequence

<220>
<223> primer

<400> 16
ggaagatctc cgctcgagac atcgggcaaa cacccg                          36

<210> 17
<211> 38
<212> DNA
<213> Artificial Sequence

<220>
<223> primer

<400> 17
tcccccggga gatctcacta gtattaccct gttatccc                        38

<210> 18
<211> 41
<212> DNA
<213> Artificial Sequence

<220>
<223> primer

<400> 18
ctcgagacca tgggcaaata tcatgtctac gaccccctcg c                41

<210> 19
<211> 43
<212> DNA
<213> Artificial Sequence

<220>
<223> primer

<400> 19
gtgcatagtg tcagagtttt tgtcgacgtc gtaattatag acc              43

<210> 20
<211> 39
<212> DNA
<213> Artificial Sequence

<220>
<223> primer

<400> 20
aatggaatac atatgaataa aggtttacat cgcattatc                   39

<210> 21
<211> 31
<212> DNA
<213> Artificial Sequence

<220>
<223> primer

<400> 21
ccaactagtg tttttcgcta cttggagctg t                           31

<210> 22
<211> 44
<212> DNA
<213> Artificial Sequence

<220>
<223> primer

<400> 22
aatggaatac atatgaataa aggtttacat cgcattatct ttag             44

<210> 23
<211> 45
<212> DNA
<213> Artificial Sequence

<220>
<223> primer

<400> 23
ggggccactc gaggtttttc gctacttgga gctgtttcag atagg 45

<210> 24
<211> 4217
<212> DNA
<213> Neisseria meningitidis

<400> 24
atgaataaag gtttacatcg cattatcttt agtaaaaagc acagcaccat ggttgcagta 60
gccgaaactg ccaacagcca gggcaaaggt aaacaggcag gcagttcggt ttctgtttca 120
ctgaaaactt caggcgacct ttgcggcaaa ctcaaaacca cccttaaaac tttggtctgc 180
tctttggttt ccctgagtat ggtattgcct gcccatgccc aaattaccac cgacaaatca 240
gcacctaaaa accagcaggt cgttatcctt aaaaccaaca ctggtgcccc cttggtgaat 300
atccaaactc cgaatggacg cggattgagc cacaaccgct atacgcagtt tgatgttgac 360
aacaaagggg cagtgttaaa caacgaccgt aacaataatc cgtttgtggt caaaggcagt 420
gcgcaattga ttttgaacga ggtacgcggt acggctagca aactcaacgg catcgttacc 480
gtaggcggtc aaaaggccga cgtgattatt gccaacccca acggcattac cgttaatggc 540
ggcggcttta aaaatgtcgg tcggggcatc ttaactaccg gtgcgcccca aatcggcaaa 600
gacggtgcac tgacaggatt tgatgtgcgt caaggcacat tgaccgtagg agcagcaggt 660
tggaatgata aaggcggagc cgactacacc ggggtacttg ctcgtgcagt tgctttgcag 720
gggaaattac agggtaaaaa cctggcggtt tctaccggtc ctcagaaagt agattacgcc 780
agcggcgaaa tcagtgcagg tacggcagcg ggtacgaaac cgactattgc ccttgatact 840
gccgcactgg gcggtatgta cgccgacagc atcacactga ttgccaatga aaaaggcgta 900
ggcgtcaaaa atgccggcac actcgaagcg gccaagcaat tgattgtgac ttcgtcaggc 960
cgcattgaaa acagcggccg catcgccacc actgccgacg gcaccgaagc ttcaccgact 1020
tatctctcca tcgaaaccac cgaaaaagga gcggcaggca catttatctc caatggtggt 1080
cggatcgaga gcaaaggctt attggttatt gagacgggag aagatatcag cttgcgtaac 1140
ggagccgtgg tgcagaataa cggcagtcgc ccagctacca cggtattaaa tgctggtcat 1200
aatttggtga ttgagagcaa aactaatgtg aacaatgcca aaggcccggc tactctgtcg 1260
gccgacggcc gtaccgtcat caaggaggcc agtattcaga ctggcactac cgtatacagt 1320
tccagcaaag gcaacgccga attaggcaat aacacacgca ttaccggggc agatgttacc 1380
gtattatcca acggcaccat cagcagttcc gccgtaatag atgccaaaga caccgcacac 1440
atcgaagcag gcaaaccgct ttctttggaa gcttcaacag ttacctccga tatccgctta 1500
aacggaggca gtatcaaggg cggcaagcag cttgctttac tggcagacga taacattact 1560
gccaaaacta ccaatctgaa tactcccggc aatctgtatg ttcatacagg taaagatctg 1620
aatttgaatg ttgataaaga tttgtctgcc gccagcatcc atttgaaatc ggataacgct 1680
gcccatatta ccggcaccag taaaaccctc actgcctcaa aagacatggg tgtggaggca 1740
ggctcgctga atgttaccaa taccaatctg cgtaccaact cgggtaatct gcacattcag 1800
gcagccaaag gcaatattca gcttcgcaat accaagctga acgcagccaa ggctctcgaa 1860
accaccgcat tgcagggcaa tatcgtttca gacggccttc atgctgtttc tgcagacggt 1920
catgtatcct tattggccaa cggtaatgcc gactttaccg gtcacaatac cctgacagcc 1980
aaggccgatg tcaatgcagg atcggttggt aaaggccgtc tgaaagcaga caataccaat 2040
atcacttcat cttcaggaga tattacgttg gttgccggca acggtattca gcttggtgac 2100
ggaaaacaac gcaattcaat caacggaaaa cacatcagca tcaaaaacaa cggtggtaat 2160
gccgacttaa aaaaccttaa cgtccatgcc aaaagcgggg cattgaacat tcattccgac 2220
cgggcattga gcatagaaaa taccaagctg gagtctaccc ataatacgca tcttaatgca 2280
caacacgagc gggtaacgct caaccaagta gatgcctacg cacaccgtca tctaagcatt 2340
accggcagcc agatttggca aaacgacaaa ctgccttctg ccaacaagct ggtggctaac 2400
ggtgtattgg cactcaatgc gcgctattcc caaattgccg acaacaccac gctgagagcg 2460
ggtgcaatca accttactgc cggtaccgcc ctagtcaagc gcggcaacat caattggagt 2520
accgtttcga ccaaaacttt ggaagataat gccgaattaa aaccattggc cggacggctg 2580
aatattgaag caggtagcgg cacattaacc atcgaacctg ccaaccgcat cagtgcgcat 2640
accgacctga gcatcaaaac aggcggaaaa ttgctgttgt ctgcaaaagg aggaaatgca 2700
ggtgcgccta gtgctcaagt ttcctcattg gaagcaaaag gcaatatccg tctggttaca 2760
ggagaaacag atttaagagg ttctaaaatt acagccggta aaaacttggt tgtcgccacc 2820
accaaaggca agttgaatat cgaagccgta aacaactcat tcagcaatta ttttcctaca 2880
caaaaagcgg ctgaactcaa ccaaaaatcc aaagaattgg aacagcagat tgcgcagttg 2940

aaaaaaagct cgcctaaaag caagctgatt ccaaccctgc aagaagaacg cgaccgtctc 3000
gctttctata ttcaagccat caacaaggaa gttaaaggta aaaaacccaa aggcaaagaa 3060
tacctgcaag ccaagctttc tgcacaaaat attgacttga tttccgcaca aggcatcgaa 3120
atcagcggtt ccgatattac cgcttccaaa aaactgaacc ttcacgccgc aggcgtattg 3180
ccaaaggcag cagattcaga ggcggctgct attctgattg acggcataac cgaccaatat 3240
gaaattggca agcccaccta caagagtcac tacgacaaag ctgctctgaa caagccttca 3300
cgtttgaccg gacgtacagg ggtaagtatt catgcagctg cggcactcga tgatgcacgt 3360
attattatcg gtgcatccga aatcaaagct ccctcaggca gcatagacat caaagcccat 3420
agtgatattg tactggaggc tggacaaaac gatgcctata ccttcttaaa aaccaaaggt 3480
aaaagcggca aaatcatcag aaaaaccaag tttaccagca cccgcgacca cctgattatg 3540
ccagcccccg tcgagctgac cgccaacggc ataacgcttc aggcaggcgg caacatcgaa 3600
gctaatacca cccgcttcaa tgcccctgca ggtaaagtta ccctggttgc gggtgaagag 3660
ctgcaactgc tggcagaaga aggcatccac aagcacgagt tggatgtcca aaaaagccgc 3720
cgctttatcg gcatcaaggt aggcaagagc aattacagta aaaacgaact gaacgaaacc 3780
aaattgcctg tccgcgtcgt cgcccaaact gcagccaccc gttcaggctg ggataccgtg 3840
ctcgaaggta ccgaattcaa aaccacgctg gccggtgcgg acattcaggc aggtgtaggc 3900
gaaaaagccc gtgccgatgc gaaaattatc ctcaaaggca ttgtgaaccg tatccagtcg 3960
gaagaaaaat tagaaaccaa ctcaaccgta tggcagaaac aggccggacg cggcagcact 4020
atcgaaacgc tgaaactgcc cagcttcgaa agccctactc cgcccaaact gaccgccccc 4080
ggtggctata tcgtcgacat tccgaaaggc aatttgaaaa ccgaaatcga aaagctggcc 4140
aaacagcccg agtatgccta tctgaaacag ctccaagtag cgaaaaacac tagtggccac 4200
catcaccatc accatta 4217